# EUROPEAN PATENT SPECIFICATION

(11) **EP 1 364 003 B1**
(45) Date of publication and mention of the grant of the patent: **18.06.2008**
(21) Application number: 01981370.8
(22) Date of filing: 03.10.2001
(51) Int. Cl.: C12N 15/00

(54) **G-PROTEIN FUSION RECEPTORS**
G-PROTEIN FUSIONSREZEPTOREN
RECEPTEURS DE FUSION DE PROTEINES G

(30) Priority: 03.10.2000 US 679664
(43) Date of publication of application: 26.11.2003
(73) Proprietor: AstraZeneca AB, 151 85 Södertälje (SE)
(72) Inventor: STORMANN, Thomas, Salt Lake City, UT 84105 (US); HAMMERLAND, Lance, G., Bountiful Utah 84010 (US); STORJOHANN, Laura, L., Salt Lake City Utah 84102 (US); BUSBY, James, G., Simi Valley, CA 93065 (US); GARRETT, James, E., Salt Lake City, UT 84105 (US); SIMIN, Rachel, T., Durham, NC 27713 (US)
(74) Representative: Boar, Bernard Robin
(86) International application number: PCT/US2001/031074
(87) International publication number: WO 2002/029033

(56) References cited:
- WO-A-97/05252
- WO-A-99/51641
- DATABASE SWALL [Online] 1 November 1997 (1997-11-01) retrieved from EBI Database accession no. Q14832 XP002220590
- DATABASE SWALL [Online] 1 November 1997 (1997-11-01) retrieved from EBI Database accession no. Q14833 XP002220591
- COUVE ANDRES ET AL: "GABAB receptors: A new paradigm in G protein signaling." MOLECULAR AND CELLULAR NEUROSCIENCE, vol. 16, no. 4, October 2000 (2000-10), pages 296-312, XP002220588 ISSN: 1044-7431
- JONES K A ET AL: "Signal transduction by GABA(B) receptor heterodimers." NEUROPSYCHOPHARMACOLOGY: OFFICIAL PUBLICATION OF THE AMERICAN COLLEGE OF NEUROPSYCHOPHARMACOLOGY. UNITED STATES OCT 2000, vol. 23, no. 4 Suppl, October 2000 (2000-10), pages S41-S49, XP002220589 ISSN: 0893-133X
- KAUPMANN K ET AL: "EXPRESSION CLONING OF GABAB RECEPTORS UNCOVERS SIMILARITY TO METABOTROPIC GLUTAMATE RECEPTORS" NATURE, MACMILLAN JOURNALS LTD. LONDON, GB, vol. 386, no. 6622, 20 March 1997 (1997-03-20), pages 239-246, XP002032306 ISSN: 0028-0836

## Description

### FIELD OF THE INVENTION

The present invention relates to a G-protein fusion receptors, nucleic acid encoding such receptors, and uses of such receptors and nucleic acid encoding such receptors.

### BACKGROUND

The references cited herein are not admitted to be prior art to the claimed invention.

Chimeric receptors made up of peptide segments from different receptors have different uses such as being used to assess the functions of different sequence regions and to assess the activity of different compounds at a particular receptor. Examples of using chimeric receptors to assess the activity of different compounds are provided by Dull et al., U.S. Patent No. 4,859,609, Dull et al., U.S. Patent No. 5,030,576, and Puller et al., U.S. Patent No. 5,981,195.

Dull et al. U.S. Patent No. 4,859,609, and Dull et al. U.S. Patent No. 5,030,576, indicate the production and use of chimeric receptors comprising a ligand binding domain of a predetermined receptor and a heterologous reporter polypeptide. The Dull *et al*. patents provide as examples of chimerics: (1) a chimeric receptor made up of the insulin receptor extracellular a chain, and the EGF receptor transmembrane and cytoplasmic domains without any HIR B-chain sequence; and (2) a hybrid receptor made up of the v-erB oncogene product intracellular domain fused to the EGF receptor extracellular and transmembrane domains.

Fuller et al. International Publication No. WO 97/05252 feature chimeric receptors made up of metabotropic glutamate receptor (mGluR) domains and calcium receptor (CaR) domains. The chimeric receptors allow the coupling of functional aspects of a mGluR with a CaR.

An example of the use of chimeric receptors to assess the functions of different sequence regions receptors are found in studies identifying regions of different guamine nucleotide-binding protein coupled receptors important for guanine nucleotide-binding protein coupling. (See, Kobilka et al., Science 240:1310-1316, 1988; Wess et al., FEBS Lett. 258:133-136, 1989; Cotecchia et al., Proc. Natl. Acad. Sci. USA 87:2896-2900, 1990; Lechleiter et al., EMBO J. 9:4381-4390, 1990; Wess et al., Mol. Pharmacol. 38:517-523, 1990; and Pin et al., EMBO J. 13:342,348, 1994.)

### SUMMARY OF THE INVENTION

The present invention features G-protein fusion receptors, nucleic acid encoding such receptors, and the use of such receptors and nucleic acid. G-protein fusion receptors comprise at least one domain from a CaR and a mGhiR, fused through a linker to a guanine nucleotide-binding protein (G-protein).

G-proteins are peripheral membrane proteins made up of an α subunit, a β subunit, and a γ subunit. G-proteins interconvert between a GDP bound and a GTP bound form. Different types of G-proteins can affect different enzymes, such as adenylate cyclase and phospholipase-C.

Thus, a first aspect of the present invention describes a G-protein fusion receptor consisting of the amino acid sequence of SEQ ID NO:49 or SEQ ID NO:61.

"Substantially similar" refers to at least 40% sequence similarity between respective polypeptide regions making up a domain. In preferred embodiments, substantially similar refers to at least 50%, at least 75%, at least 90%, at least 95% sequence similarity, or 100% (the same sequence), between polypeptide domains, The degree to which two polypeptide domains are substantially similar is determined by comparing the amino acid sequences located in corresponding domains. Sequence similarity is preferably determined using BLASTN (Altschul et al., J. Mol. Biol. 215:403-410, 1990).

The different receptor components of the G-protein receptor come from a chimeric receptor made up of different receptor domains. By swapping different domains compounds able to effect different domains of a particular receptor can be idendified and the activity of different compounds at different domains can be measured.

The CaR region that is present: consists of the CaR intracellular domain provided in SEQ. ID. NO. 11.

Venous different mGluR subtypes present in differed organisms, including humans, are described in different patent publications as follows: mGluR₁ - WO 94/29449, EP 569 240 A1, WO 92/10583 and U.S. Patent No. 5,385,831; mGluR₂- WO 94/29449, WO 96/06167, and EP 711832 A2; mGluR₃ - WO 94/29449, and WO 95/22609; mGluR₄- WO 95/08627, WO 95/22609, and WO 96/29404; mGluR₅ - WO 94/29449; mGluR₆- WO 95/08627; mGluR₇-U.S. Patent No. 5,831,047, WO 95/08627 and WO 96/29404; and mGluR₈-U.S. Patent Nos. 6,051,688, 6,077,675, 6,084,084 and EP 816 498 A2. (Each of these references are hereby incorporated by reference herein)

The mGluR regions that are present consist of the mGluR transmembrane domain from human mGluR 3 or human mGluR 4.

The linker is a polypeptide 3 amino acids in length, and is comprised of alanine amino acids.

Another aspect of the present invention describes a recombinant cell comprising an expression vector encoding for a G-protein fusion receptor, and a cell where the G-protein fusion receptor is expressed and is functional in the cell.

Another aspect of the present invention describes a recombinant cell produced by combining (a) a cell where a G-protein fusion receptor is expressed, and (b) a vector comprising nucleic acid encoding a G-protein fusion receptor and elements for introducing heterologous nucleic acid into the cell. Preferably, the G-protein fusion receptor is functional in the cell.

Another aspect of the present invention describes a process for the production of a G-protein fusion receptor. The process is performed by growing host cells comprising a G-protein fusion receptor.

Another aspect of the present invention describes a method of measuring the ability of a compound to affect G-protein fusion receptor activity.

Preferably, the domains are functionally coupled such that a signal from the binding of an extracellular ligand is transduced to the intracellular domain when the chimeric receptor is present in a suitable host cell. A suitable host cell contains the elements for functional signal transduction far receptors coupled to a G-protein.

Various examples are described herein. These examples are not intended in any way to limit the claimed invention.

Other features and advantages of the invention will be apparent from the following drawings, the description of the invention, the examples, and the claims.

### BRIEF DESCRIPTION OF DRAWINGS

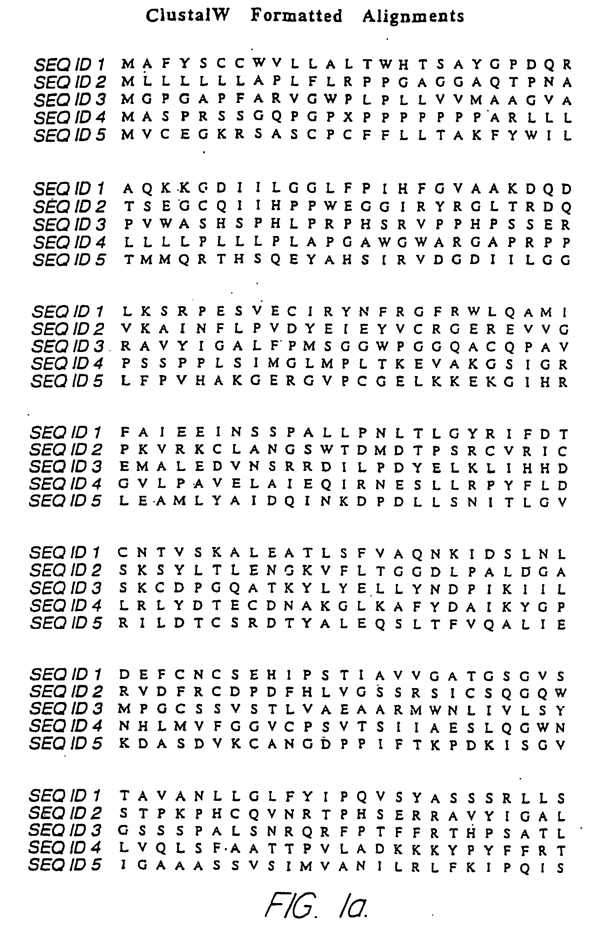

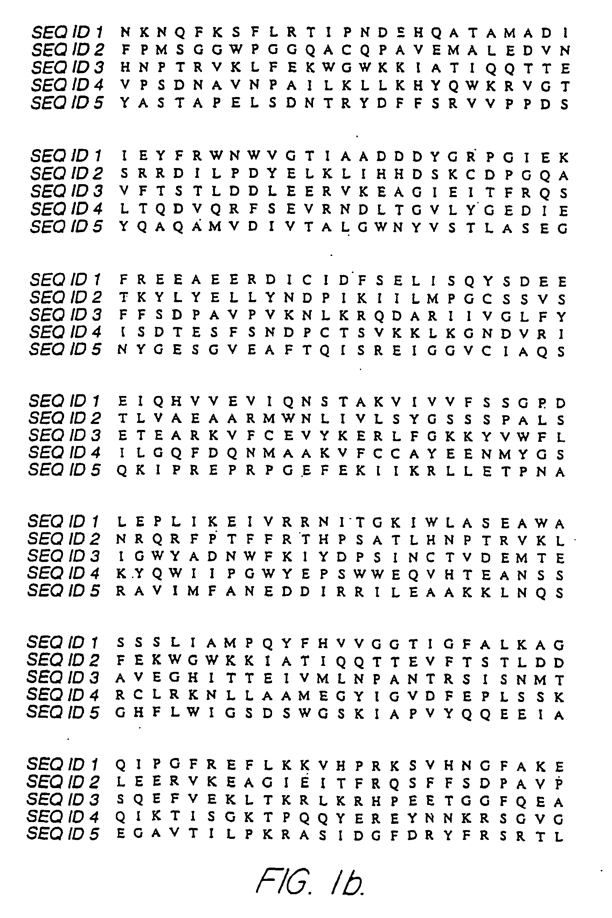

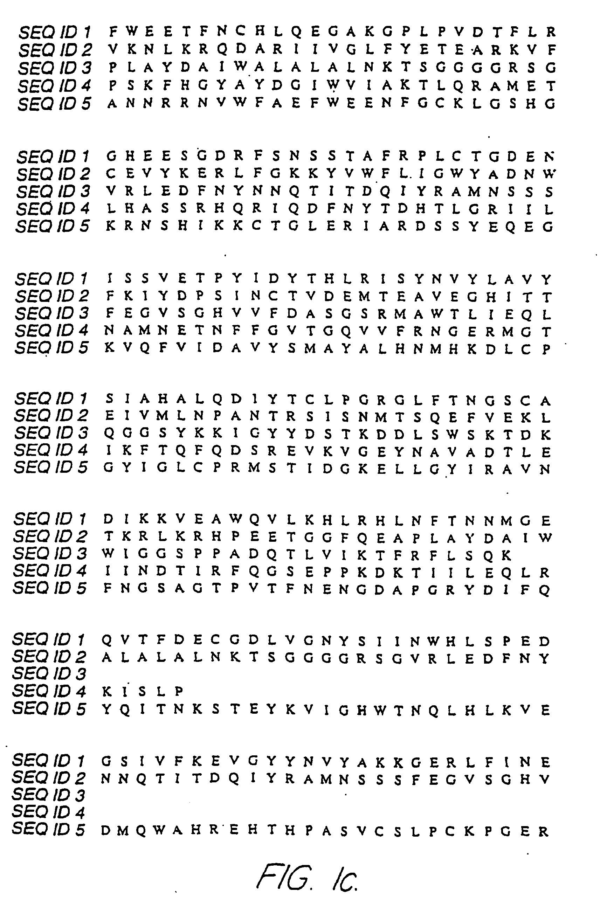

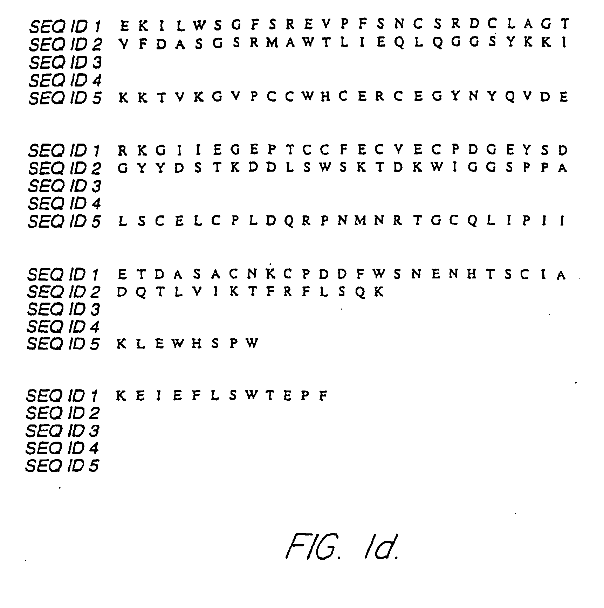

Figures 1a-1d illustrate the amino acid sequences of a human CaR extracellular domain (SEQ. ID. NO.1), a human GABA_{B}R1a extracellular domain (SEQ. ID. NO. 2), a human GABA_{B}R1b extracellular domain (SEQ. ID. NO.3), a human GABA_{B}R2 extracellular domain (SEQ. ID. NO. 4), and a human mGluR8 extracellular domain (SEQ. ID. NO. 5).

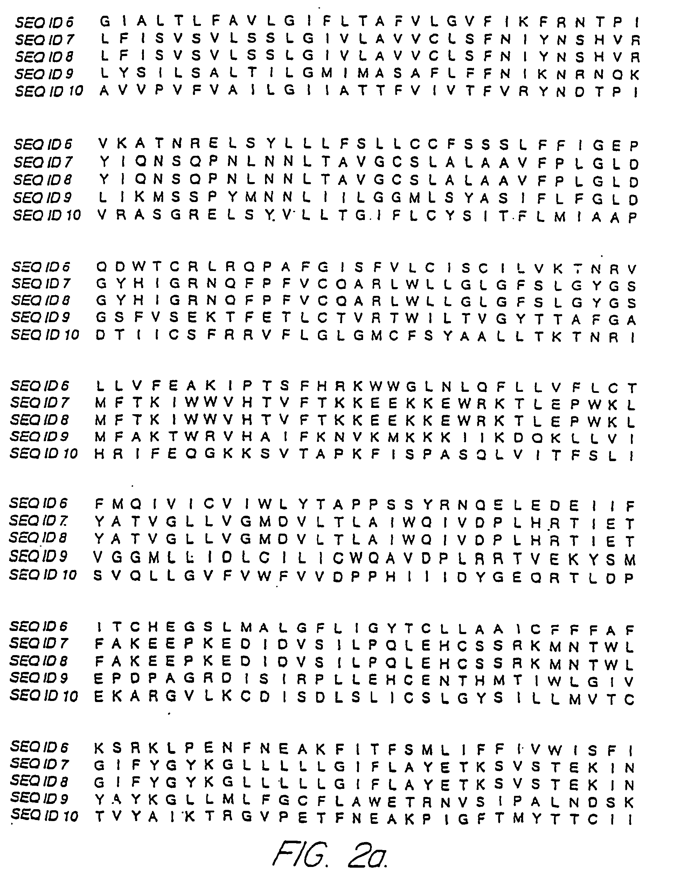

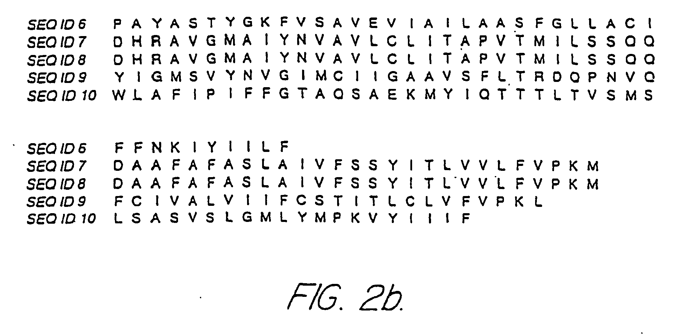

Figures 2a-2b illustrate the amino acid sequences of a human CaR transmembrane domain (SEQ. ID. NO. 6), a human GABA_{B}R1a transmembrane domain (SEQ. ID. NO. 7), a human GABA_{B}R1b transmembrane domain (SEQ. ID. NO. 8), a human GARA_{B}R2 transmembrane domain (SEQ.ID.NO.9), and a human mGluR8 transmembrane domain (SEQ. ID. NO. 10).

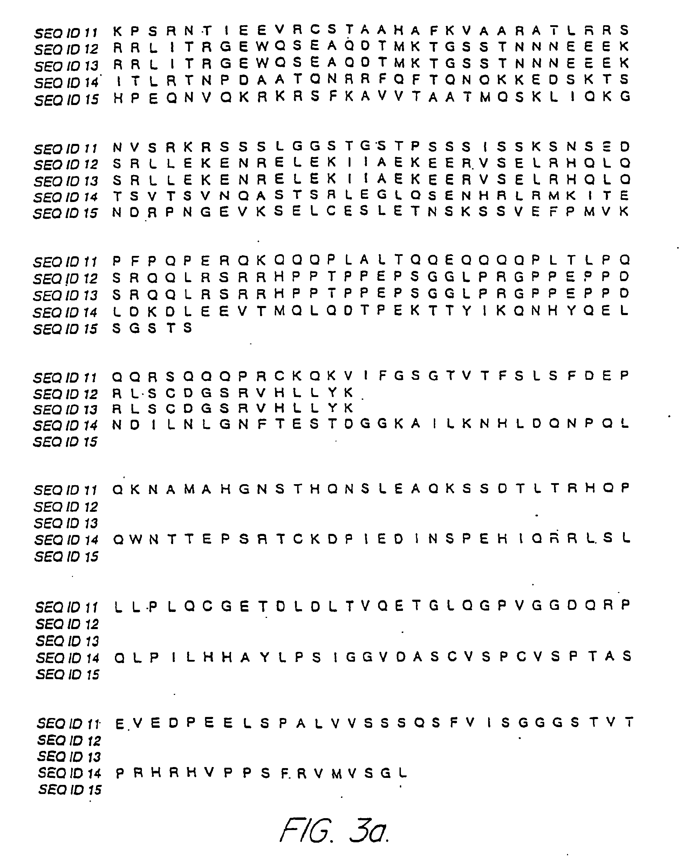

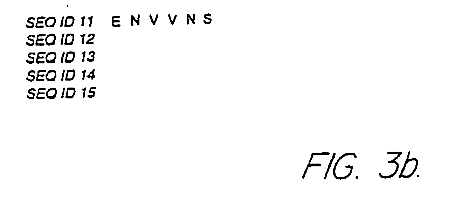

Figures 3a-3b illustrate the amino acid sequences of a human CaR intracellular domain (SEQ. ID. NO. 11), a human GABA_{B}R1a intracellular domain (SEQ. ID. NO.12), a human GABA_{B}R1b intracellular domain (SEQ. ID. NO. 13), a human GABA_{B}R2 intracellular domain (SEQ. ID. NO. 14), and a human mGluR8 intracellular domain (SEQ. ID. NO. 15).

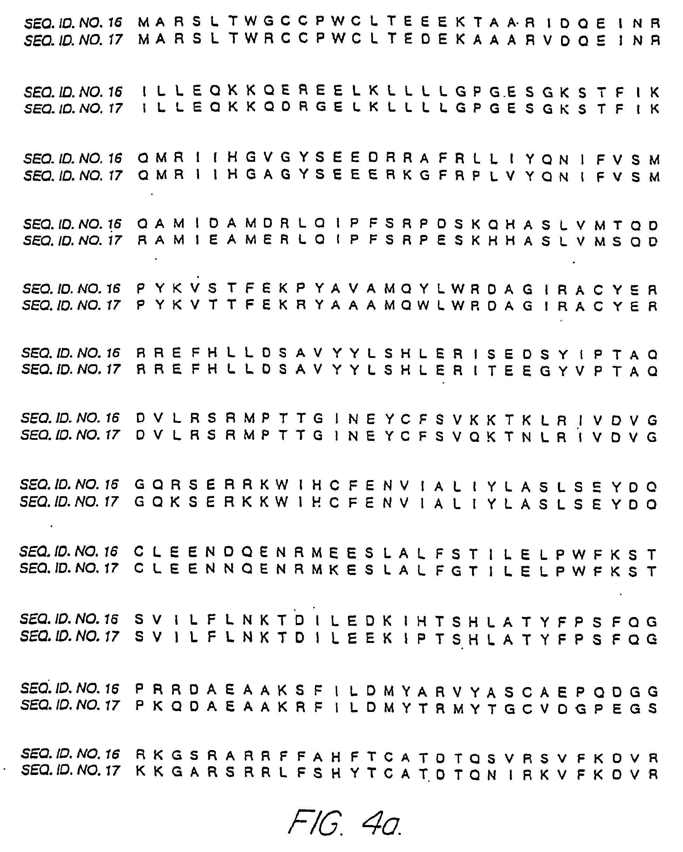

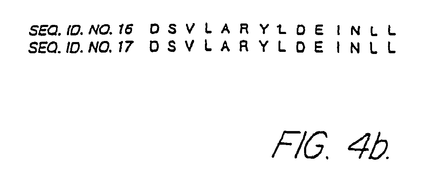

Figures 4a-4b illustrate the amino acid sequence of G₁₅ (SEQ. ID. NO.16) and G₁₆(SEQ. ID. NO.17).

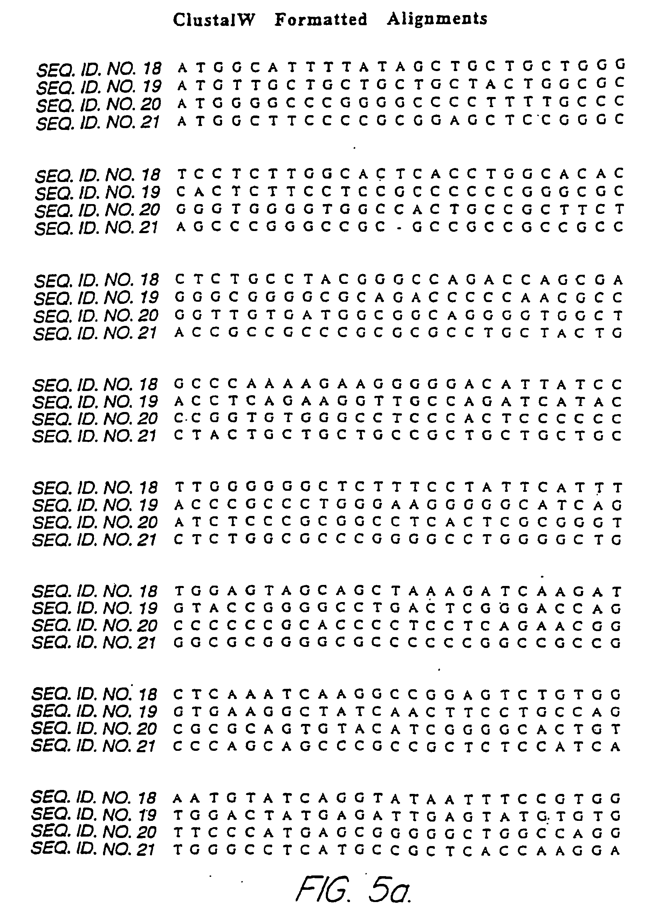

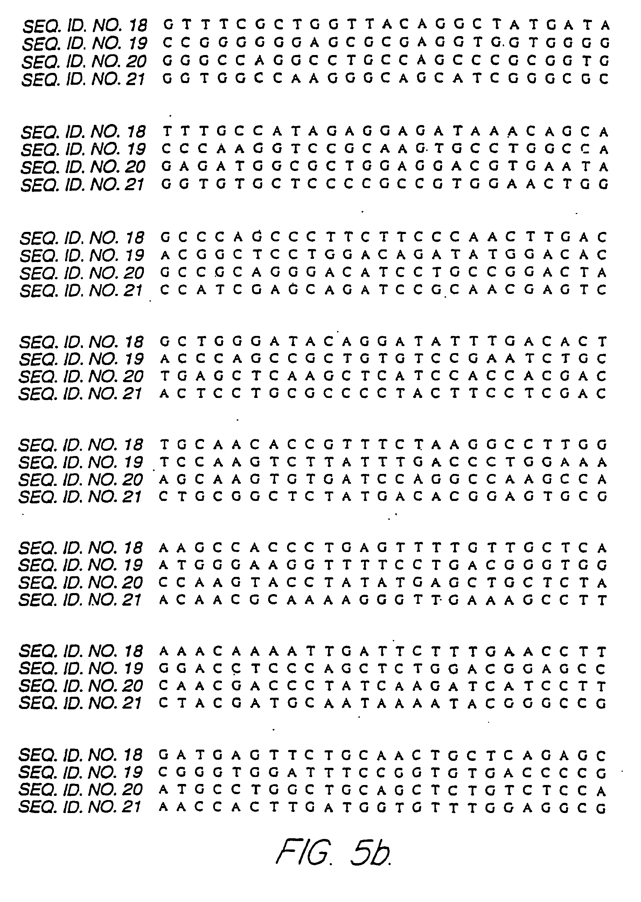

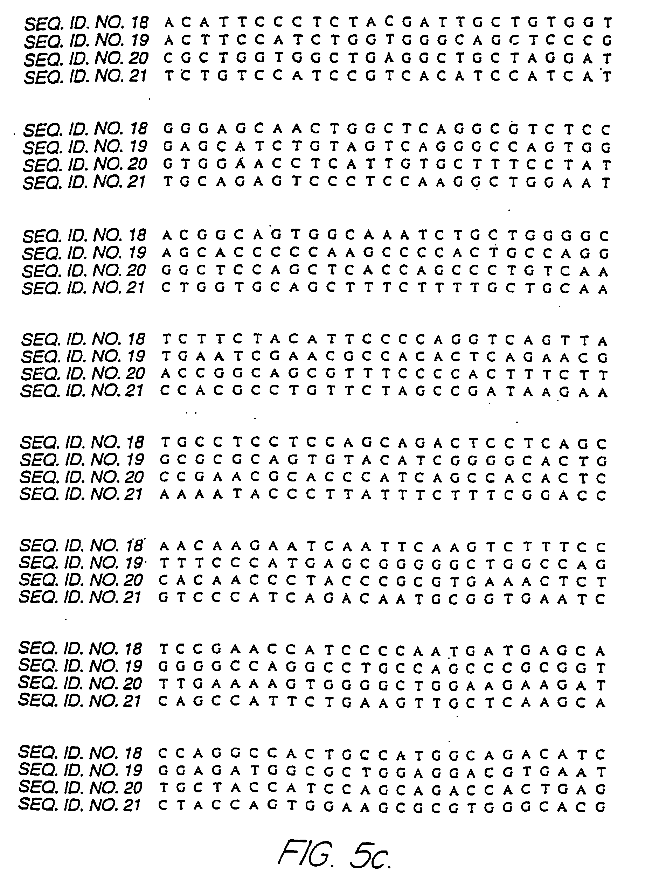

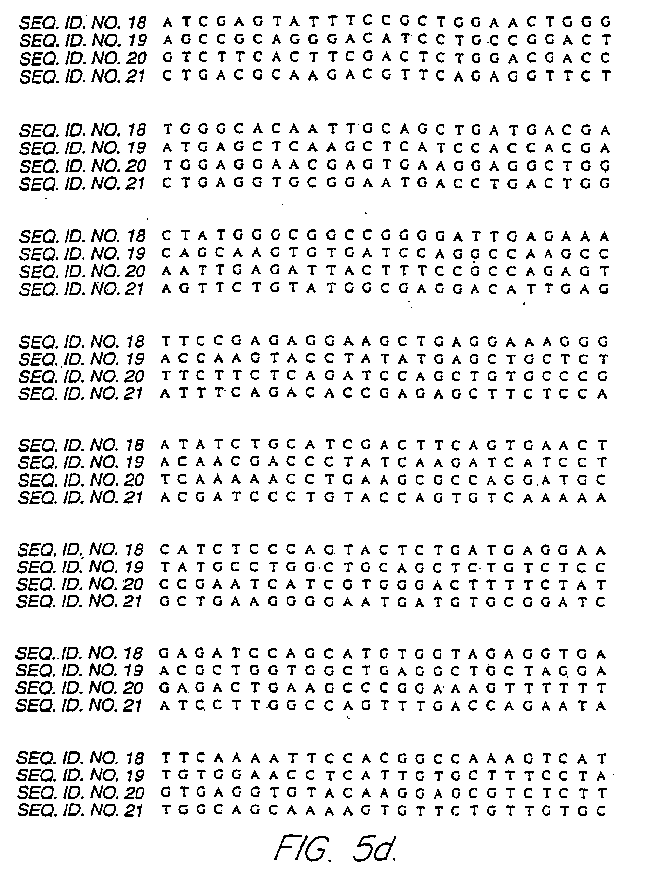

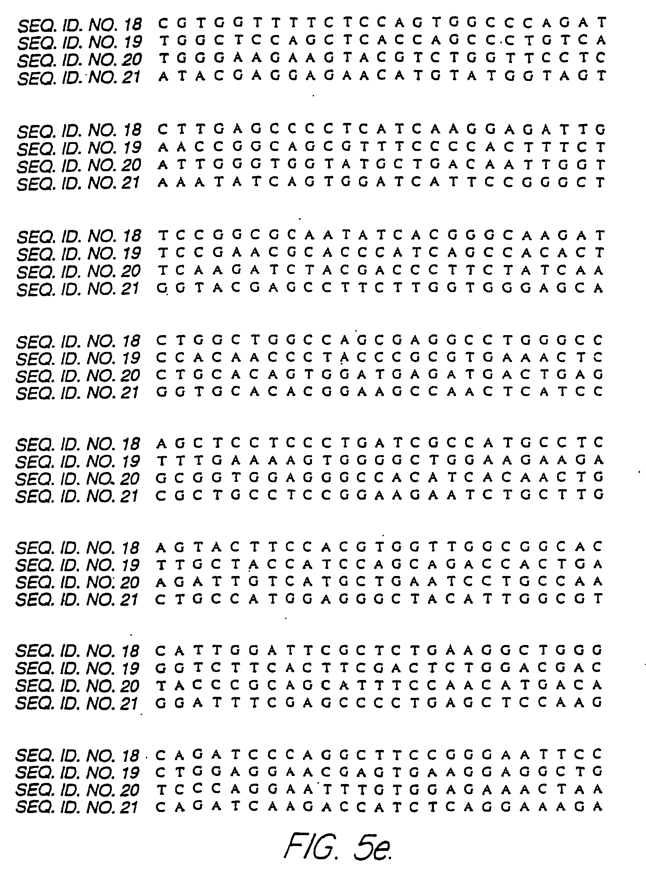

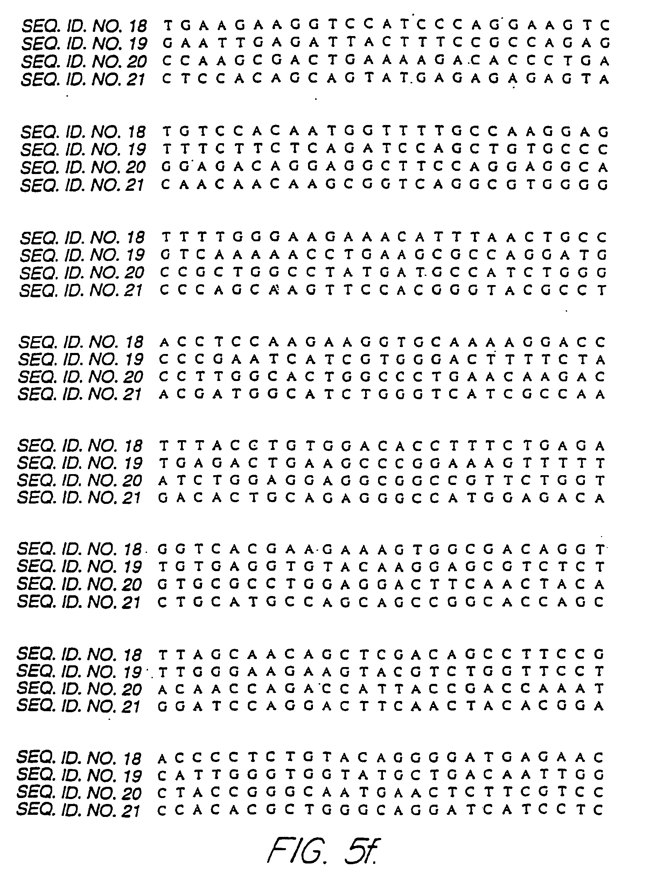

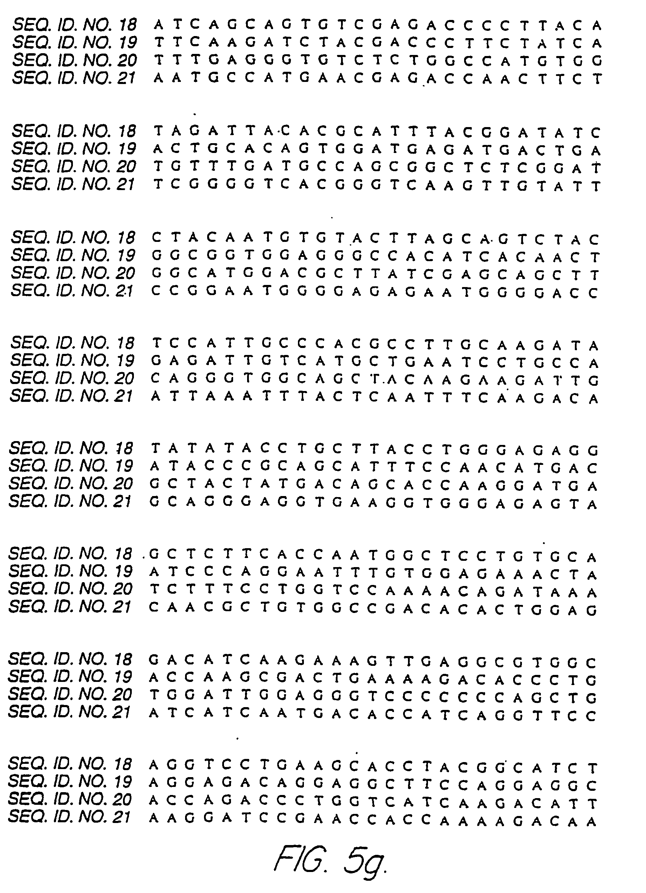

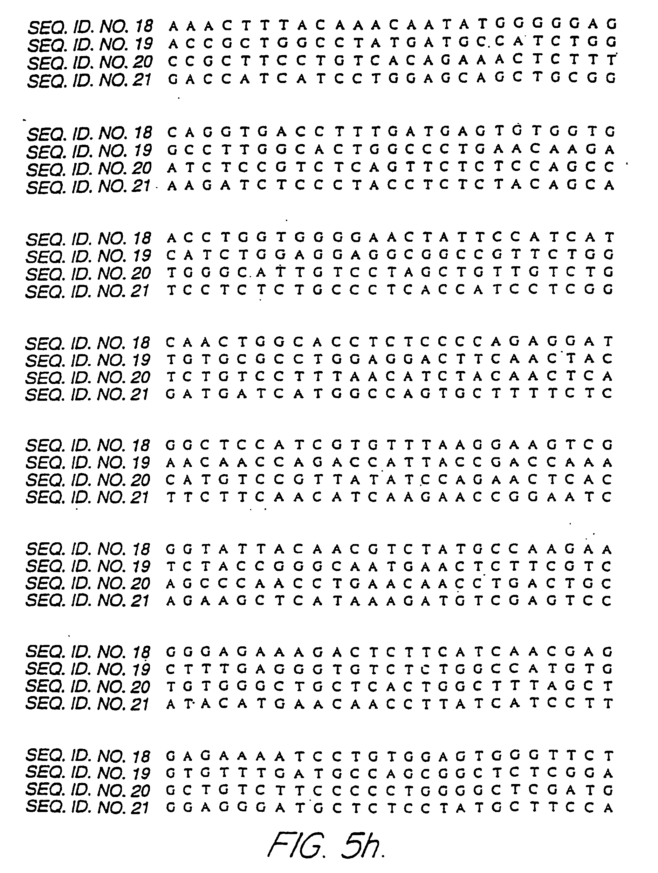

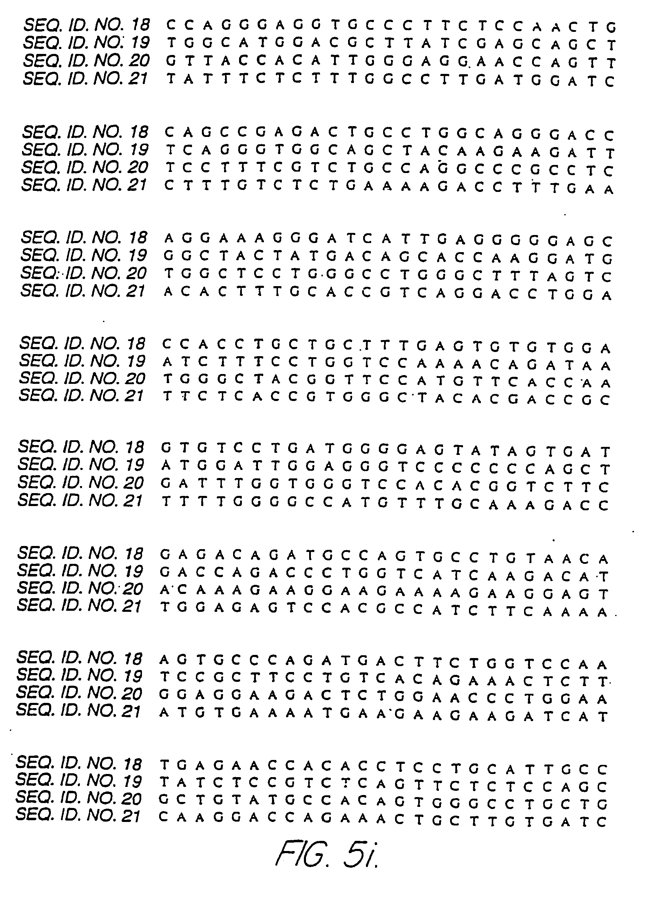

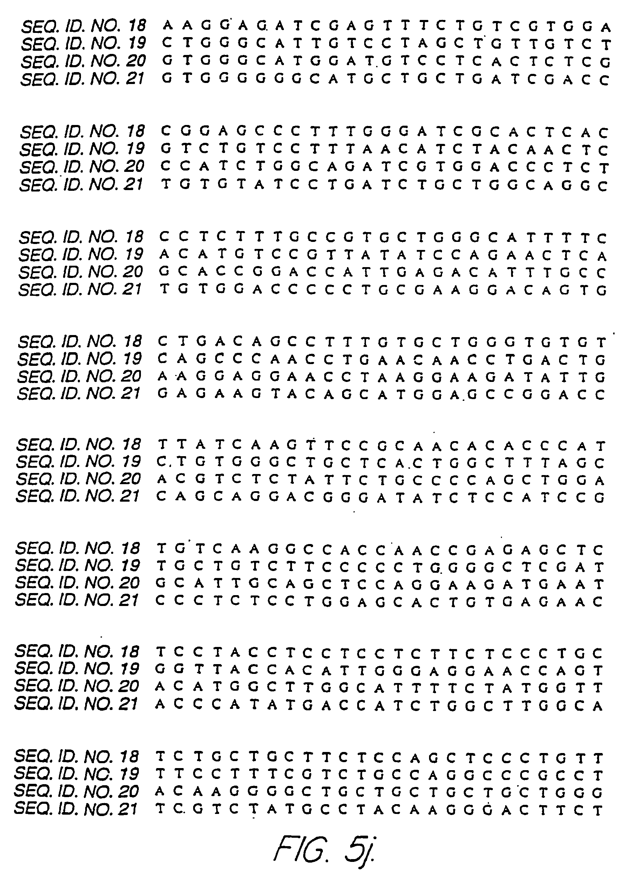

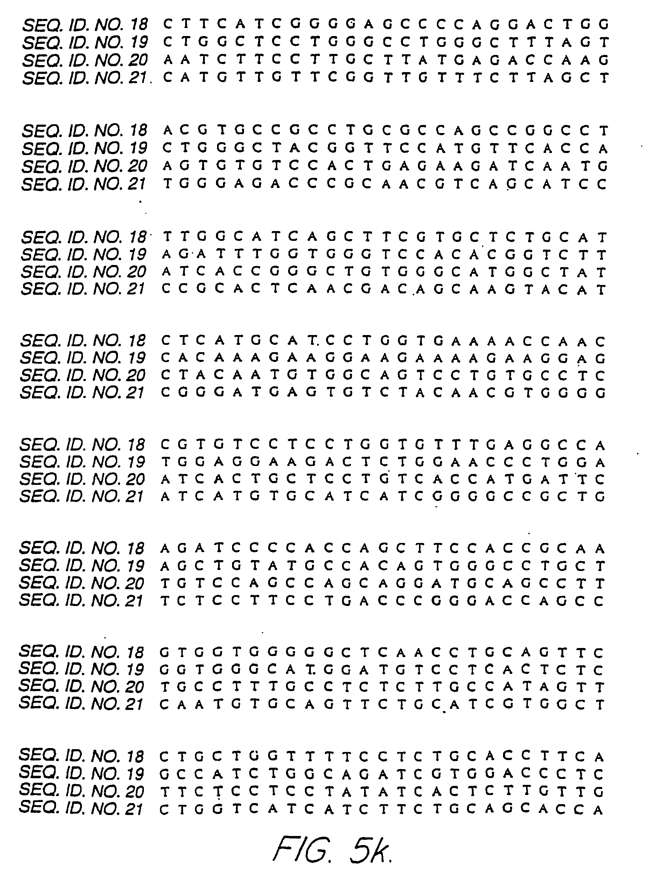

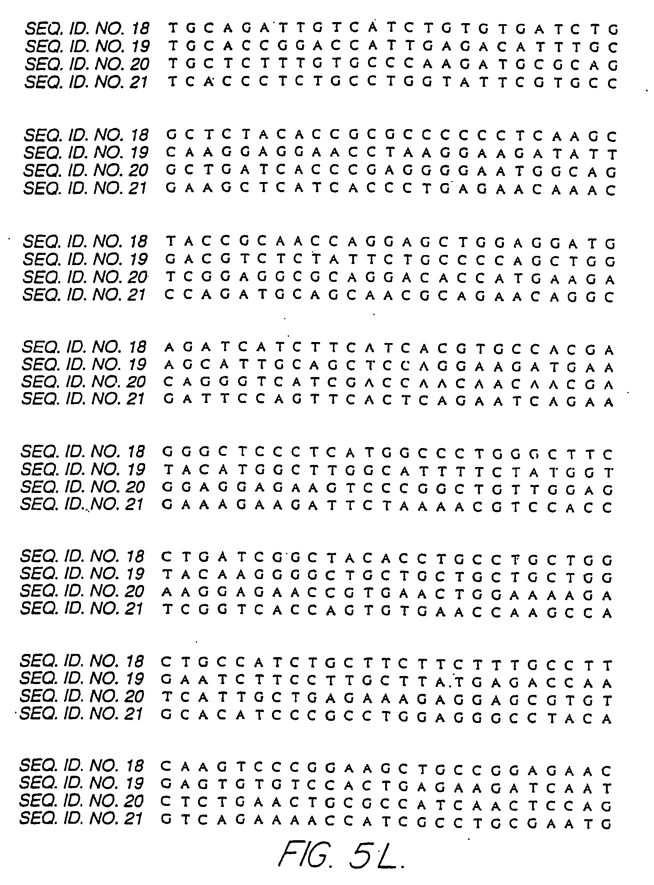

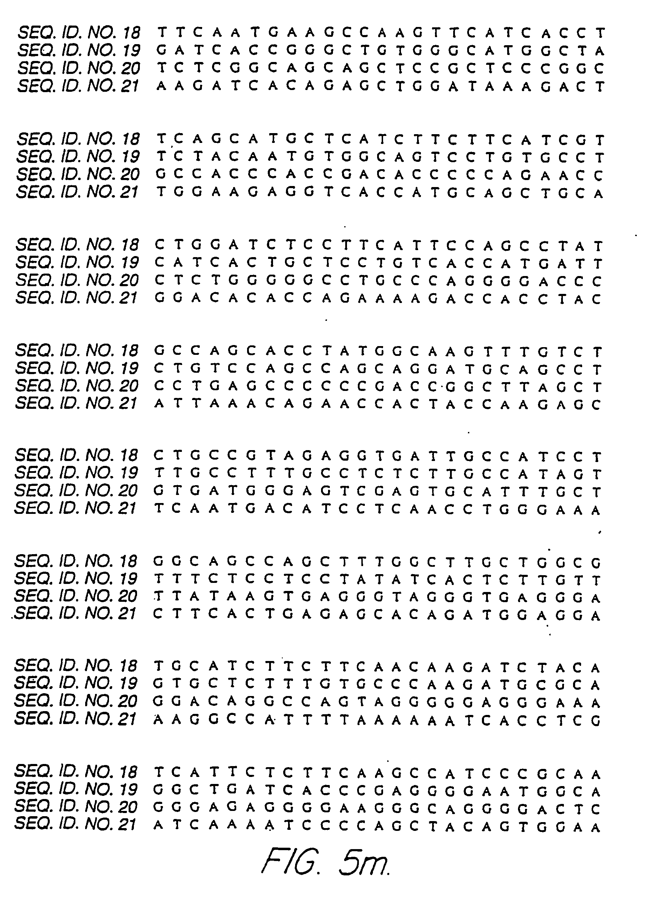

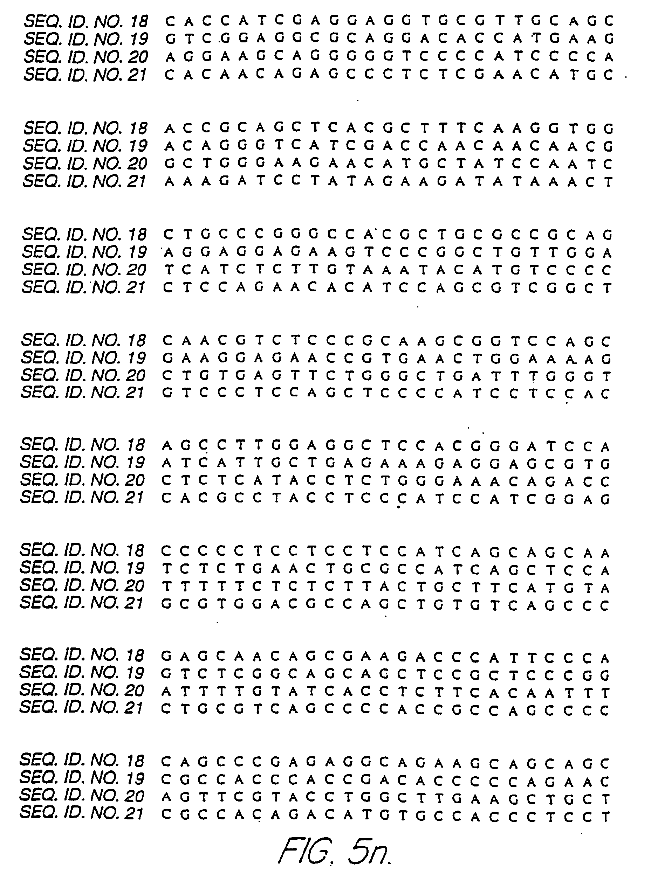

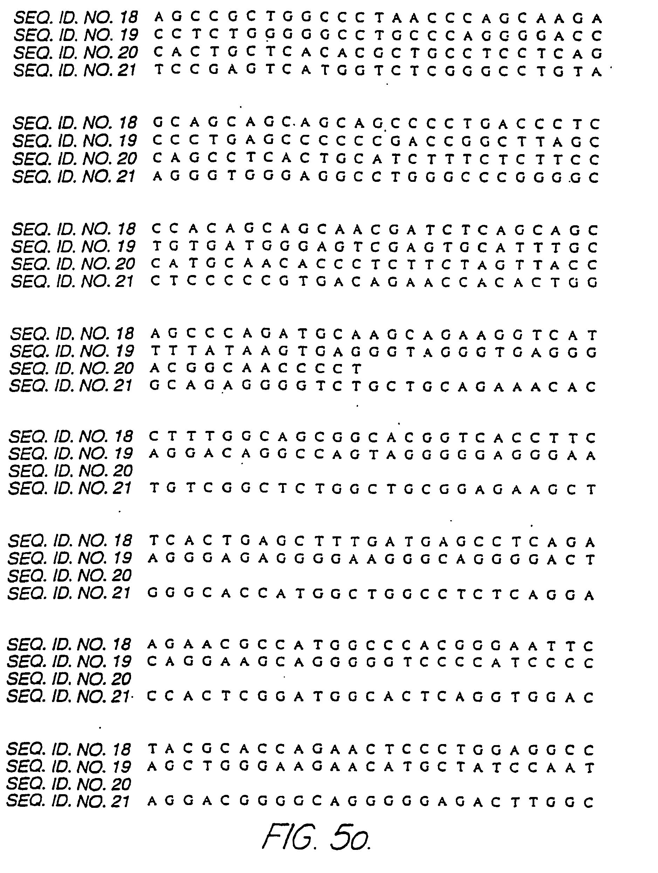

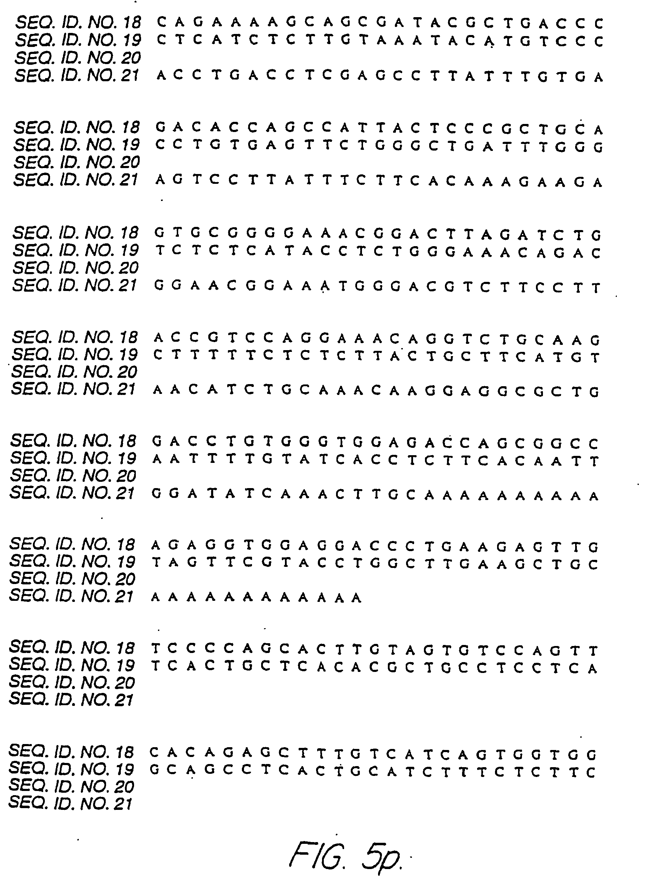

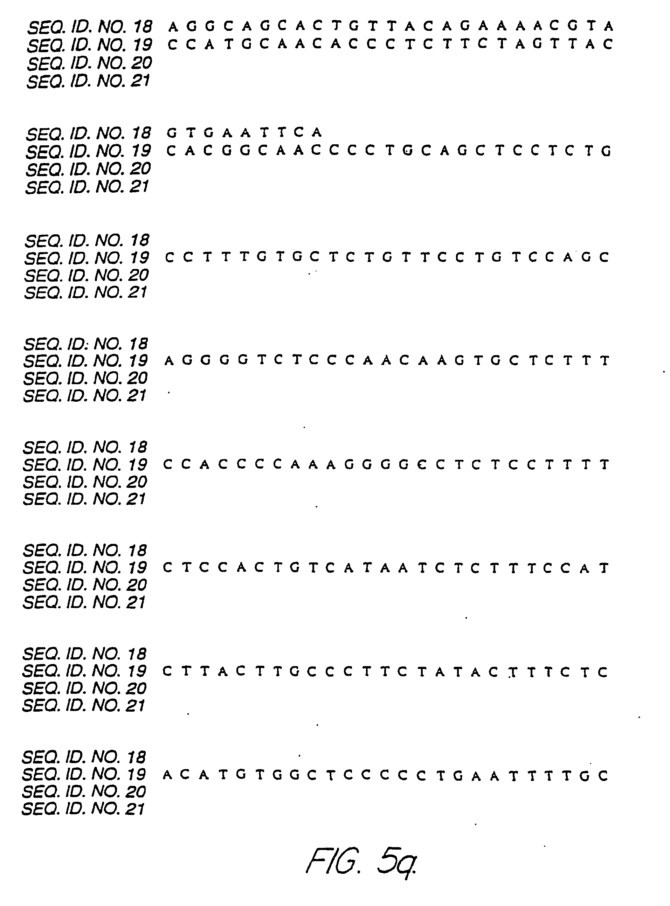

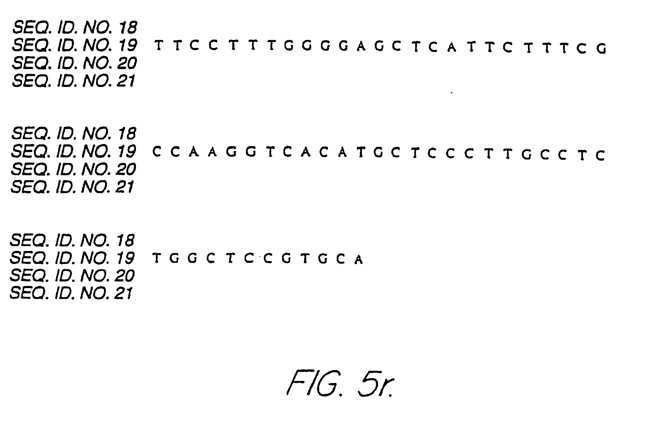

Figures 5a-5r illustrate the cDNA sequences encoding for human CaR (SBQ. ID. NO. 18), human GABA_{B}R1a (SEQ. ID. NO. 19), human GABA_{B}R1b (SEQ. ID. NO. 20), and human GABA_{B}R2 (SEQ.ID. NO.21).

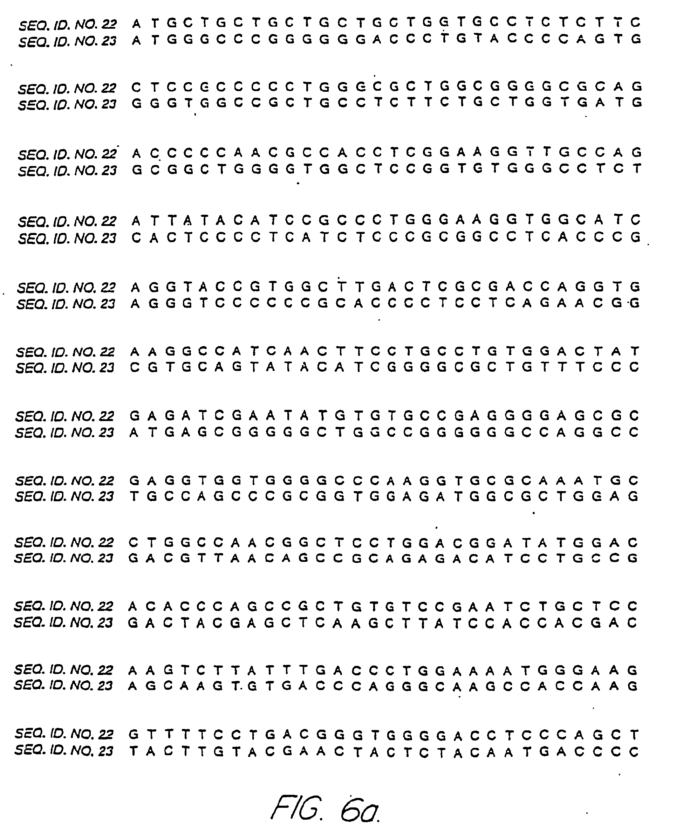

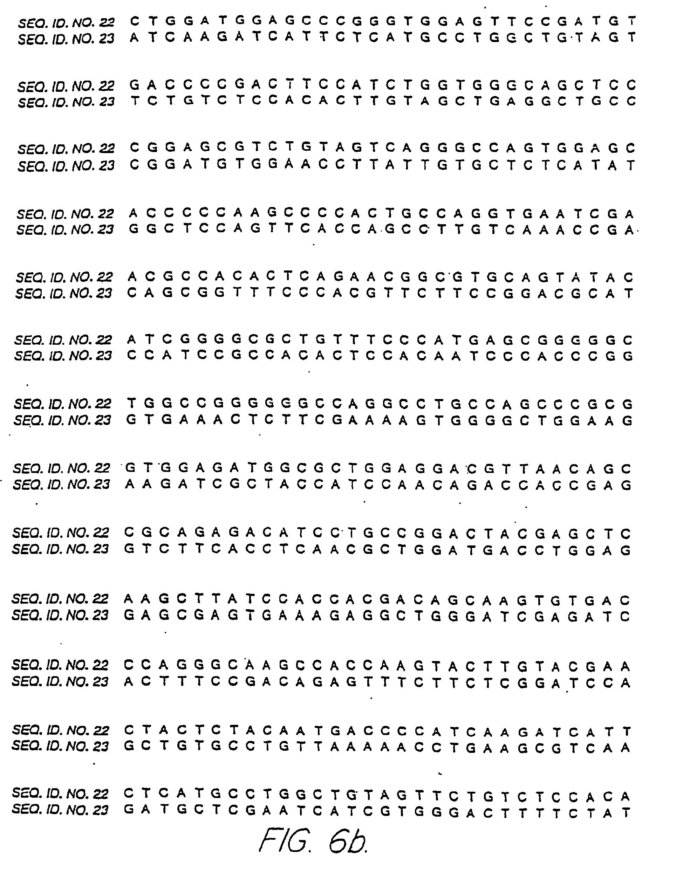

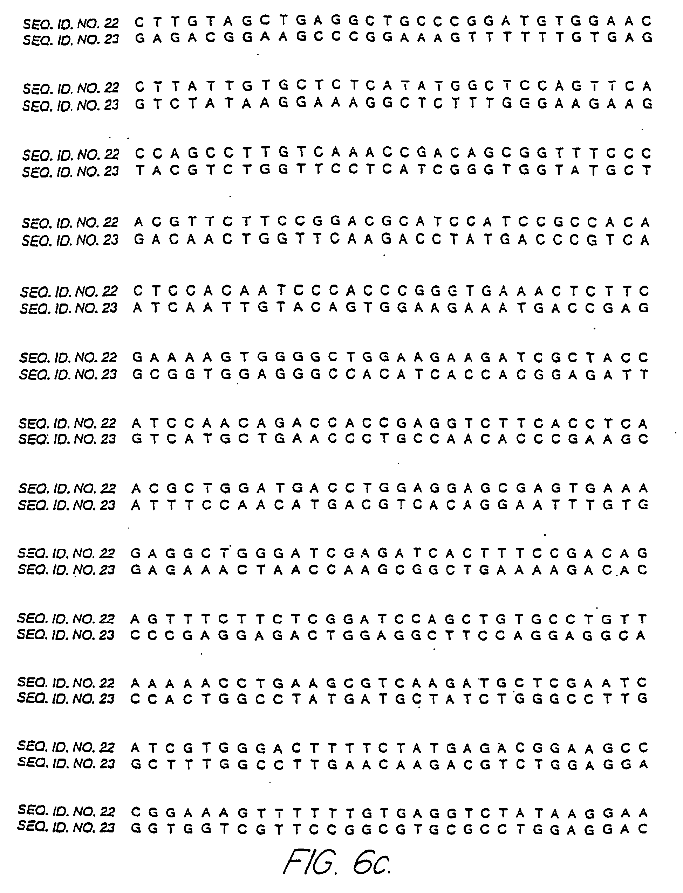

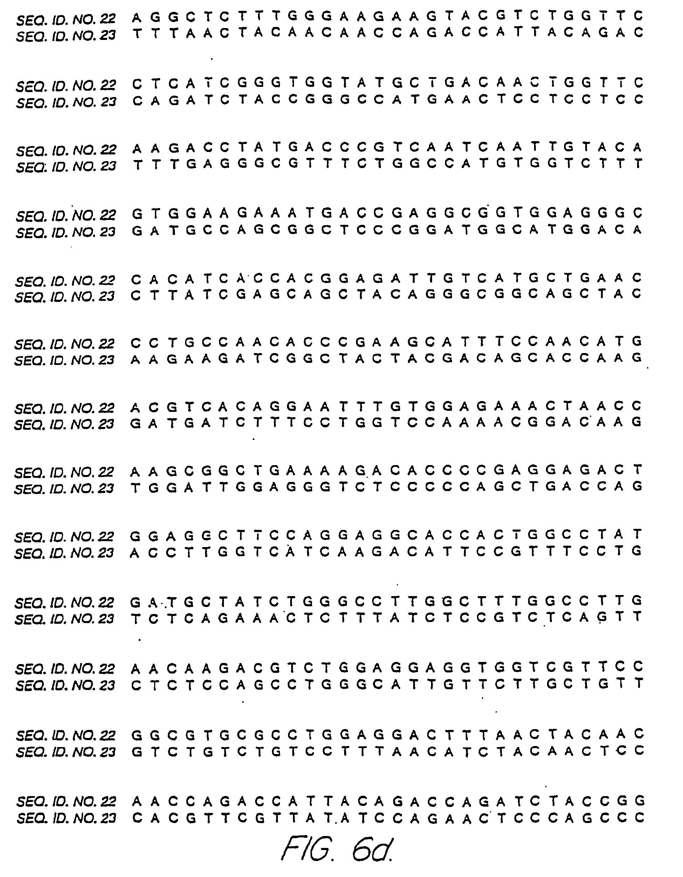

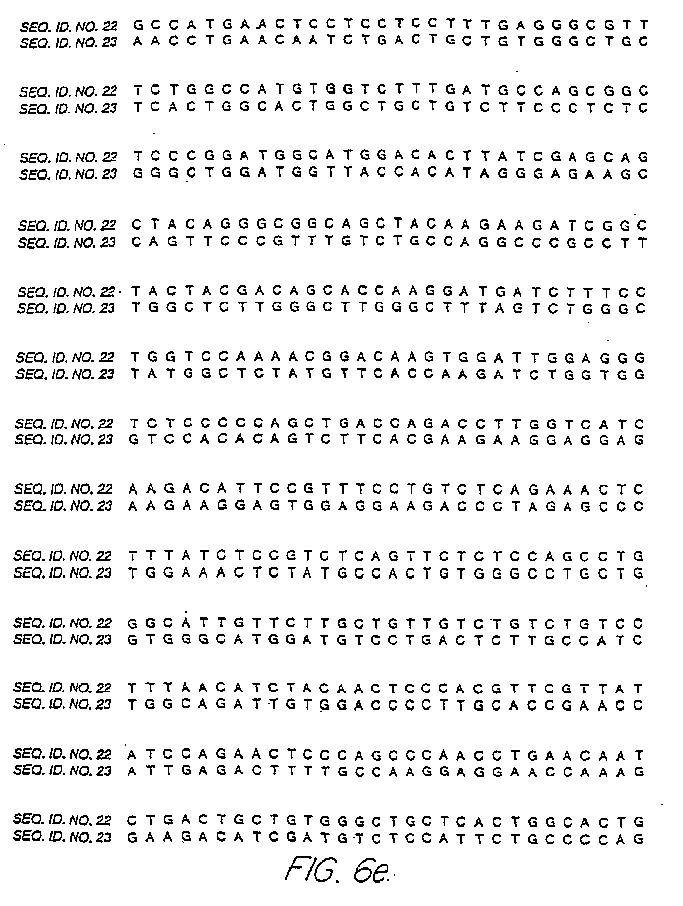

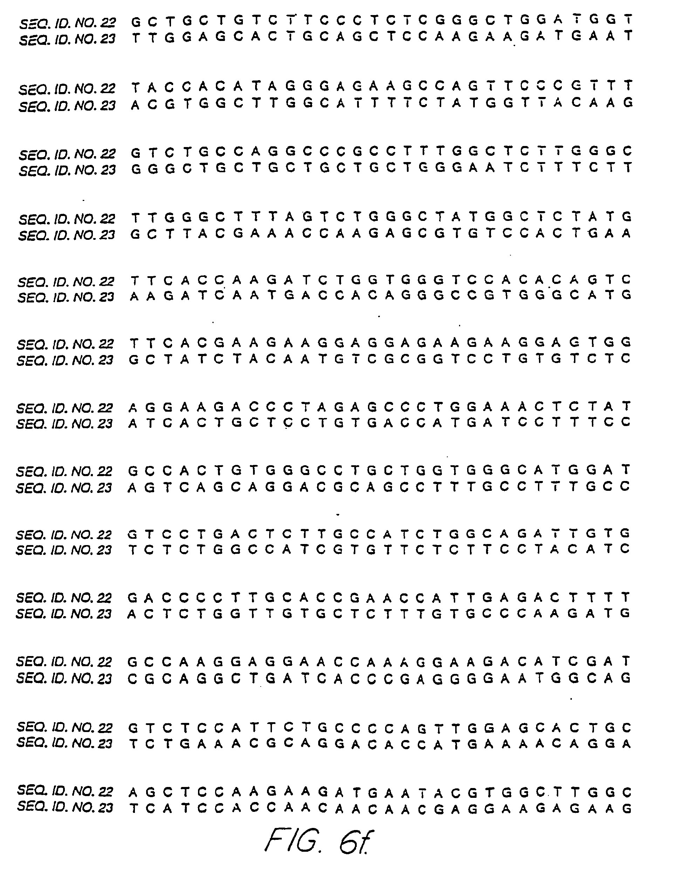

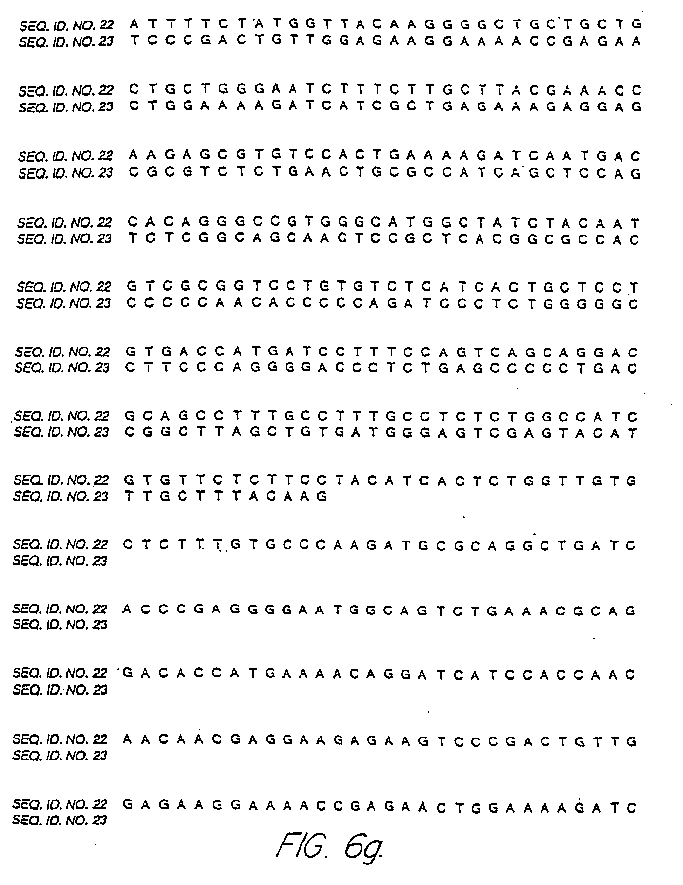

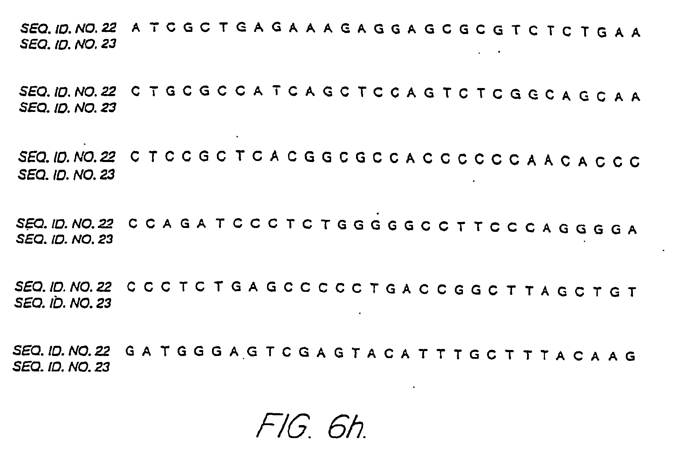

Figures 6a-6h illustrate the cDNA sequence for rat GABA_{B}R1a (SBQ. ID. NO. 22) and rat GABA_{B}R1b (SBQ. ID. NO. 23).
Figures 7a-7c illustrate the amino sequence for rat GABA_{B}R1a (SEQ. ID. NO. 24) and rat GABA_{B}R1b (SBQ.ID. NO. 25).
Figure 8 illustrates the ability of a chimeric CaR/GABA_{B}R2 (CaR extracellular and transmembrane domains, and intracellular GABA_{B}R2 domain) to transduce a signal. Signal production was measured by detecting an increase in the calcium-activated chloride current The line in the middle of the increase signifies a wash step.

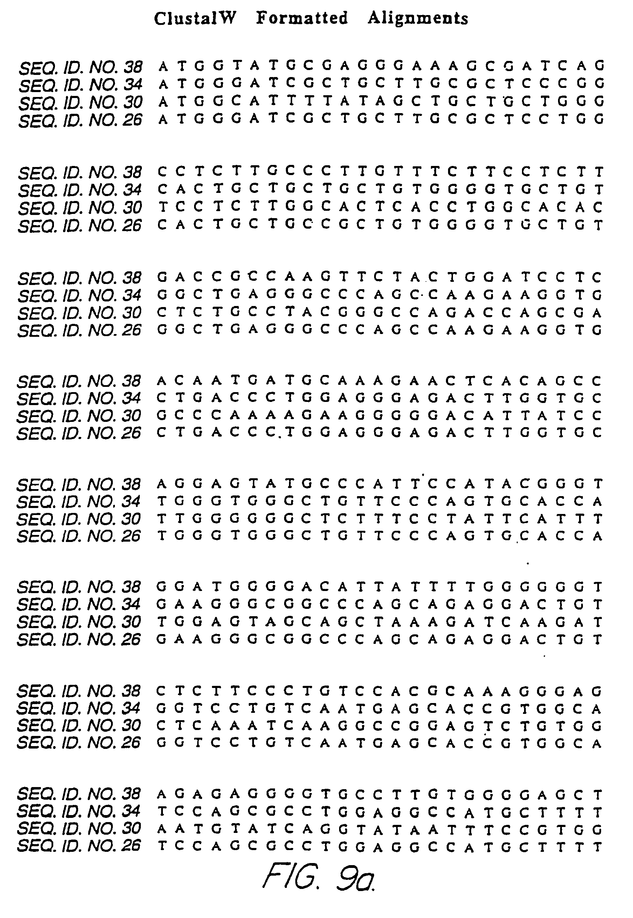

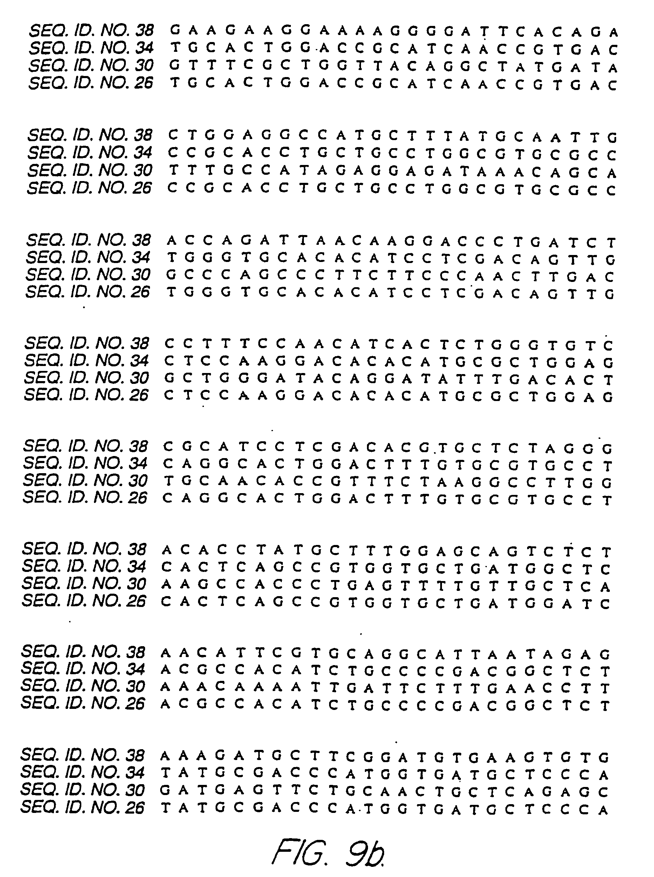

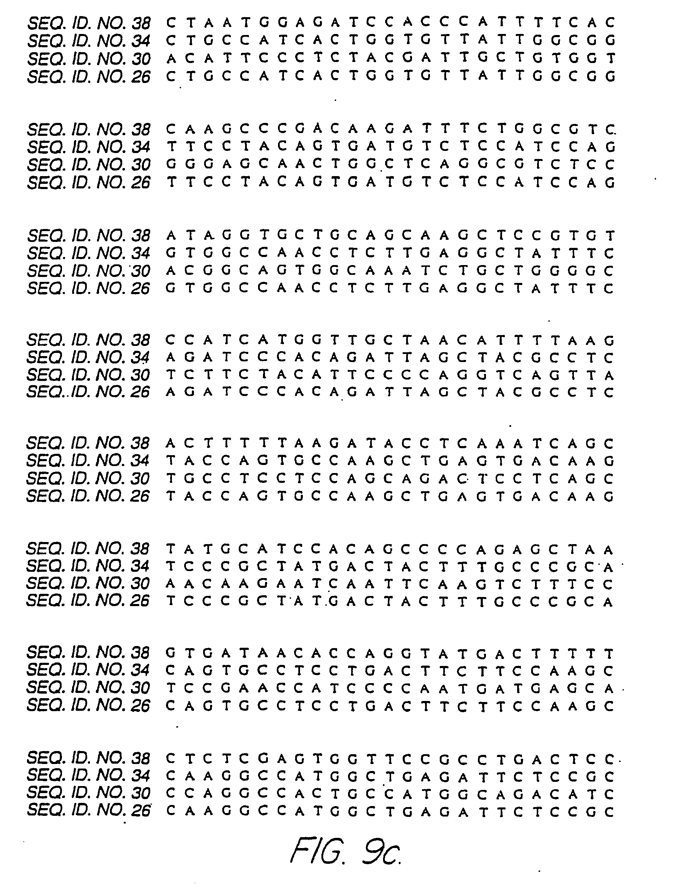

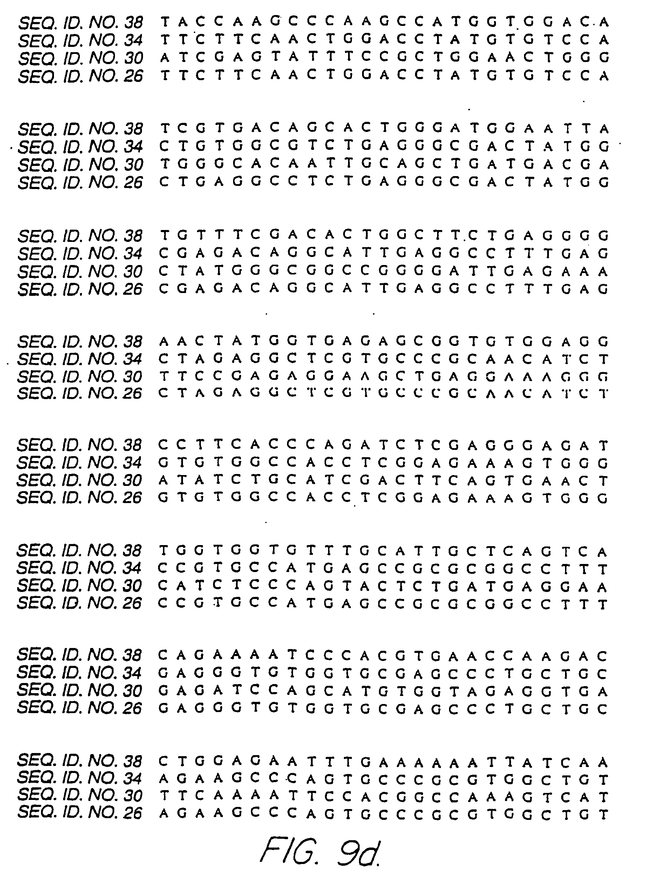

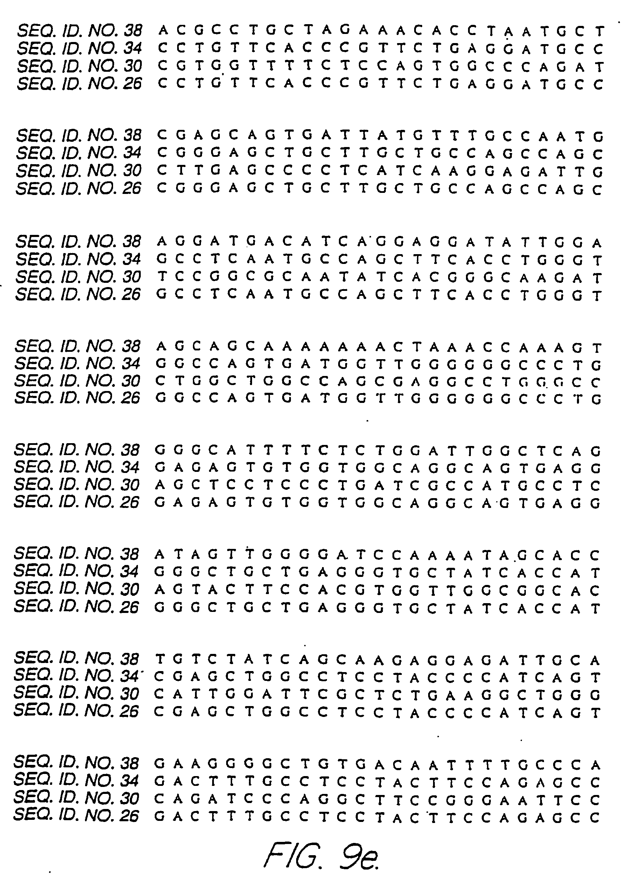

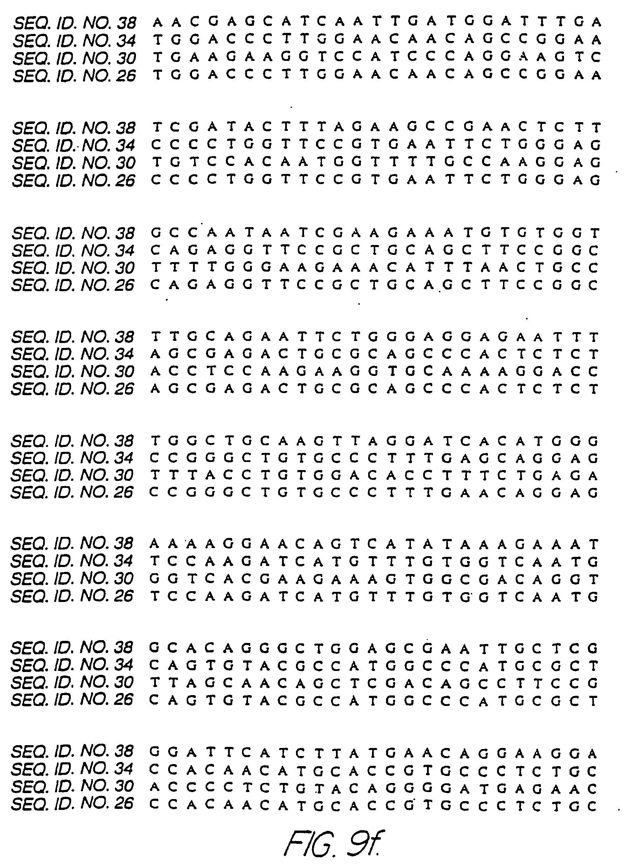

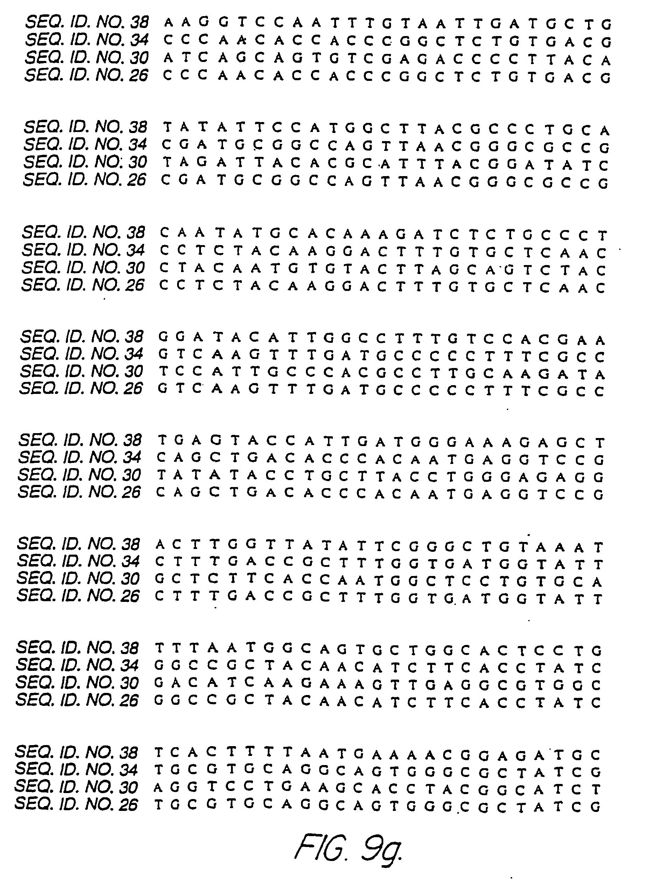

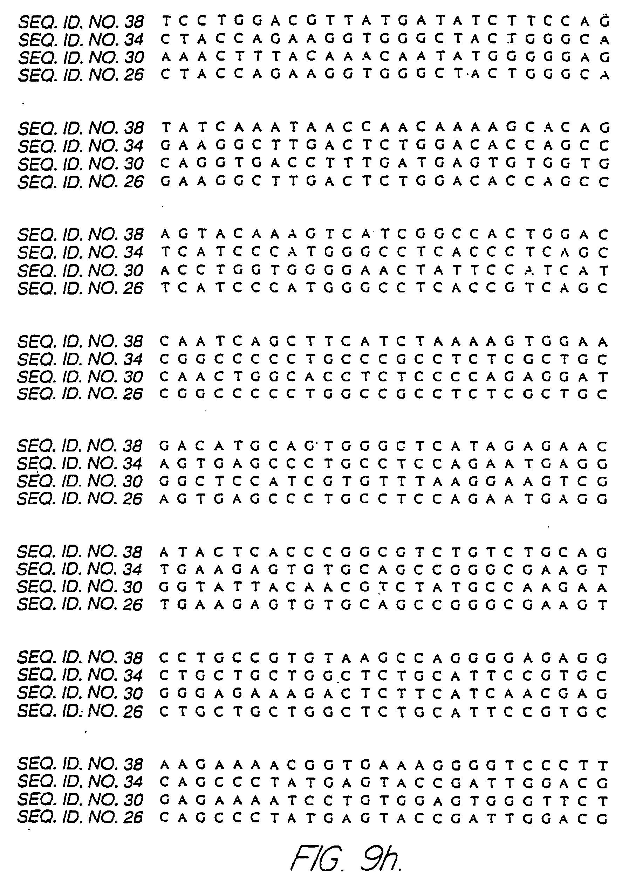

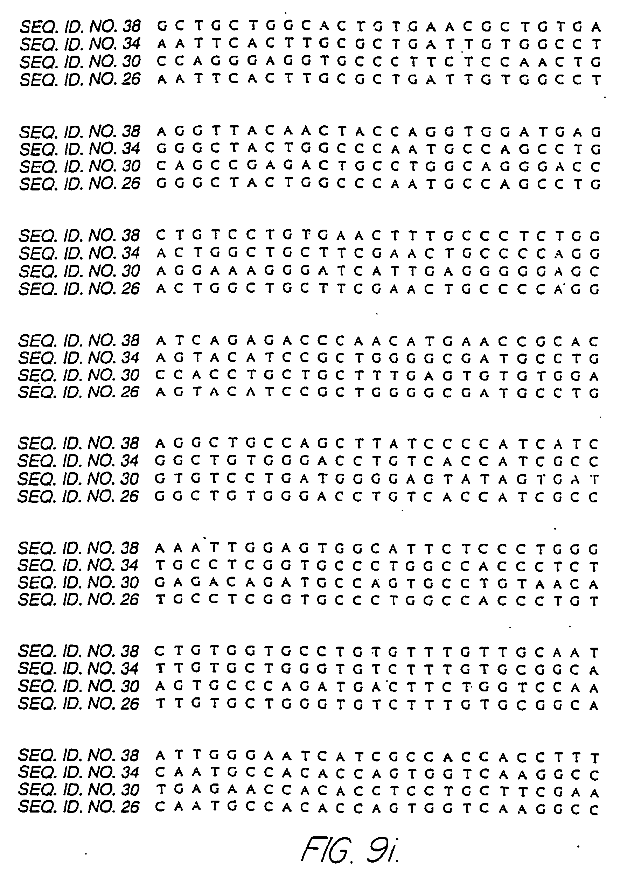

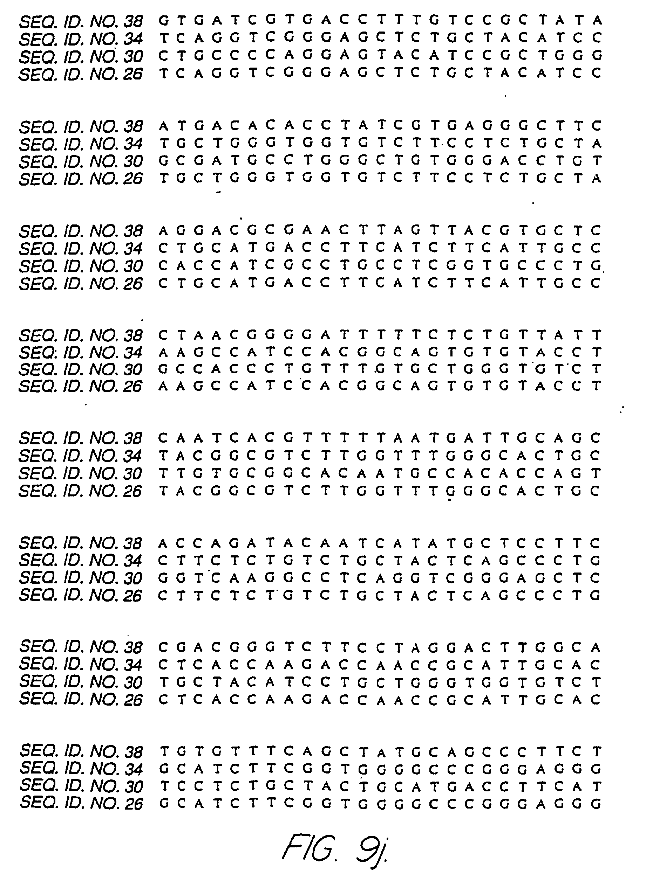

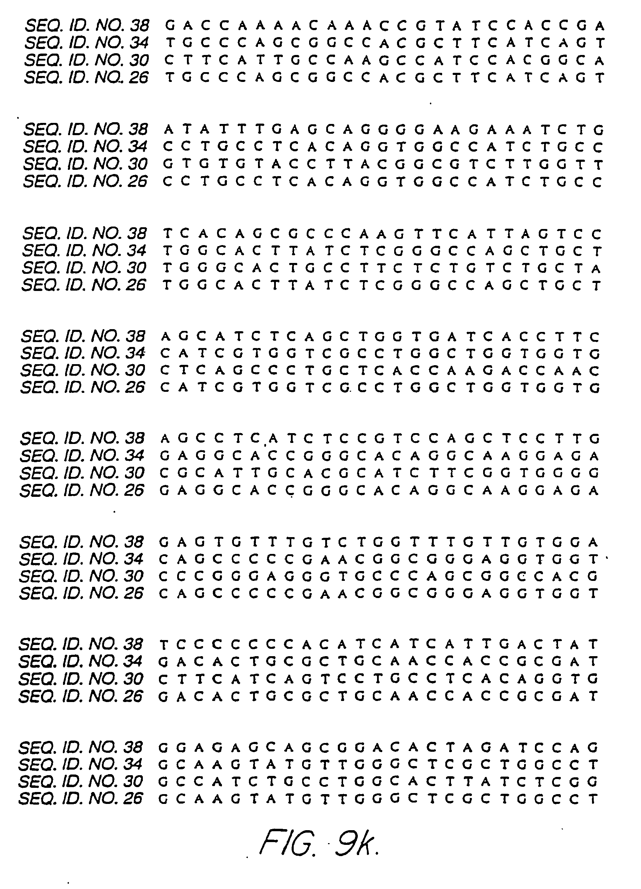

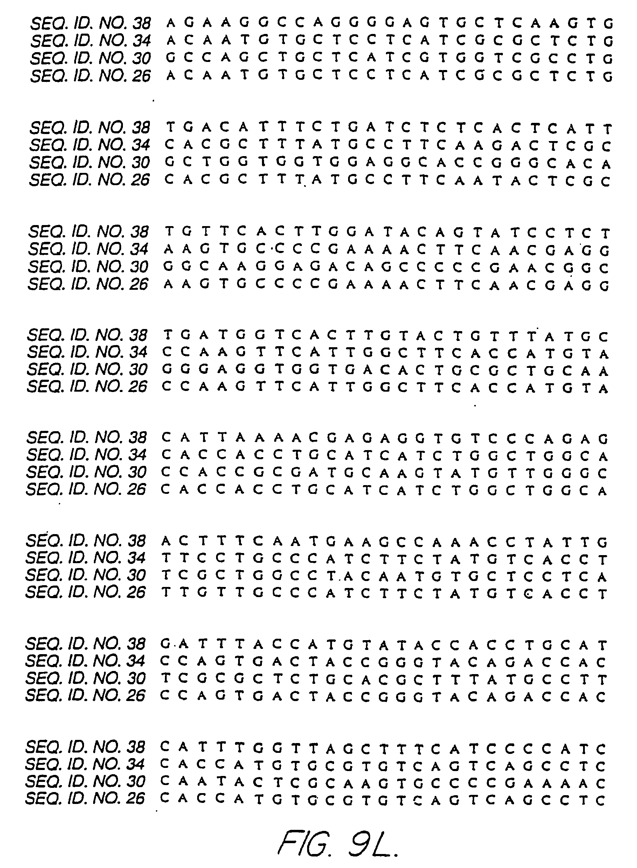

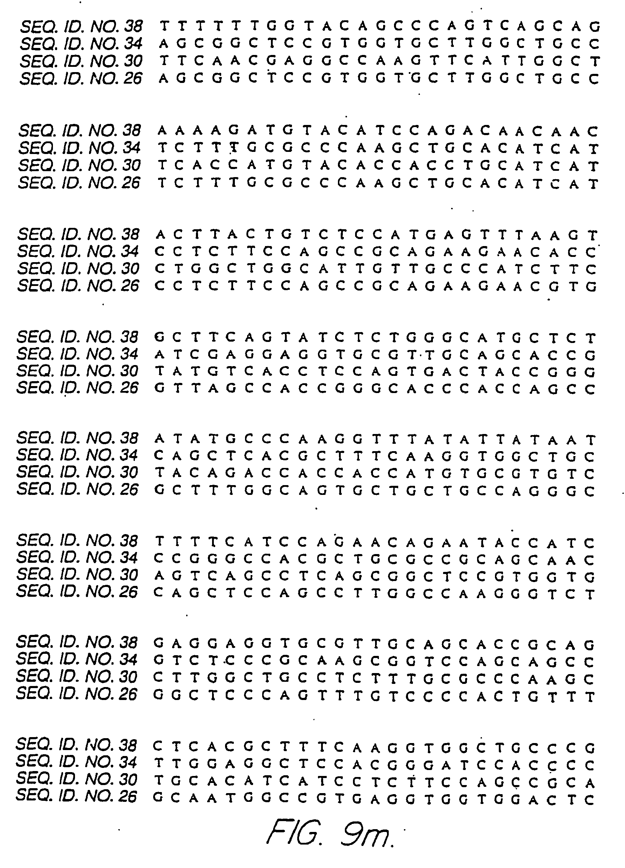

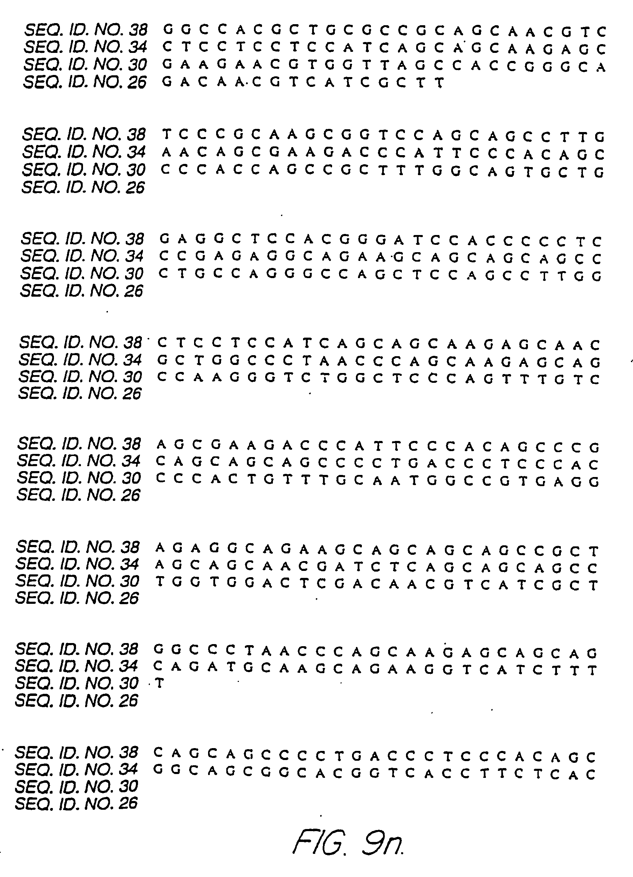

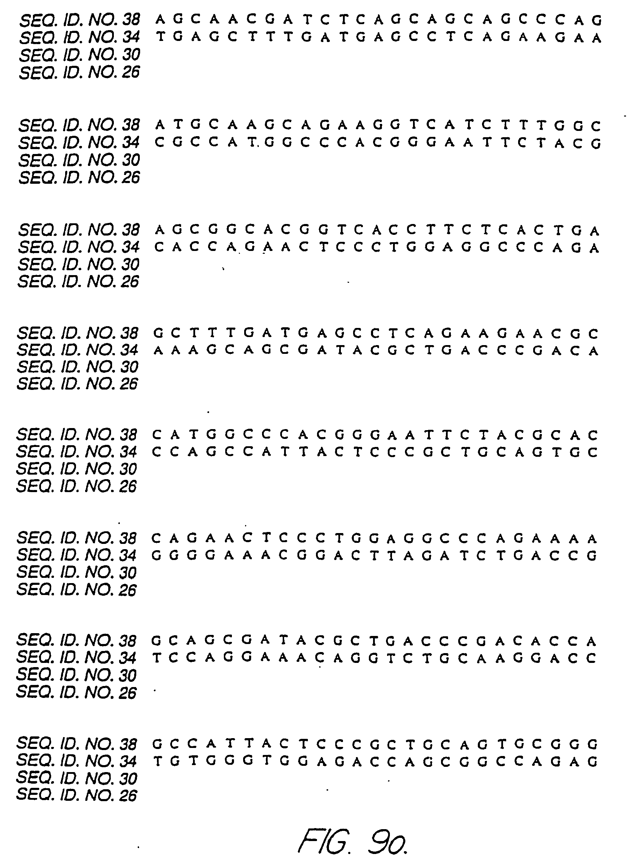

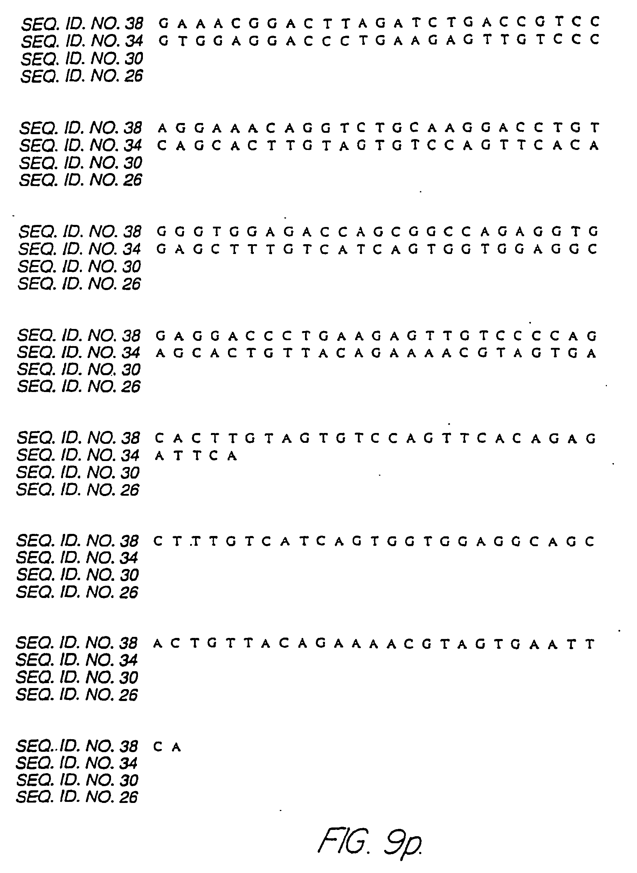

Figures 9a-9p illustrate the cDNA sequence for human mGlaR2 (SEQ, ID. NO. 26), chimeric hCAR/hmGluR2 (SEQ. ID. NO. 30), chimeric hmGluR2/hCaR (SEQ. ID. NO. 34), and chimeric hmGluR8/hCaR (SEQ. ID. NO. 38).

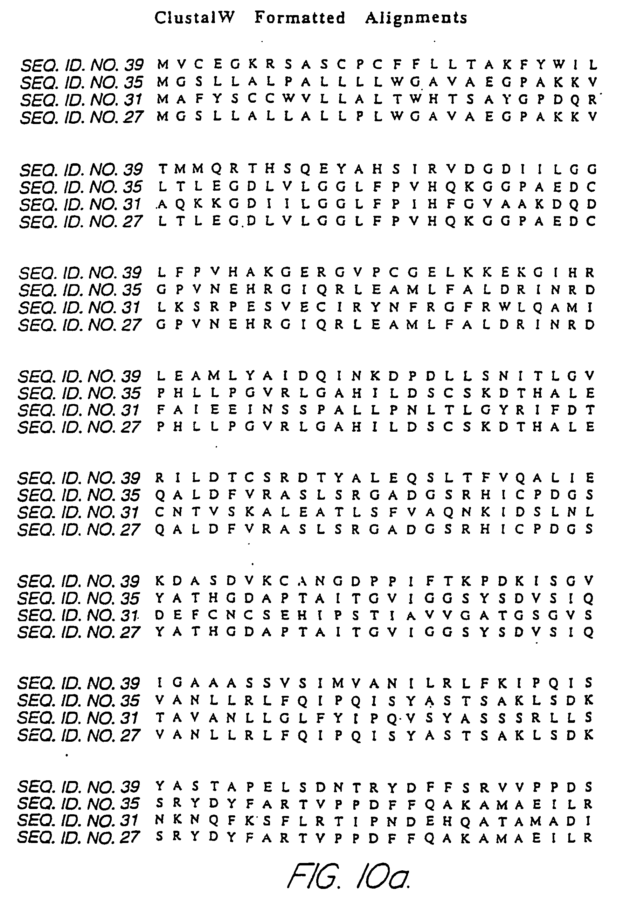

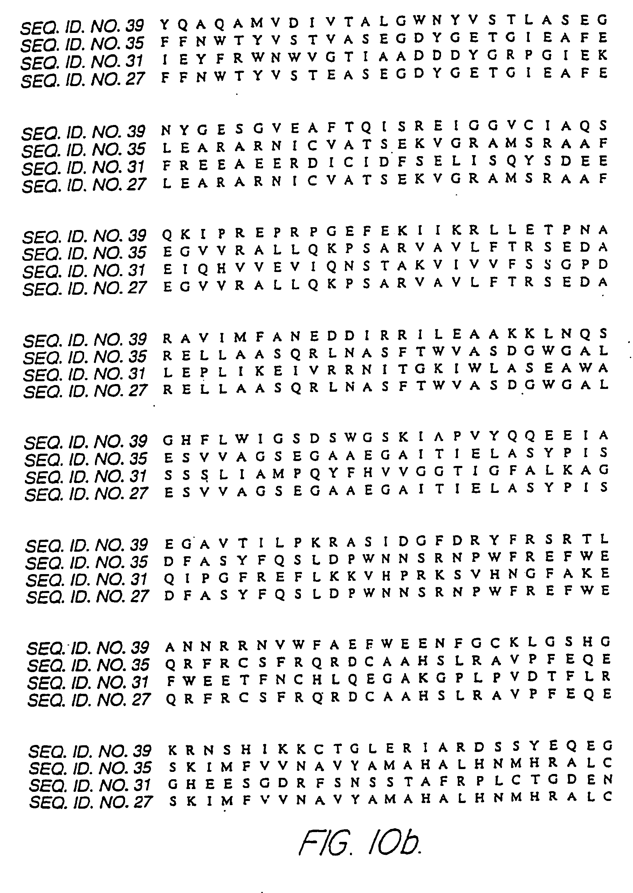

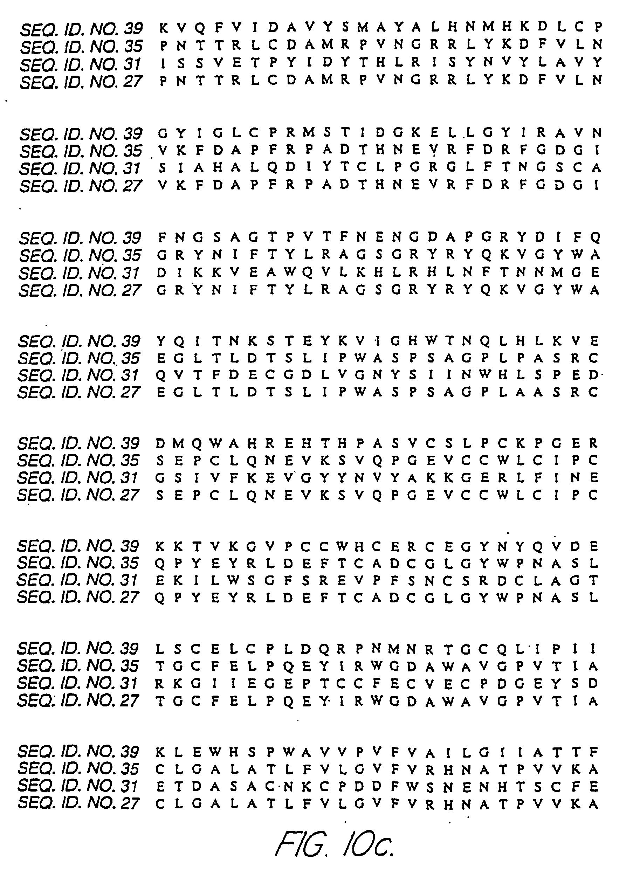

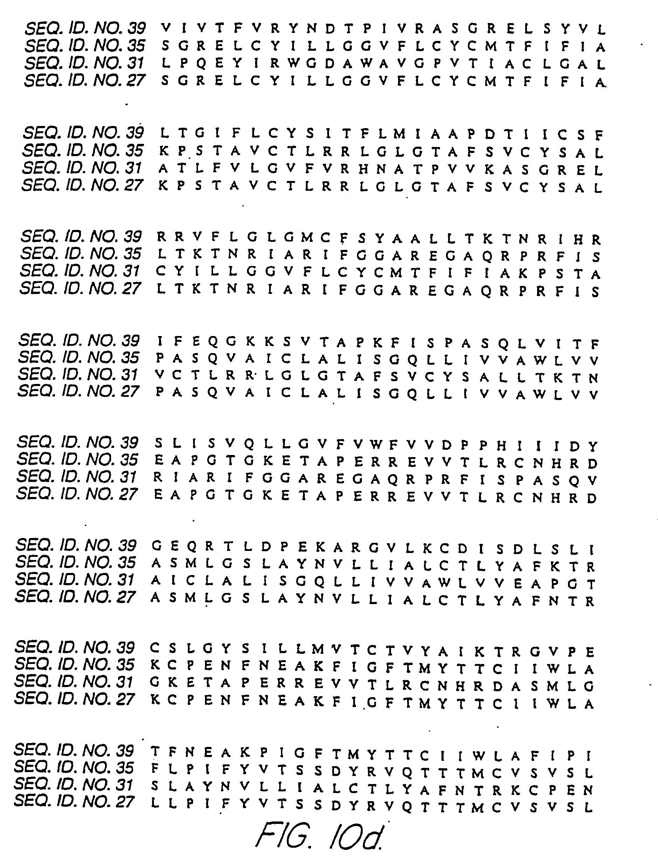

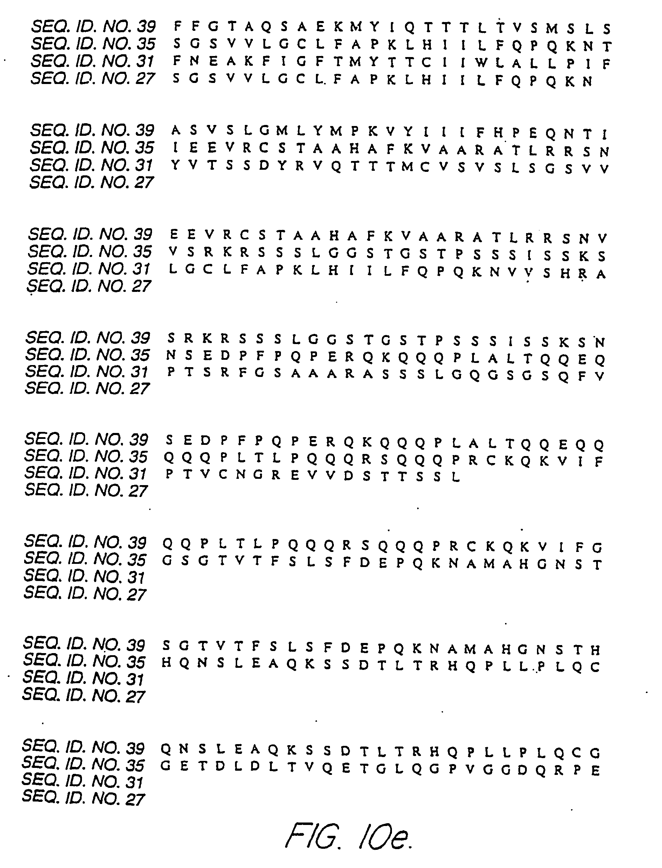

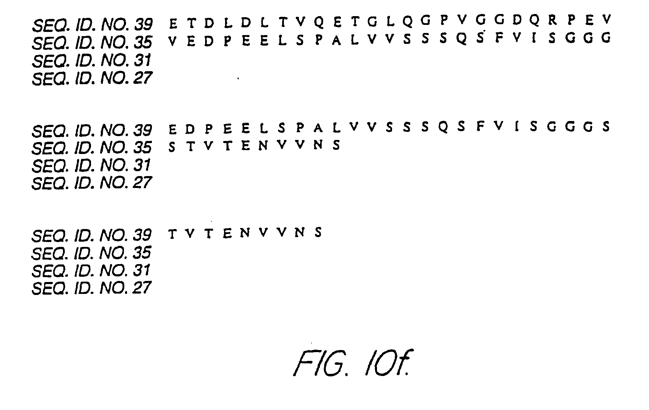

Figures 10a-10f illustrate the amino acid sequence for human mGluR2 (SEQ. ID. NO. 27), chimeric hCAR/hmGluR2 (SEQ. ID. NO. 31), chimeric hmGluR2/hCaR (SBQ. ID. NO. 35), chimeric hmGluR8/hCaR (SEQ. ID. NO. 39).

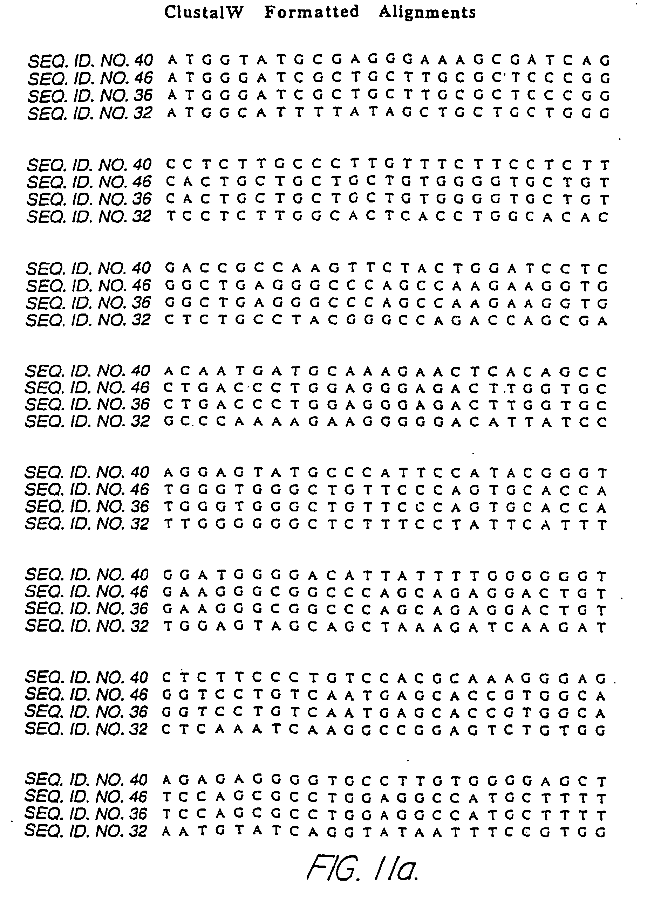

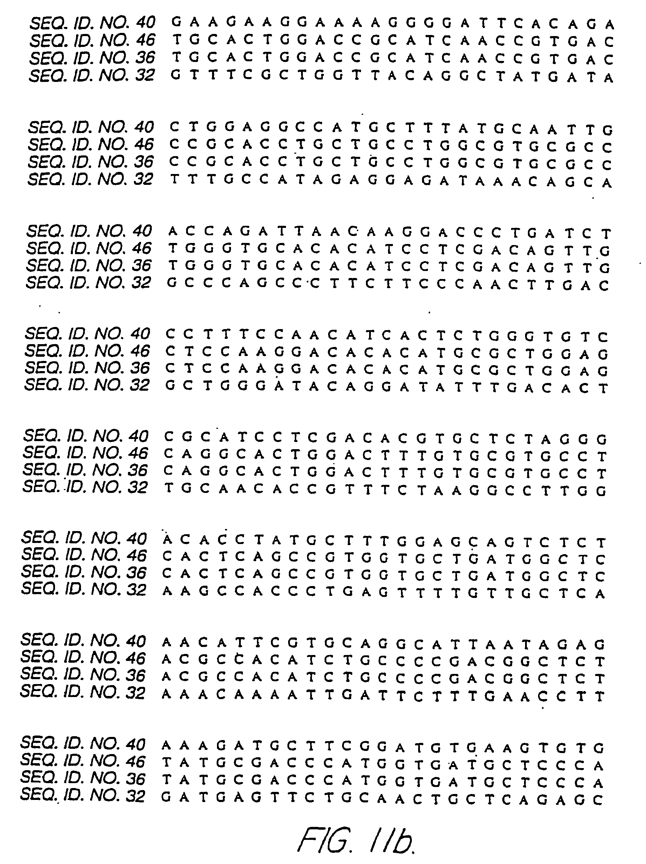

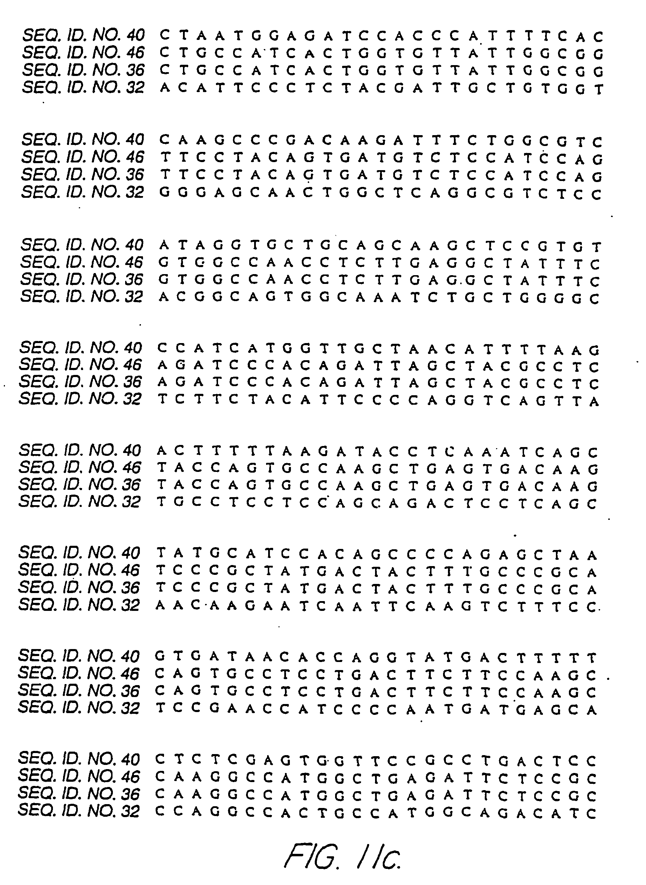

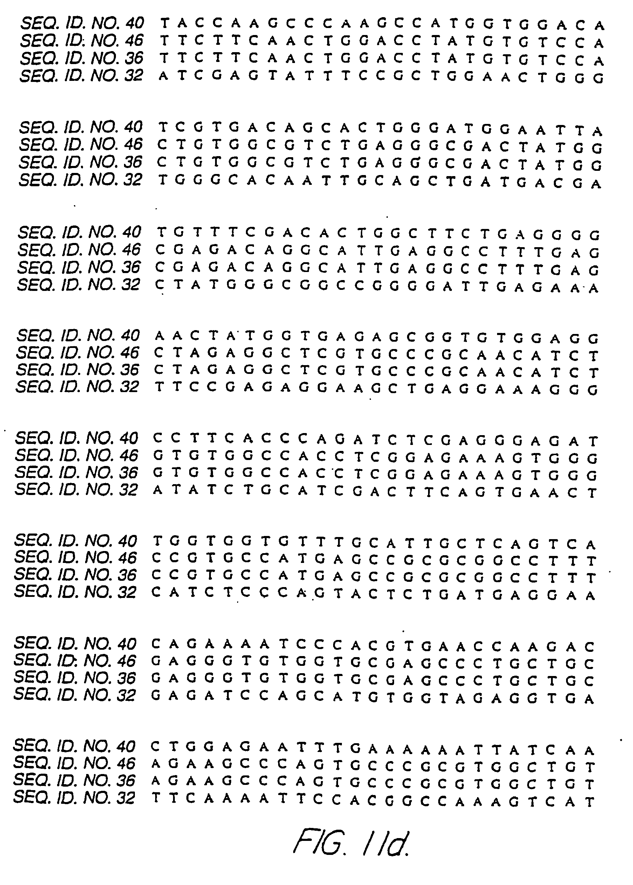

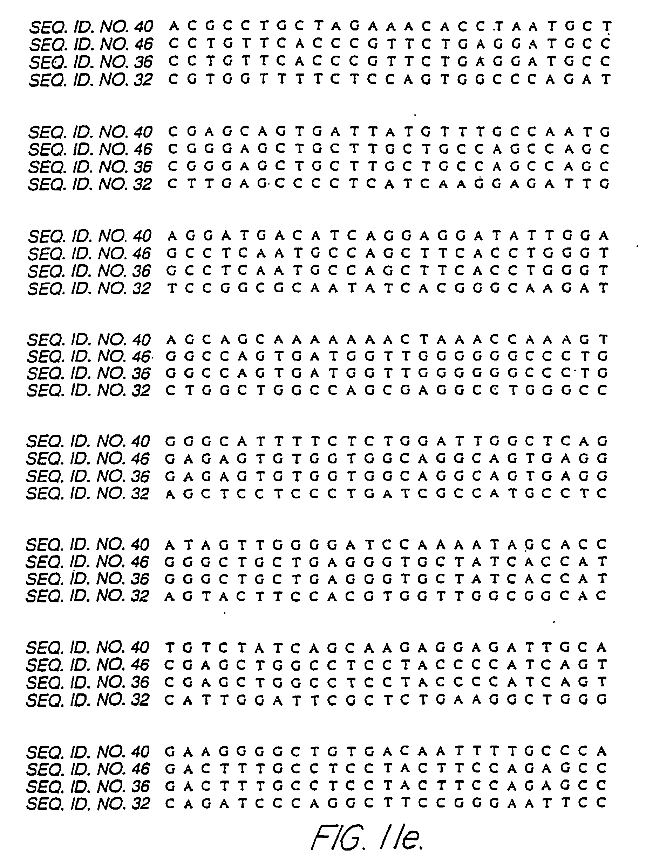

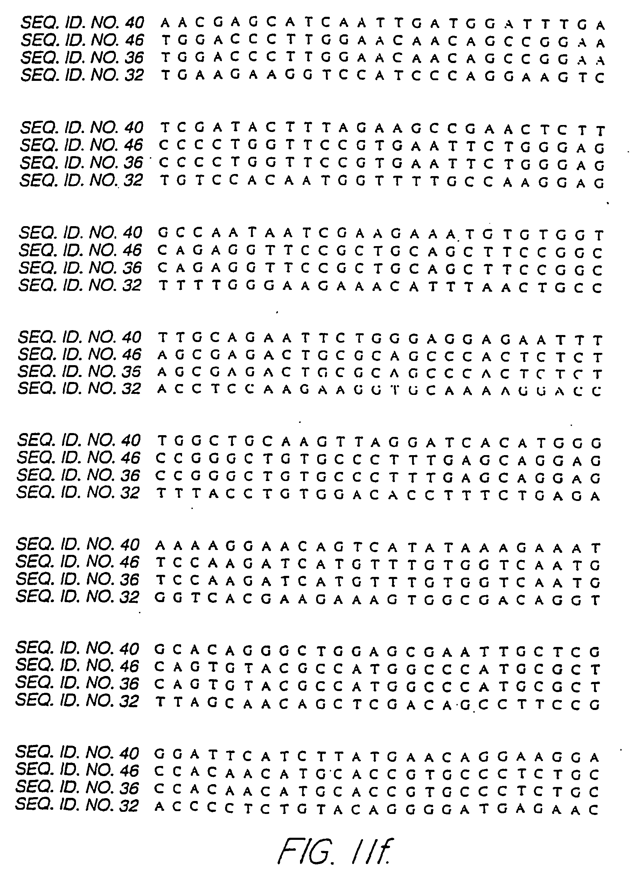

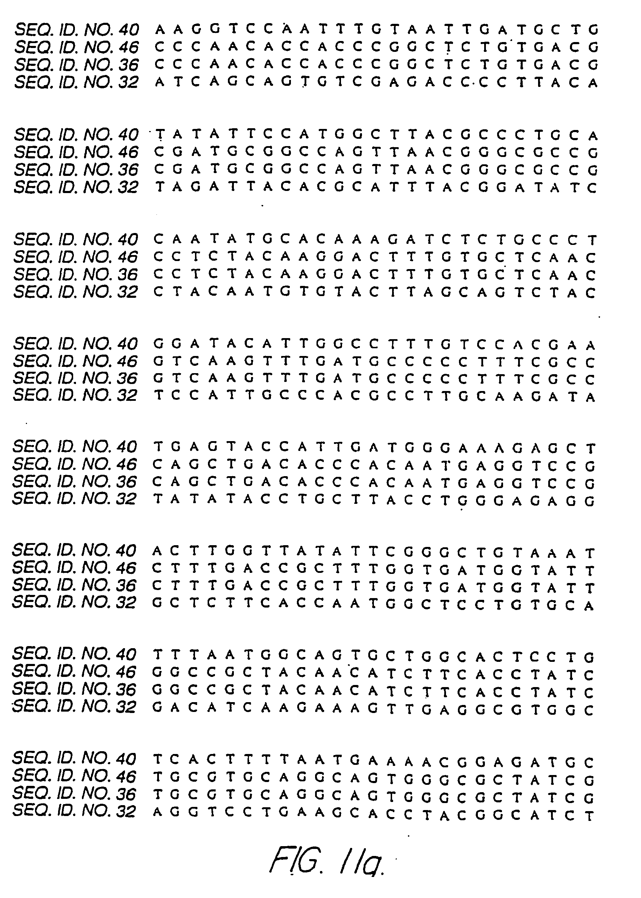

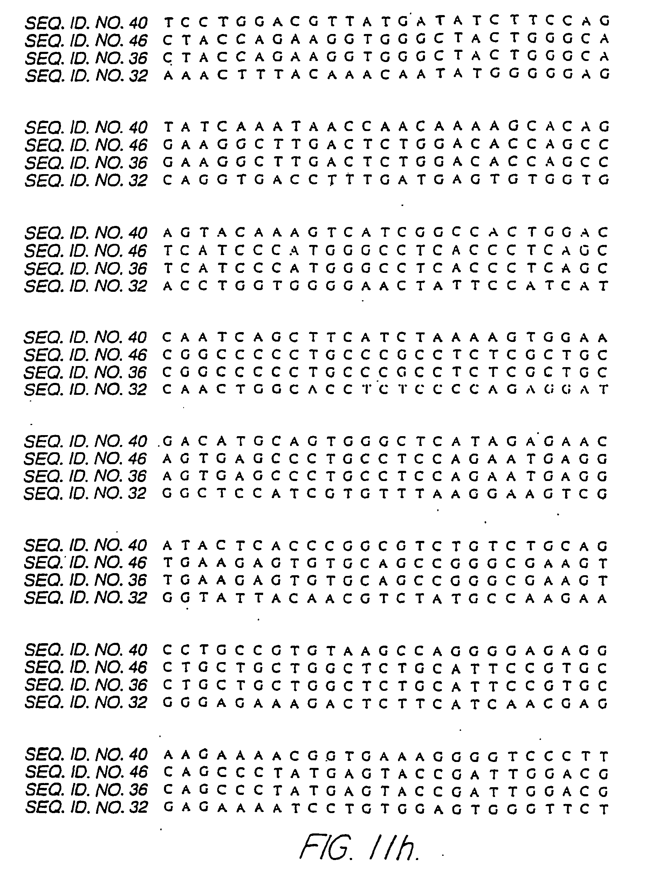

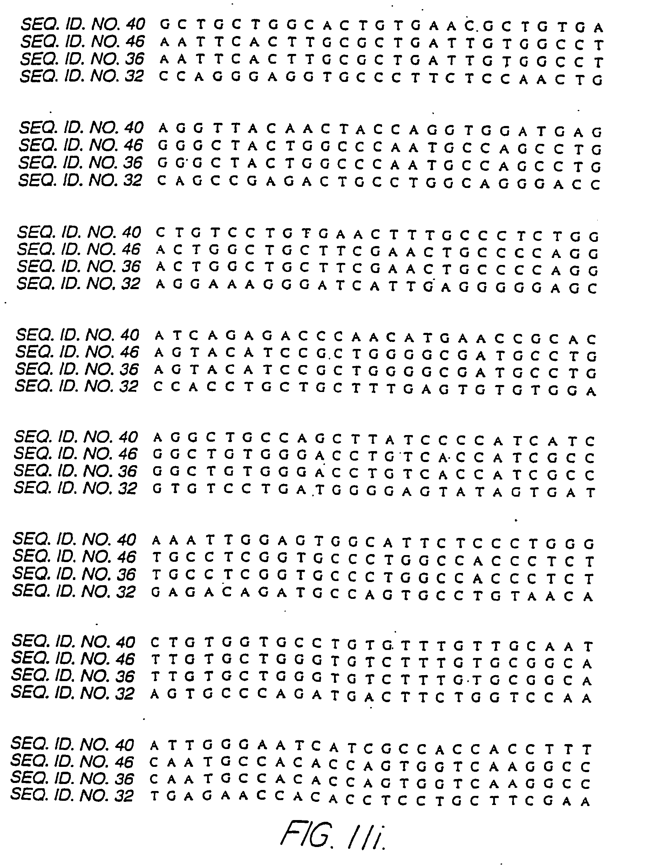

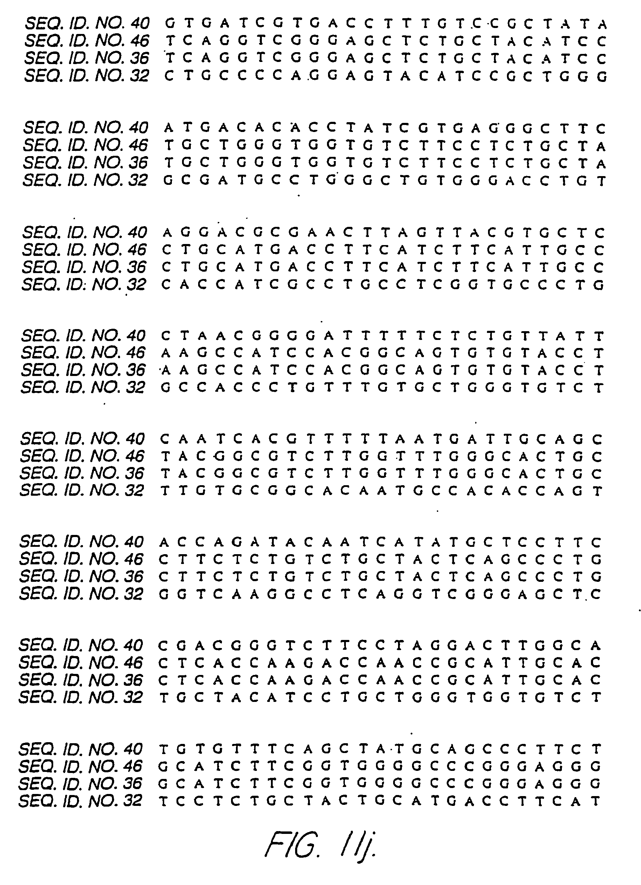

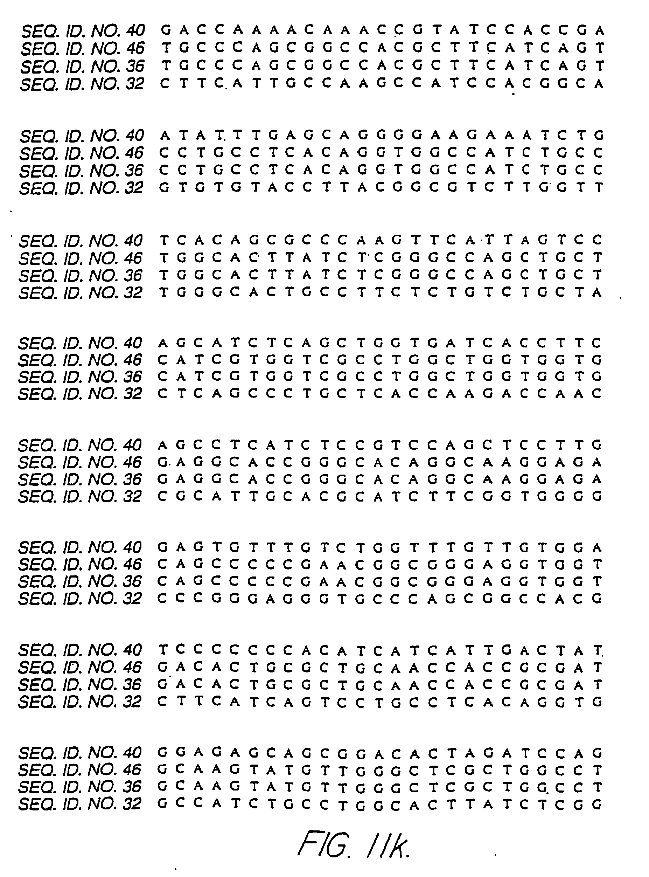

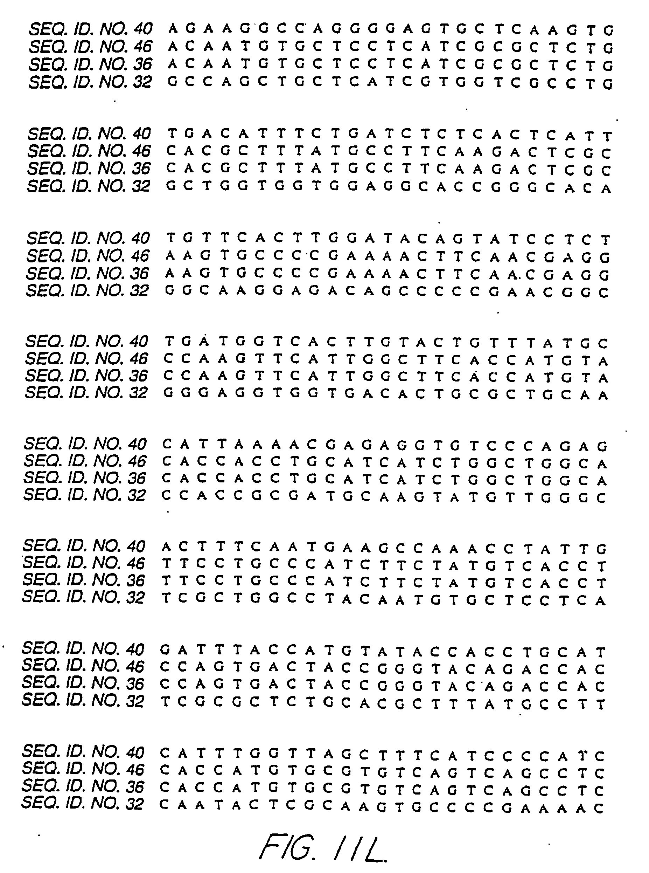

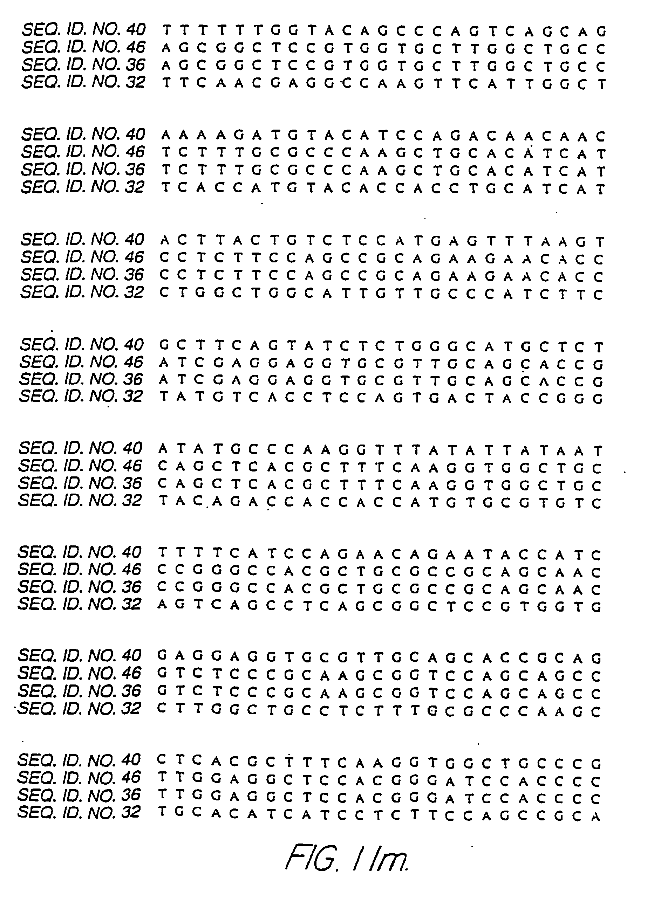

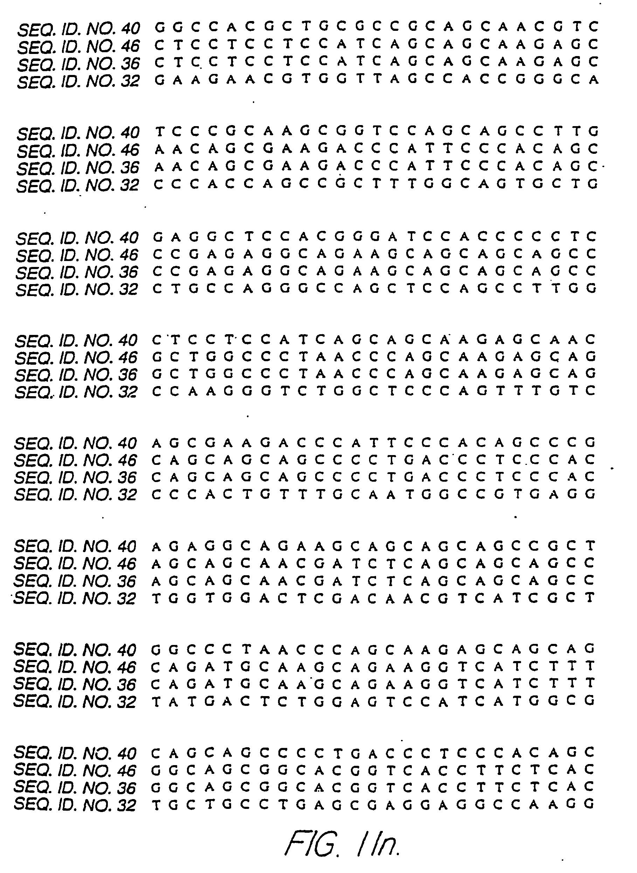

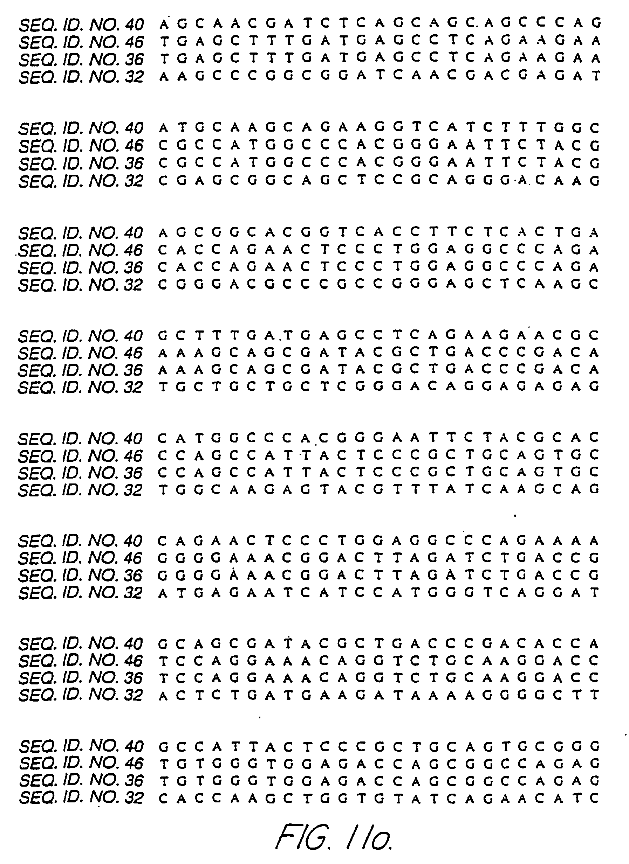

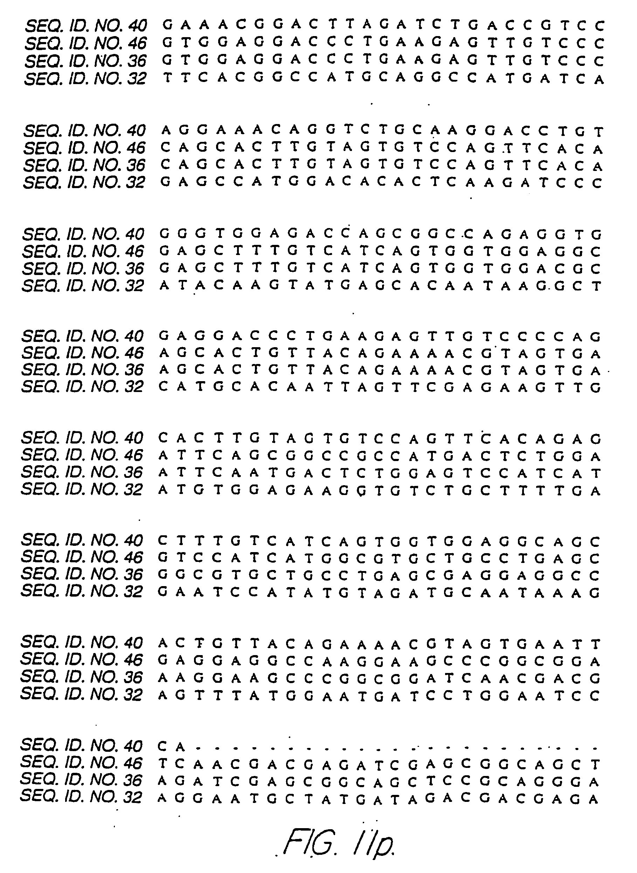

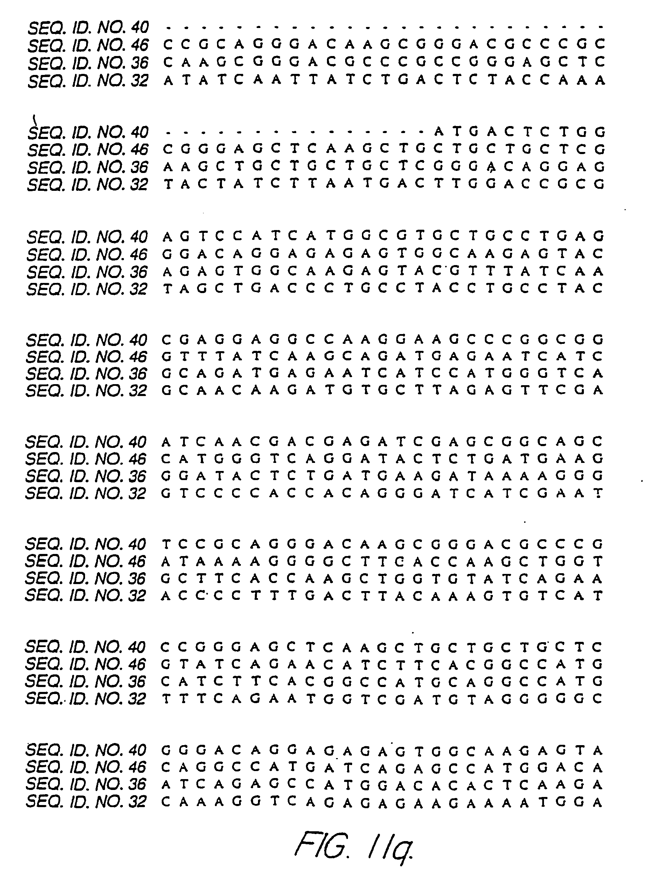

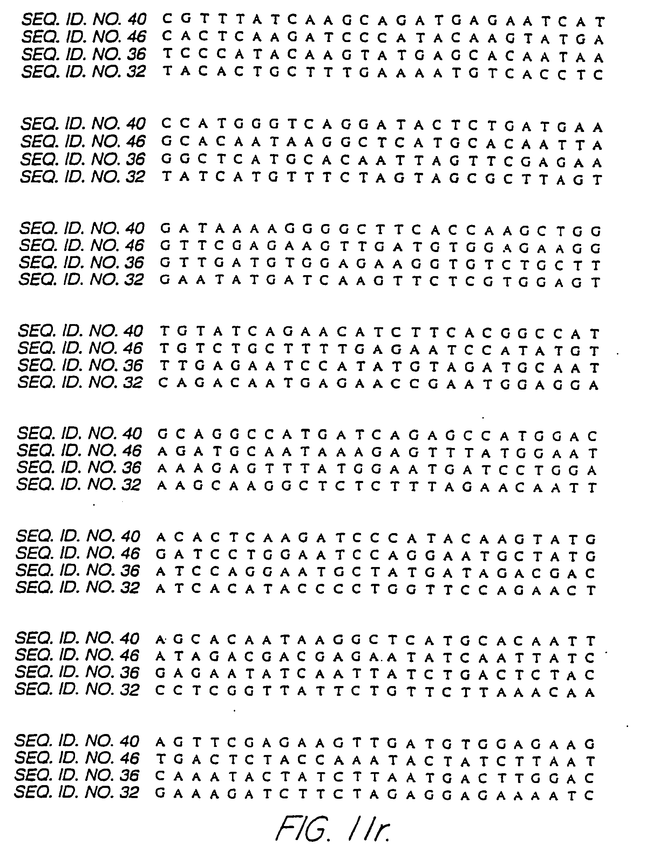

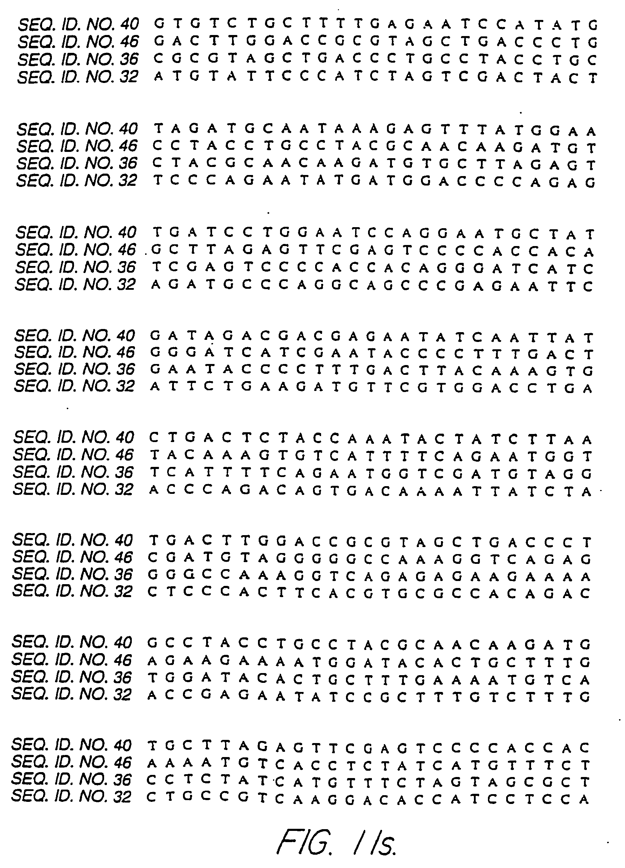

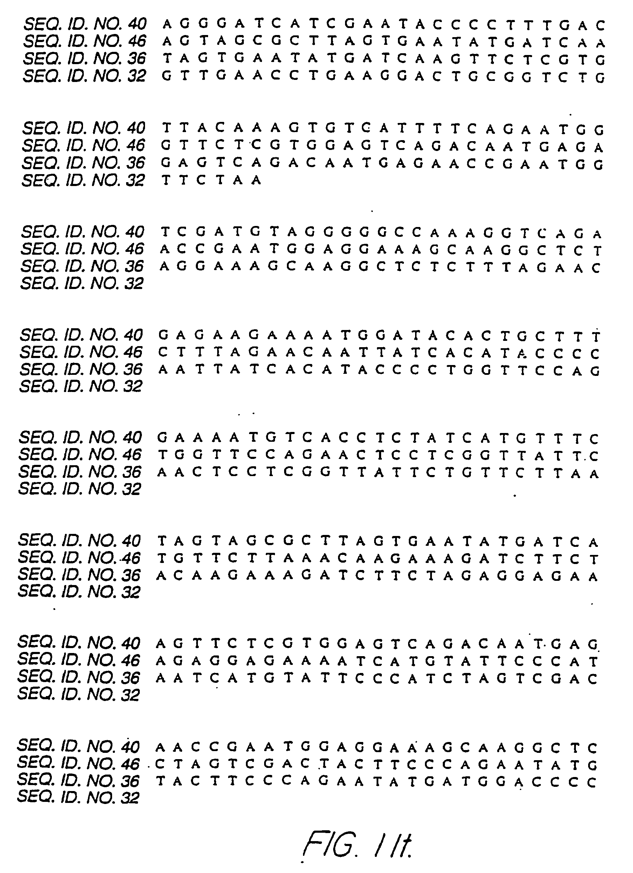

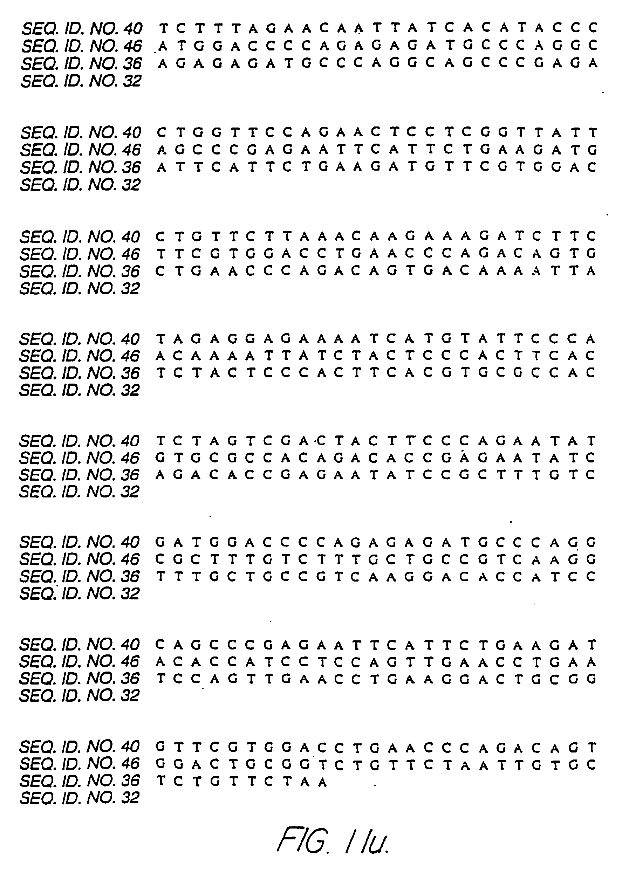

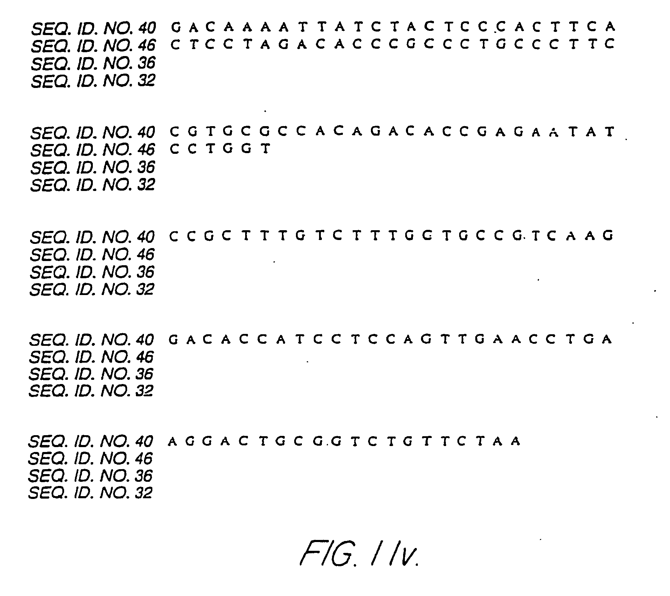

Figures 11a-11v illustrate the cDNA sequence for the phCaR/htnGlnR2*Gqi5 fusion construct (SEQ. ID. NO. 32), pmGluR2//CaR*Gα_{q}i5 fusion construct (SEQ. ID. NO. 36), pmGluR2//CaR*Gα_{q}i5+3Ala linker fusion construct (SEQ. ID. NO. 46), and the mGluR8//CaR*Gα_{q}i5 fusion construct (SEQ. ID. NO. 40).

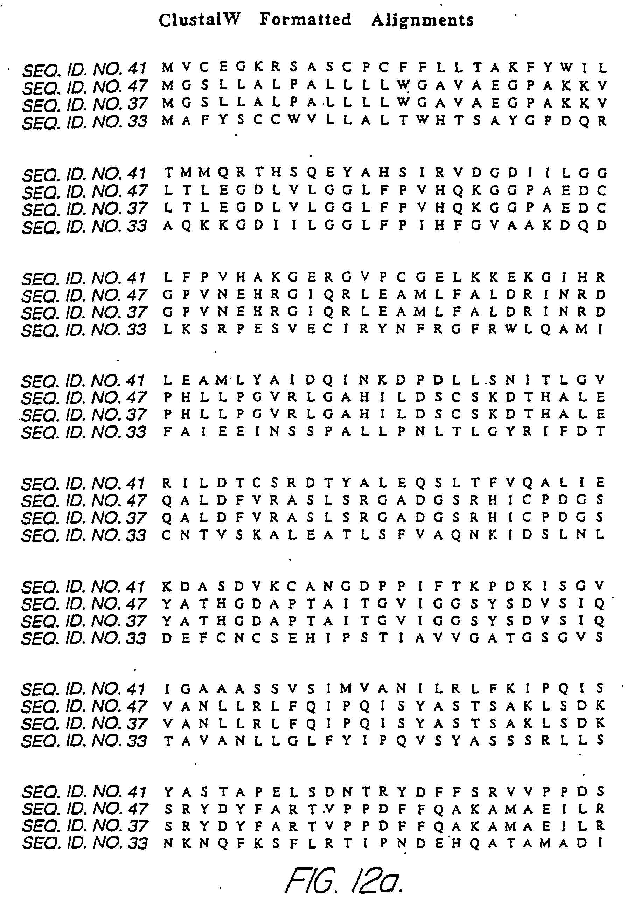

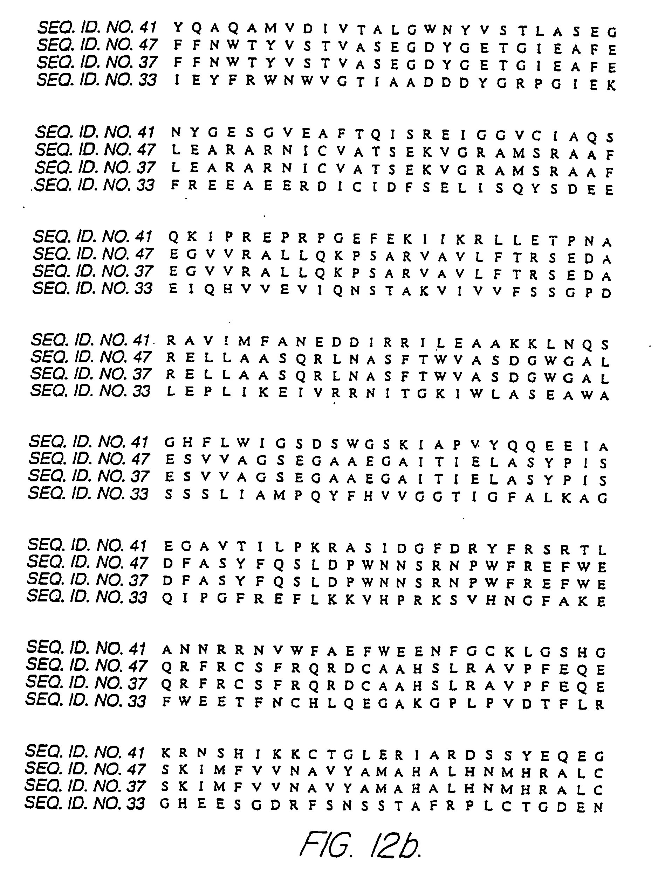

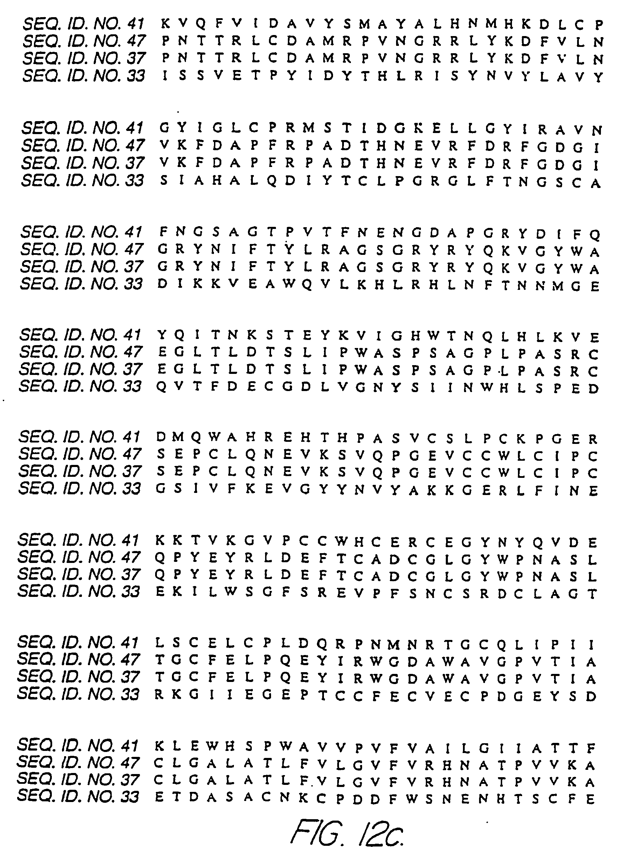

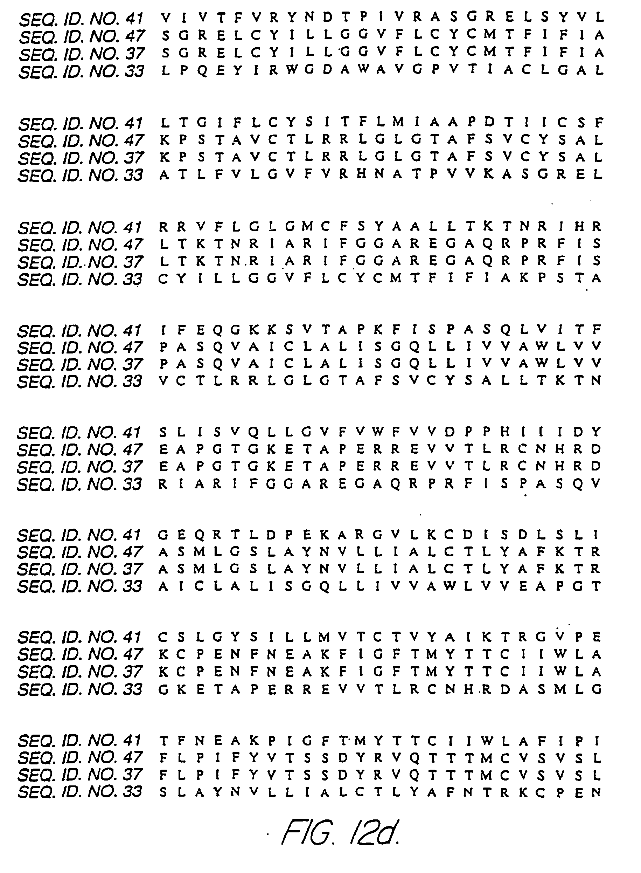

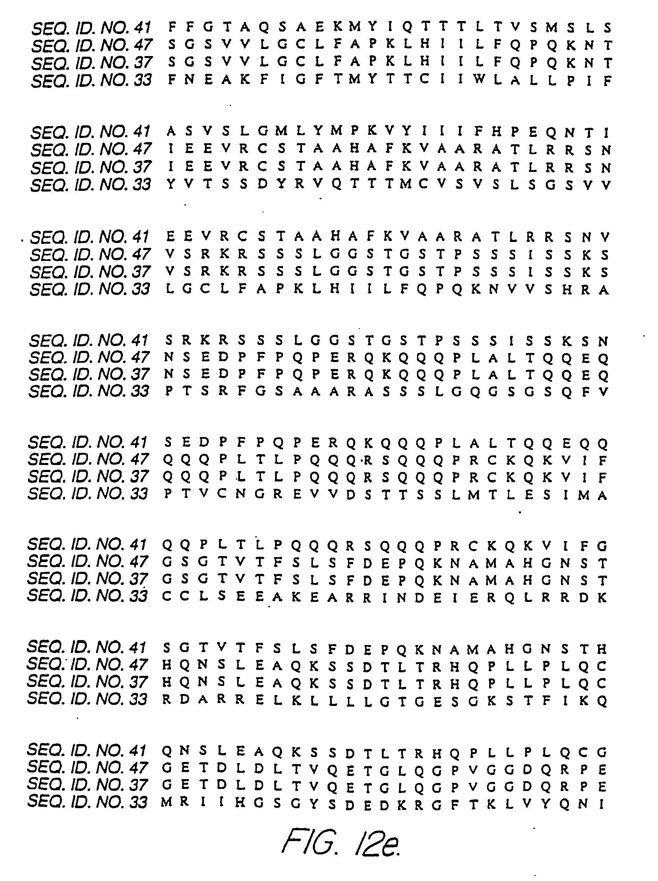

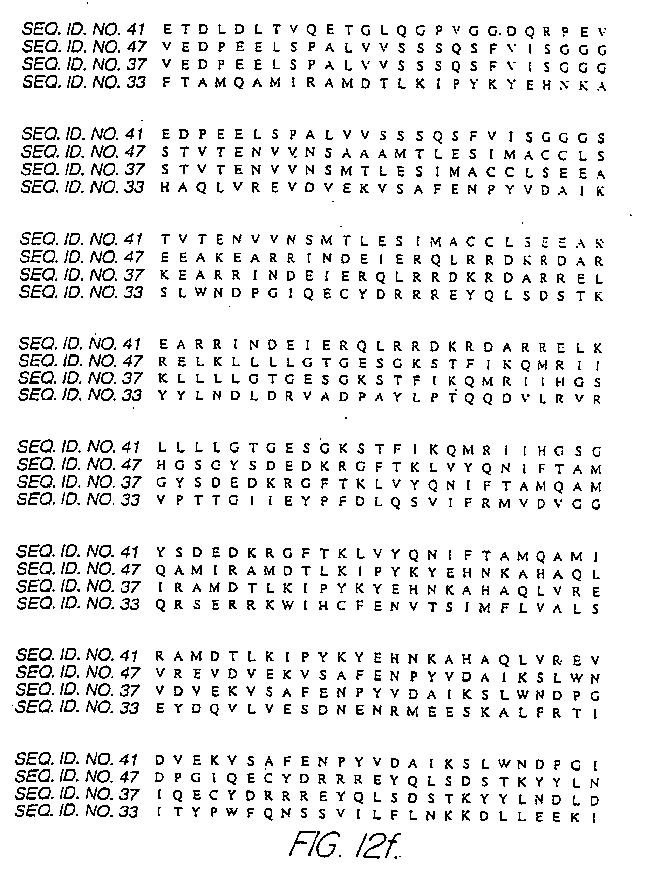

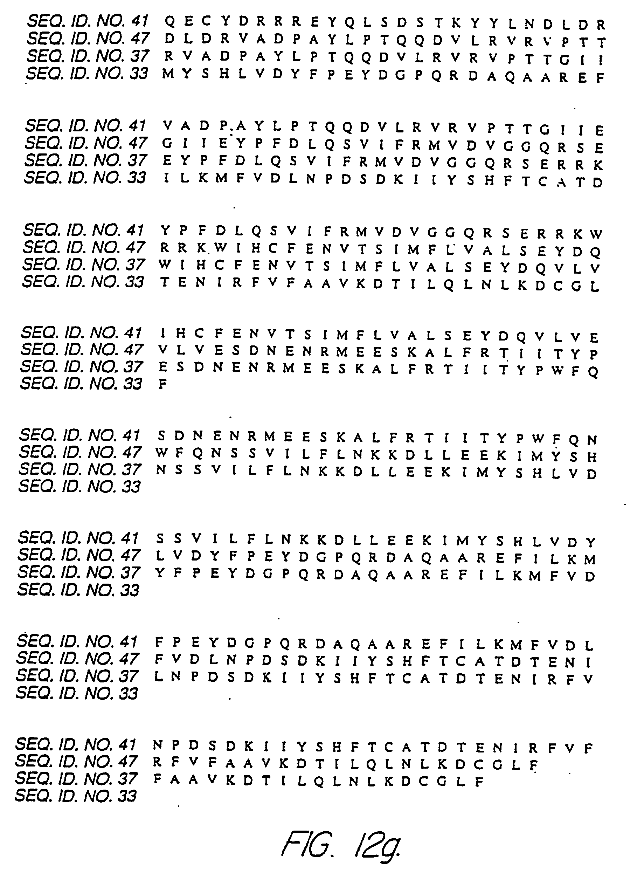

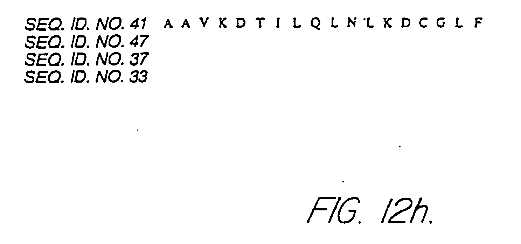

Figures 12a-12h illustrate the amino acid sequence for the phCaR/hmGluR2*Gqi5 fusion construct (SEQ. ID. NO. 33), pmGluR2//CaR*Gα_{q}i5 fusion construct (SEQ. ID. NO. 37), pmGluR2//CaR*Gα_{q}i5+3Ala linker fusion construct (SEQ. ID, NO. 47), and the mGluR8//CaR*G α_{q}i5 fusion construct (SEQ. ID. NO. 41).

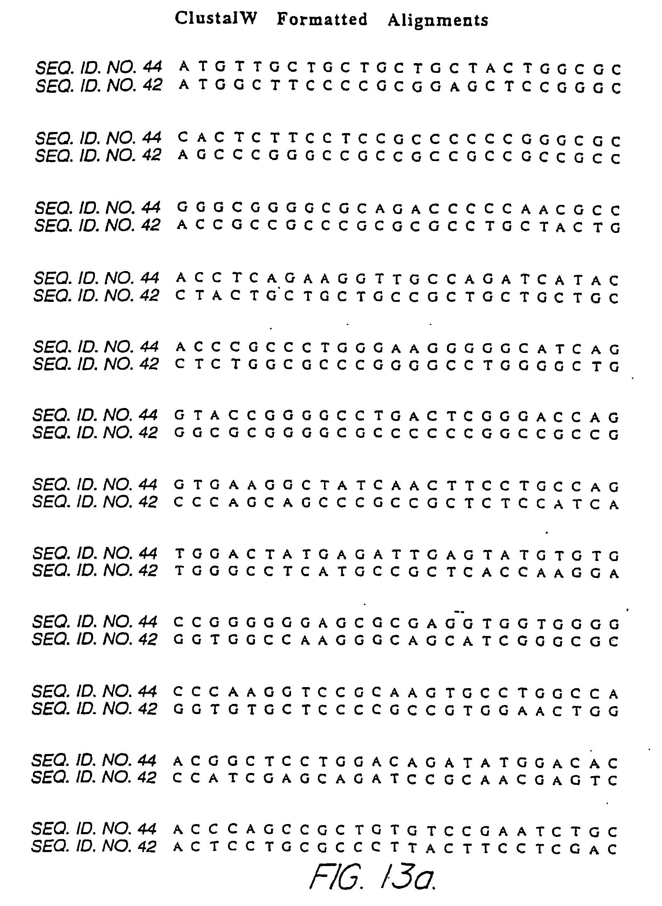

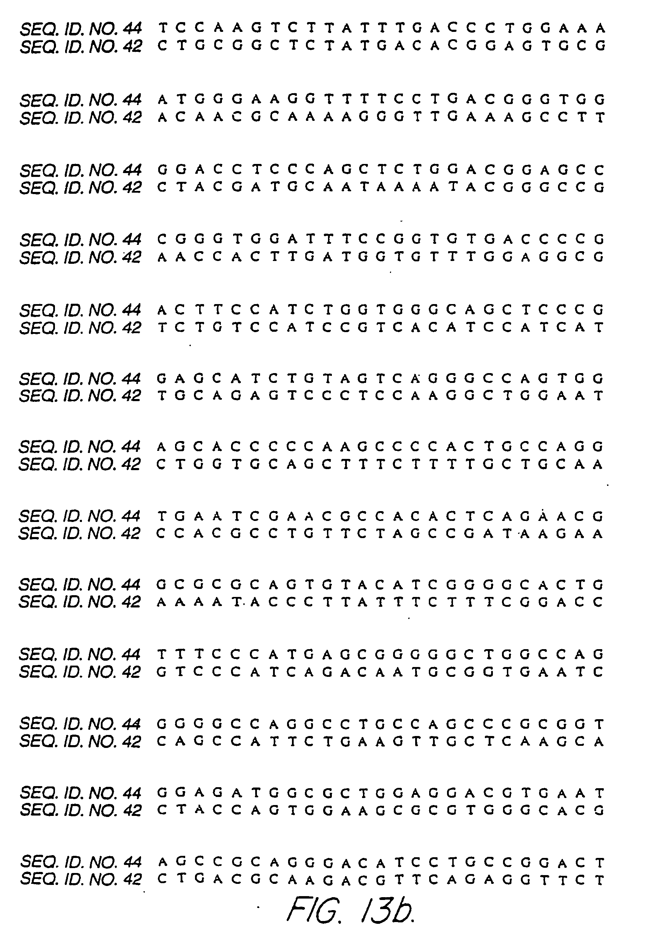

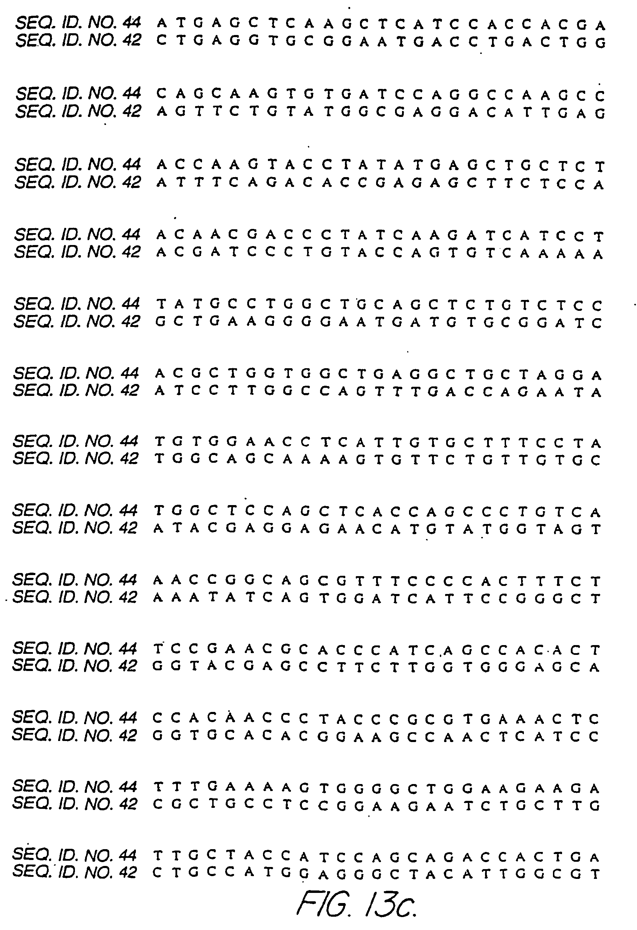

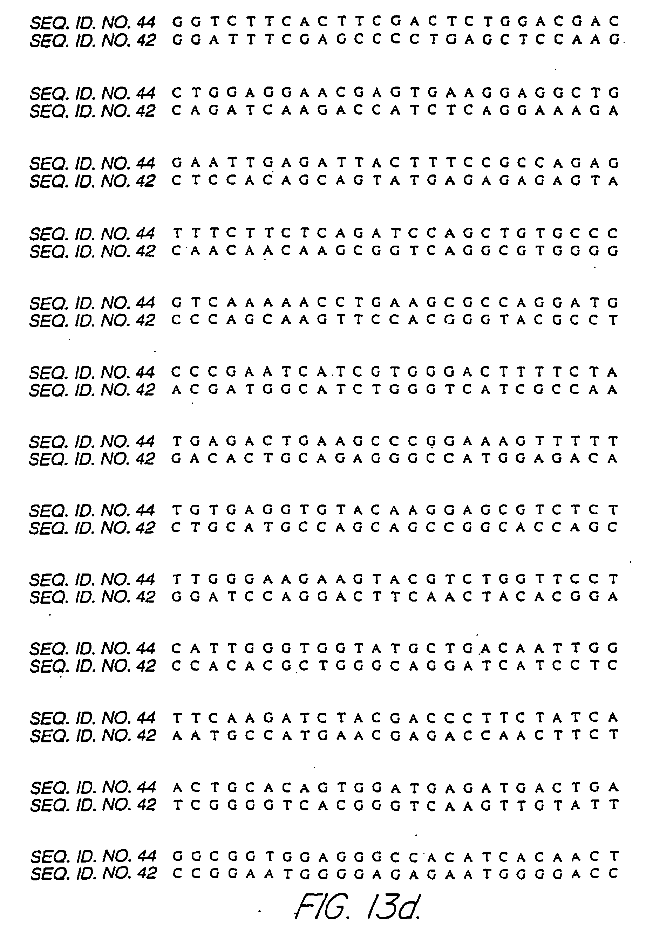

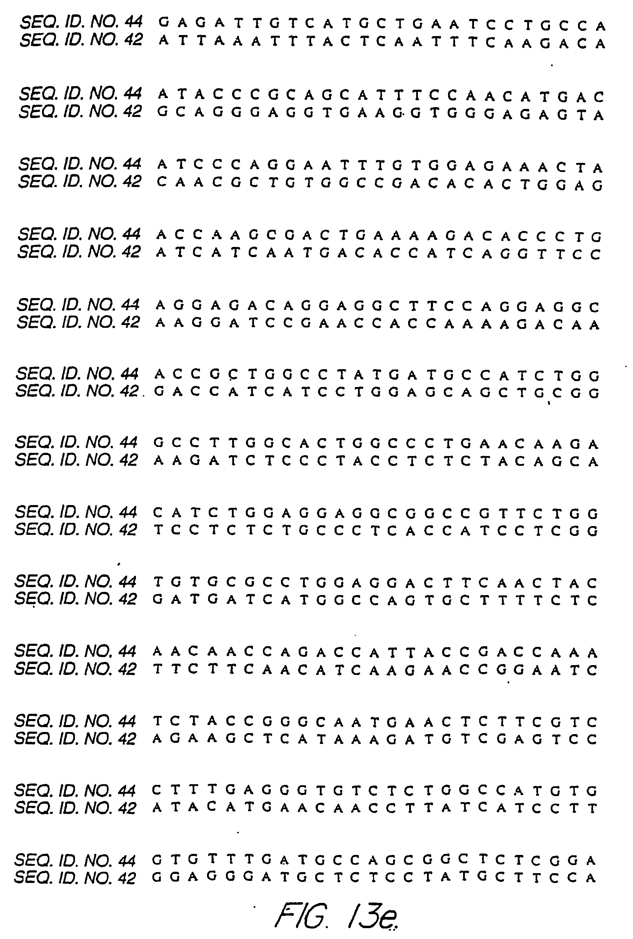

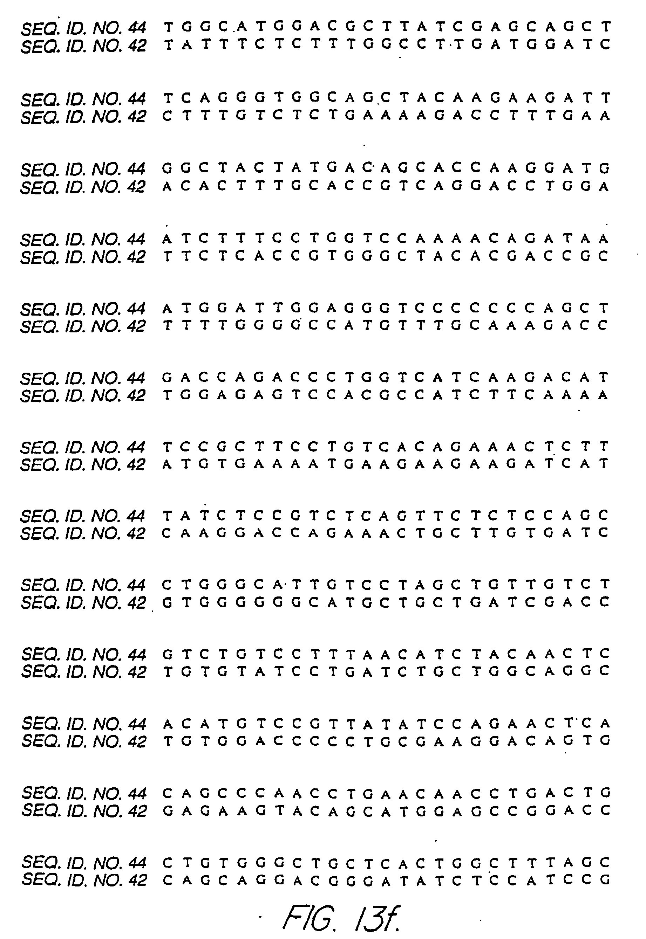

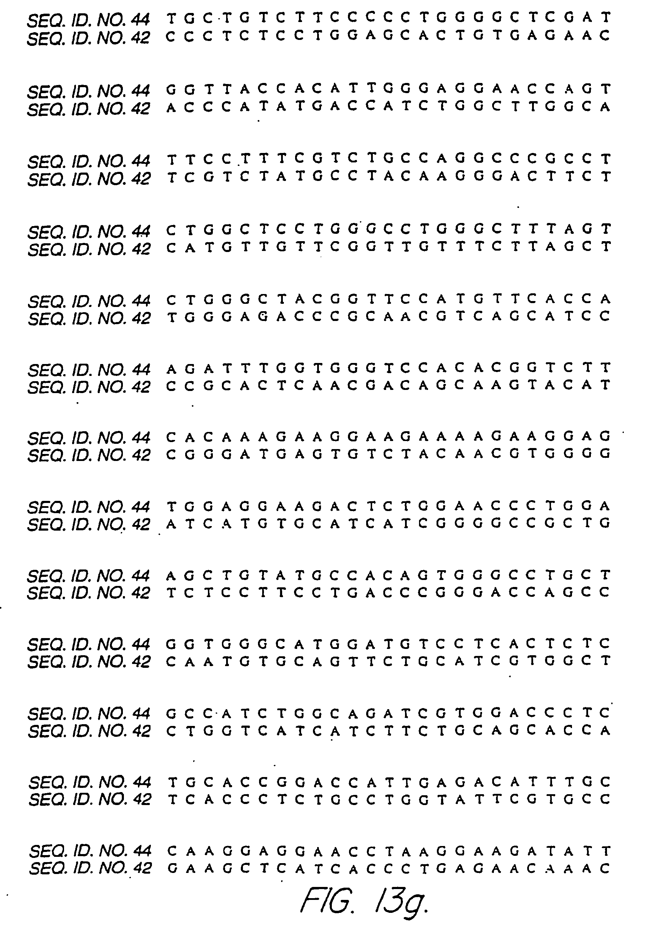

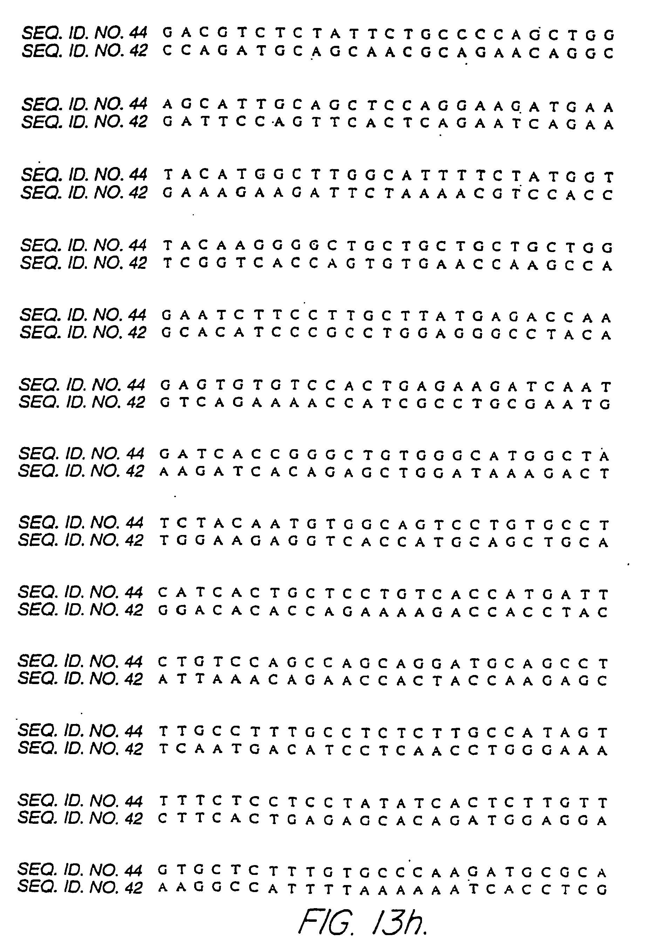

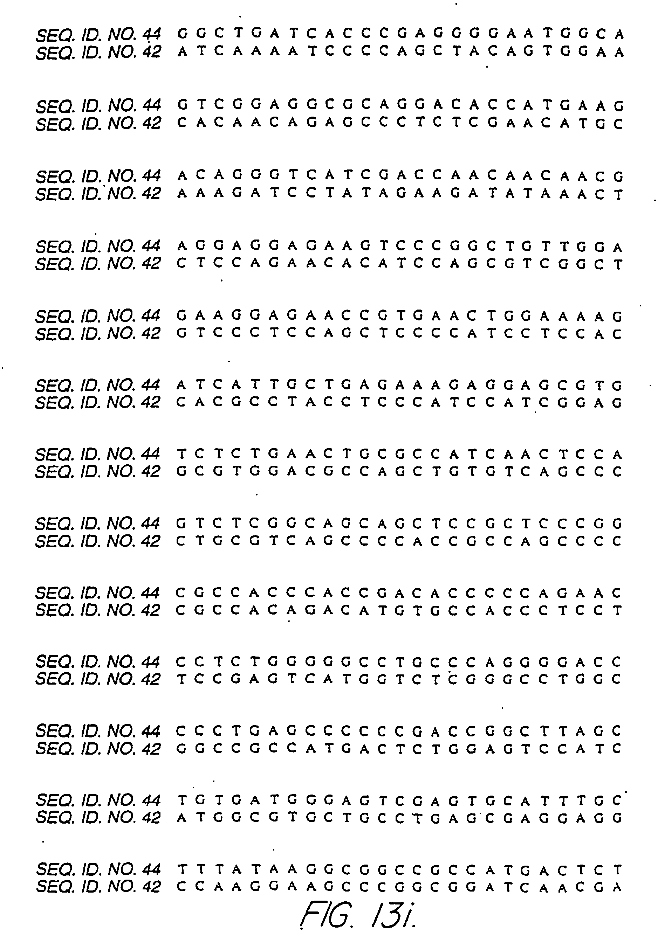

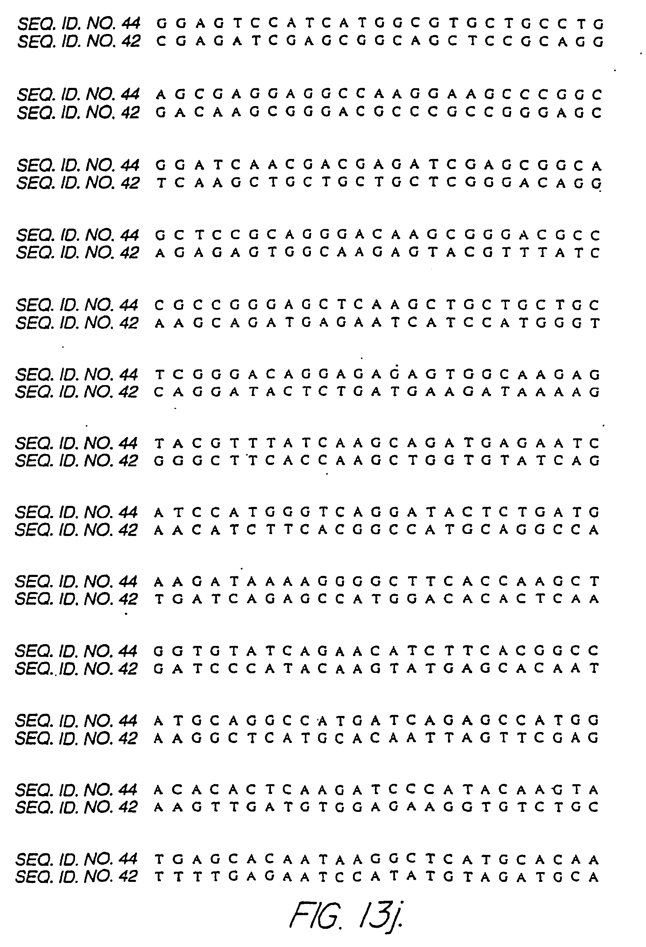

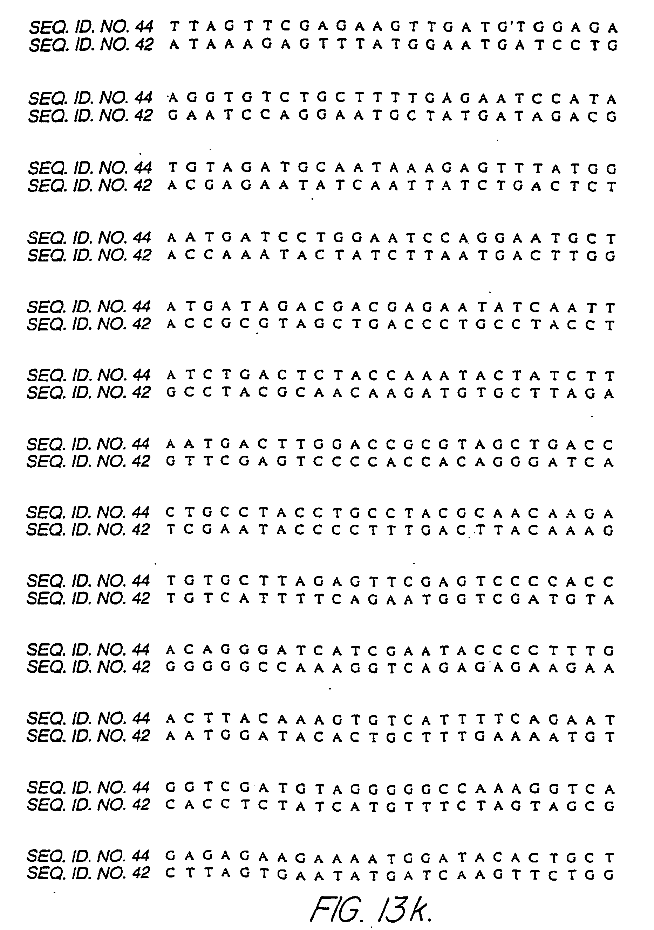

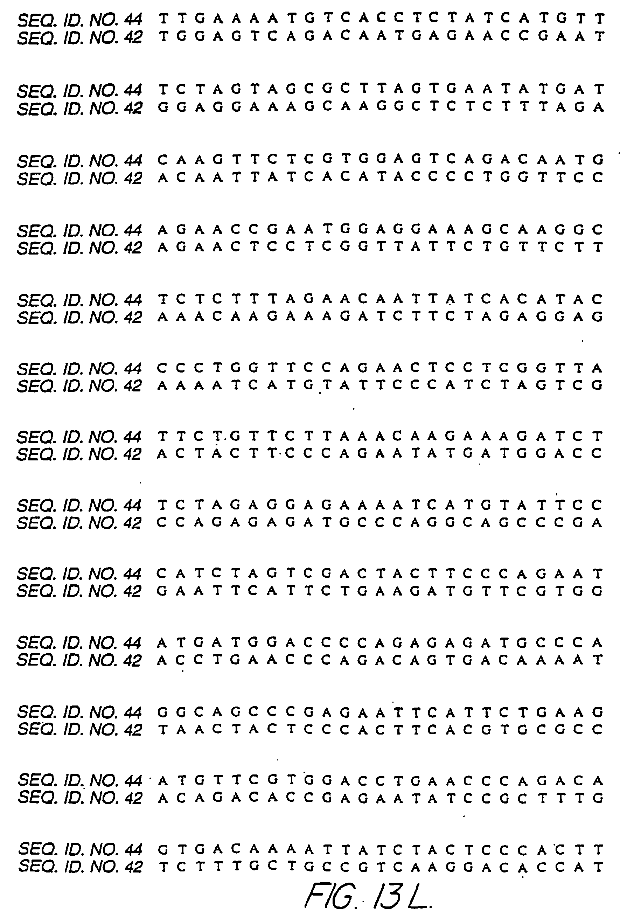

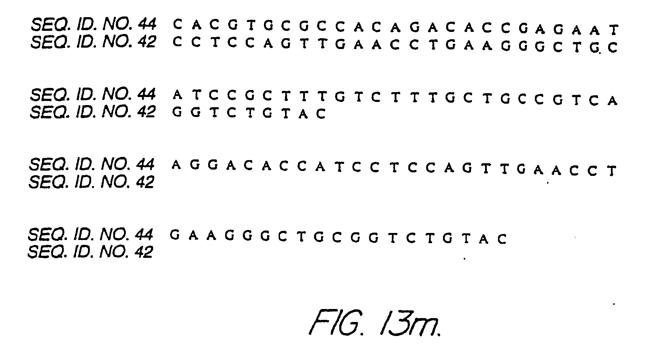

Figures 13a-13m illustrate the cDNA sequence for the GABA-R2*Gqo5 fusion construct (SEQ. ID. NO. 42) and the GABA-BR1a*Gqo5 fusion construct (SEQ. ID. NO. 44).

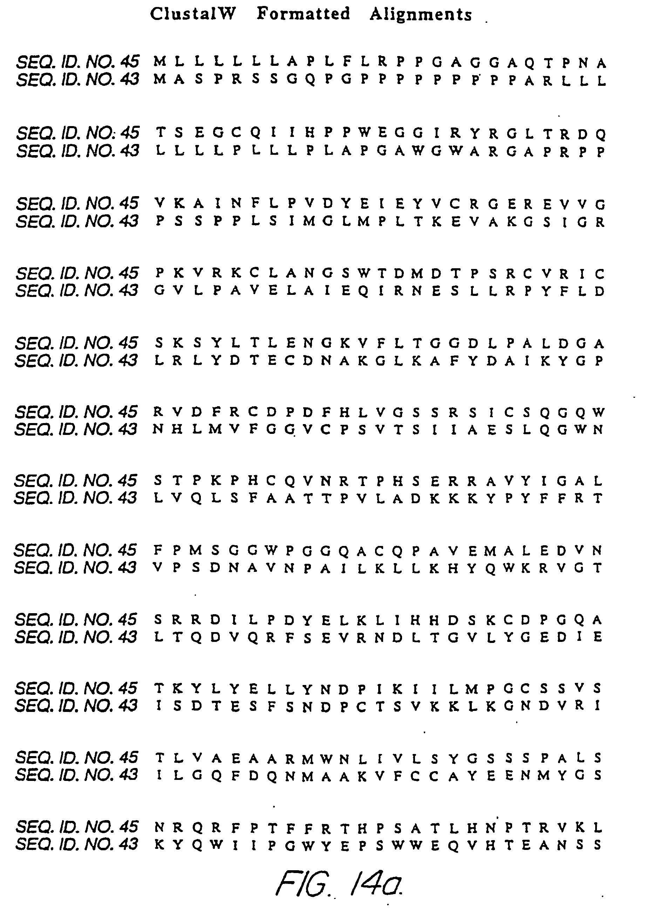

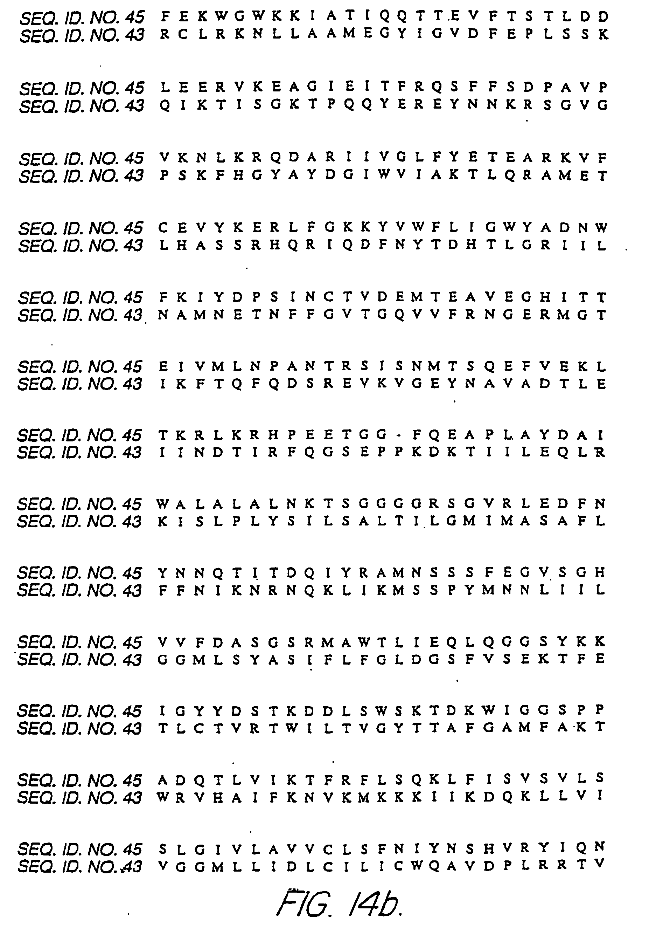

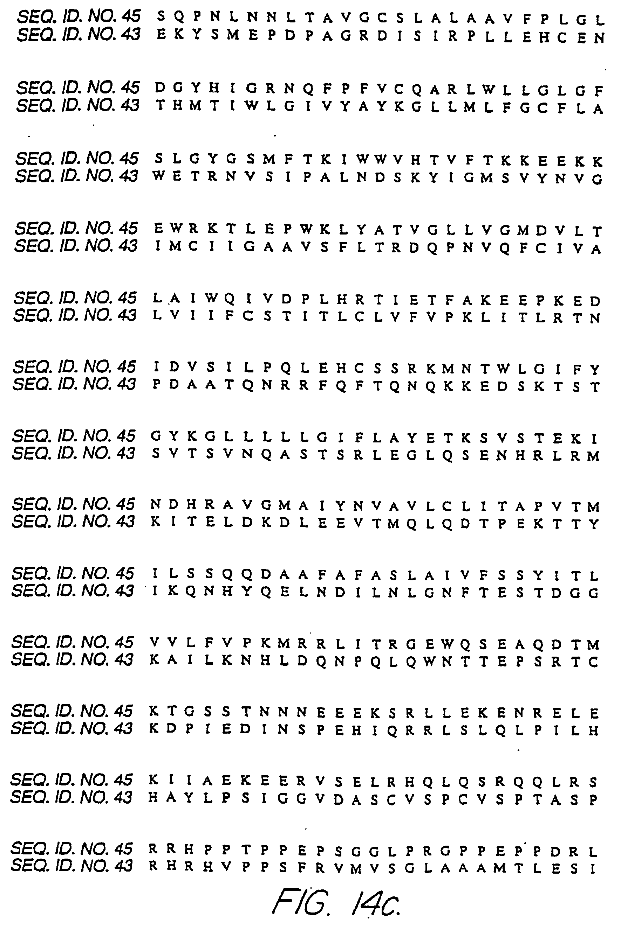

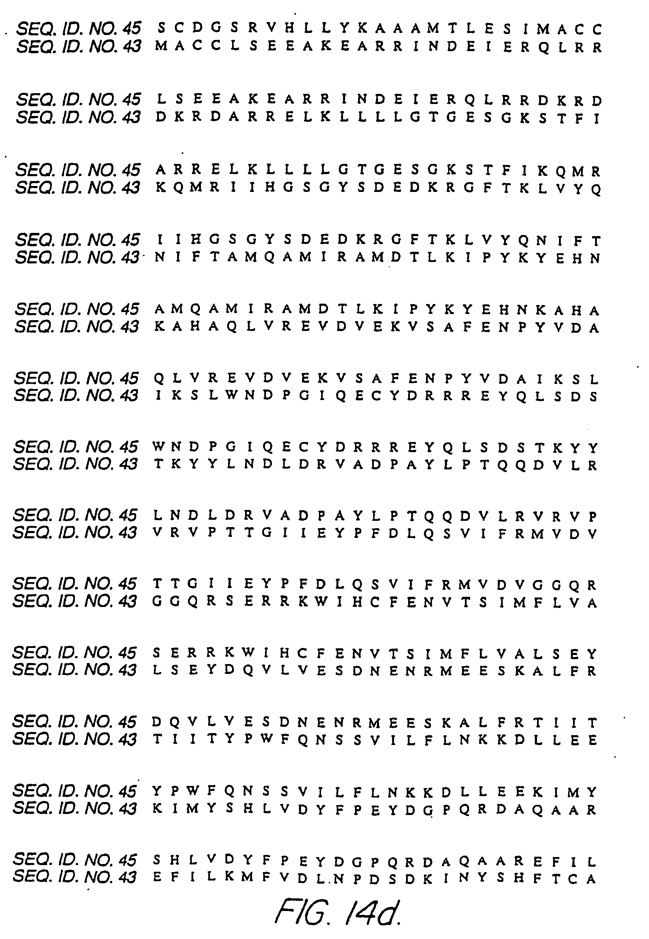

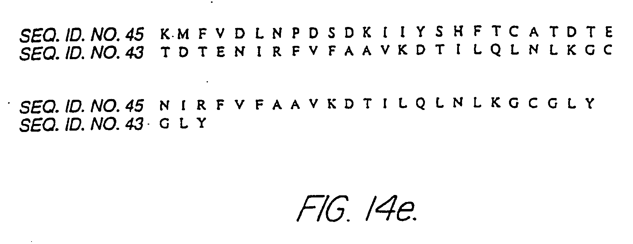

Figures 14a-14e illustrate the amino acid sequence for the GABA-BR2*Gqo5 fusion construct (SEQ. ID. NO. 43) and the GABA-BR1a*Gqo5 fusion construct (SEQ. ID. NO. 45).
Figure 15 illustrates the ability of different G-protein fusions to transduce signal resulting from ligand binding. mGluR2//CaR*Gqi5 is shown by SEQ. ID. NO. 37, CaR/mGIuR2*Gqi5 is shown by SEQ. ID. NO. 33, mGluR8//CaR*Gqi5 is shown by SEQ. ID. NO. 41.

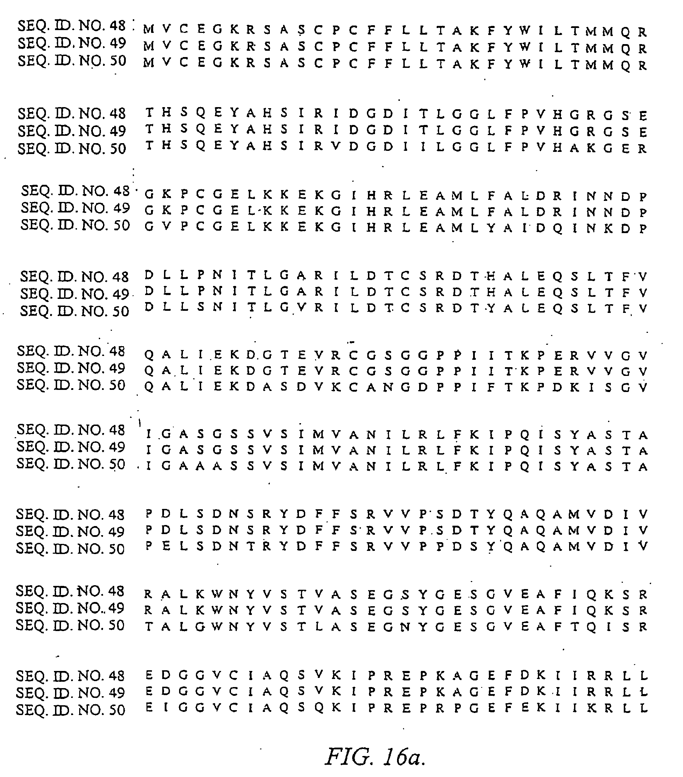

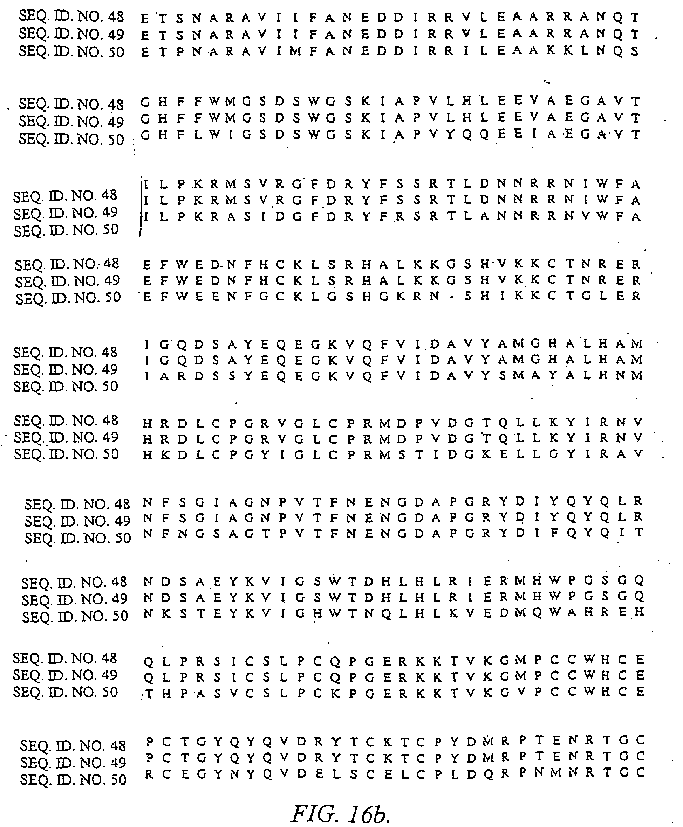

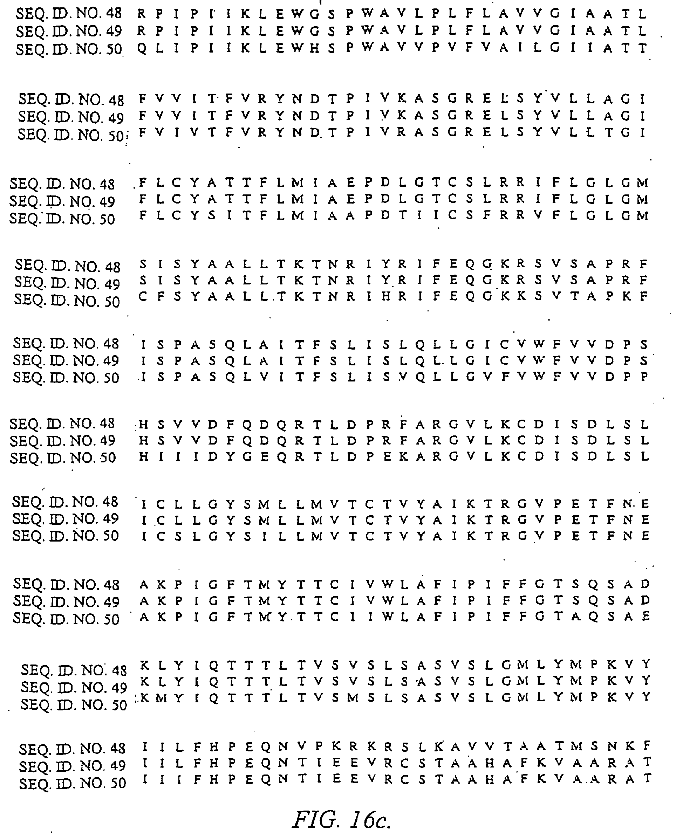

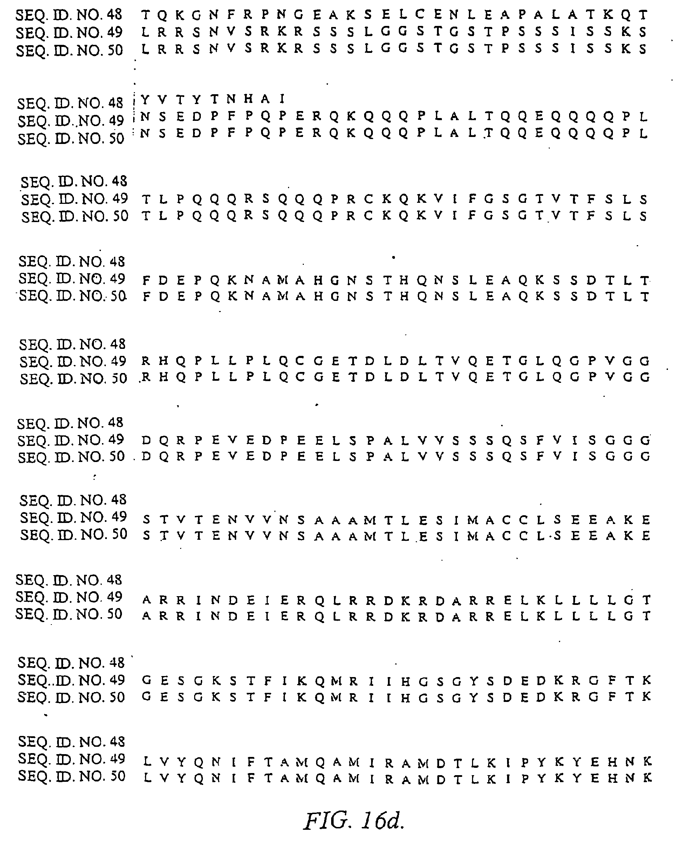

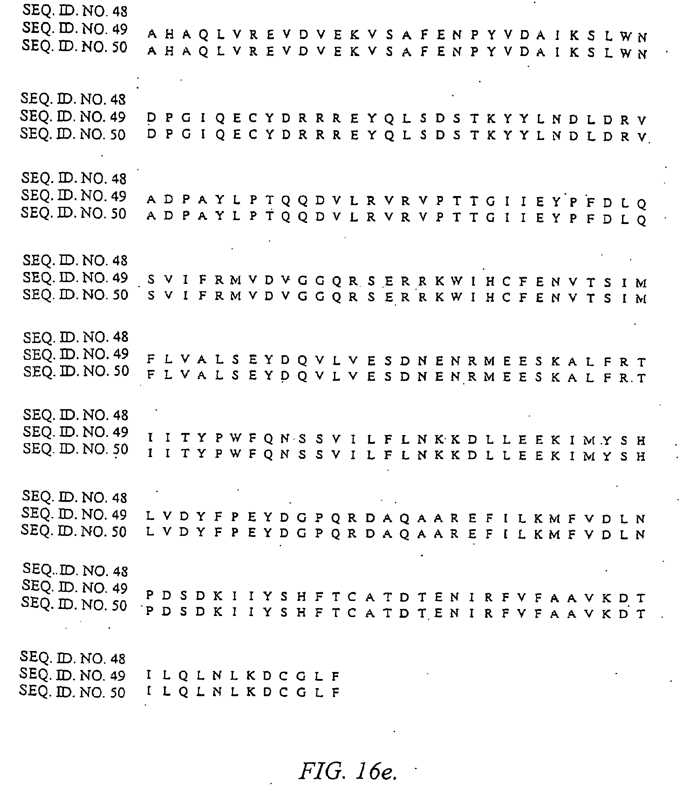

Figures 16a-16e illustrate the amino acid sequence for the ph8SPmGluR4 chimeric construct (SEQ. ID. NO.48), the amino acid sequence for the phmGluR4//CaR*AAA*Gα_{q}i5 fusion construct (SEQ. ID. NO. 49), and the phmGluR8//CaR*AAA*Gα_{q}i5 fusion construct (SEQ. ID. NO. 50).

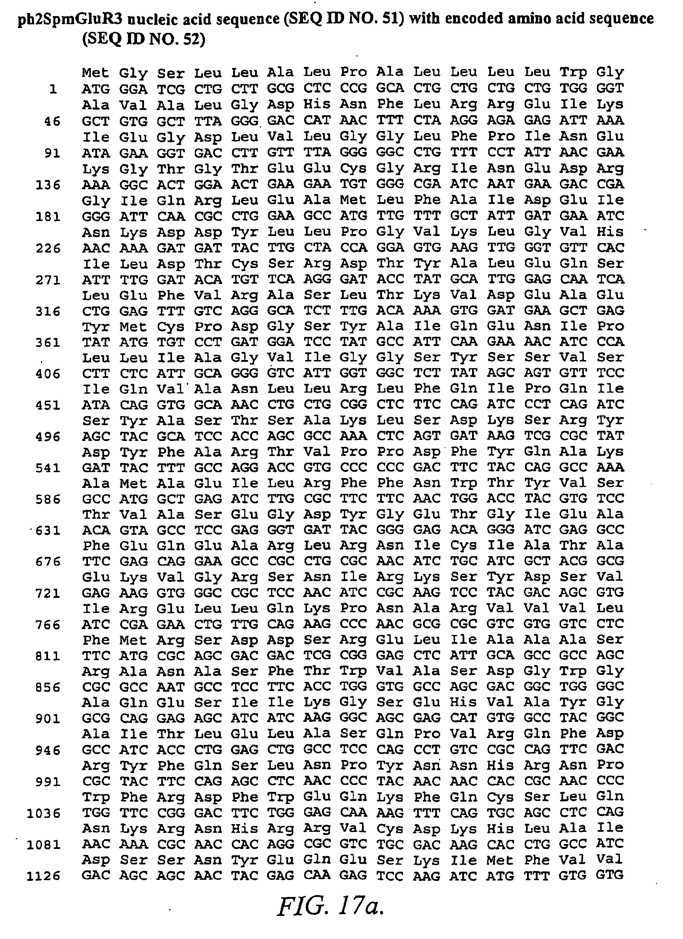

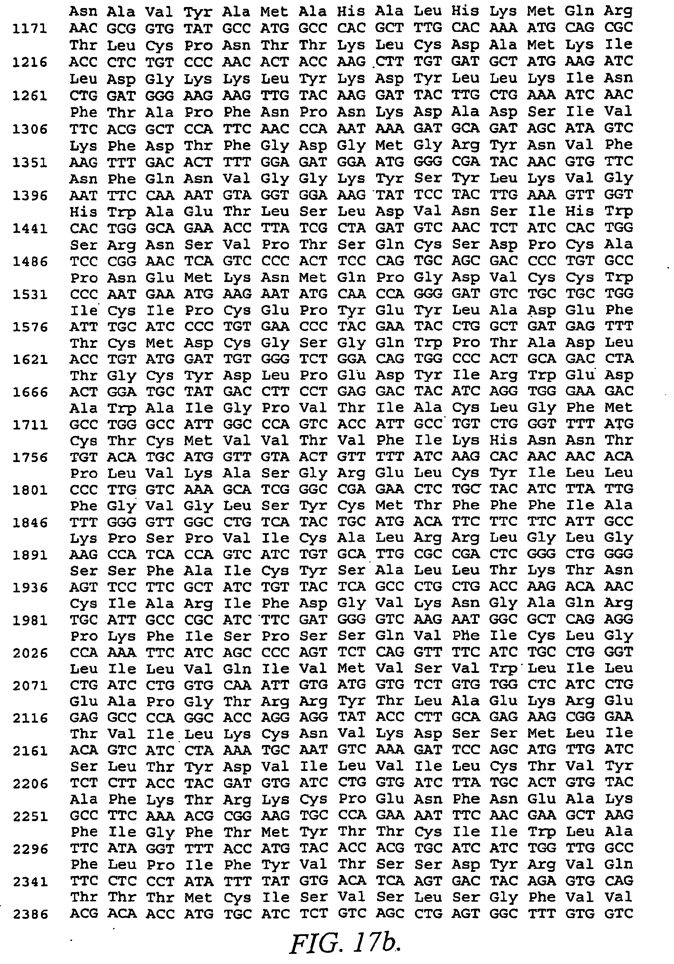

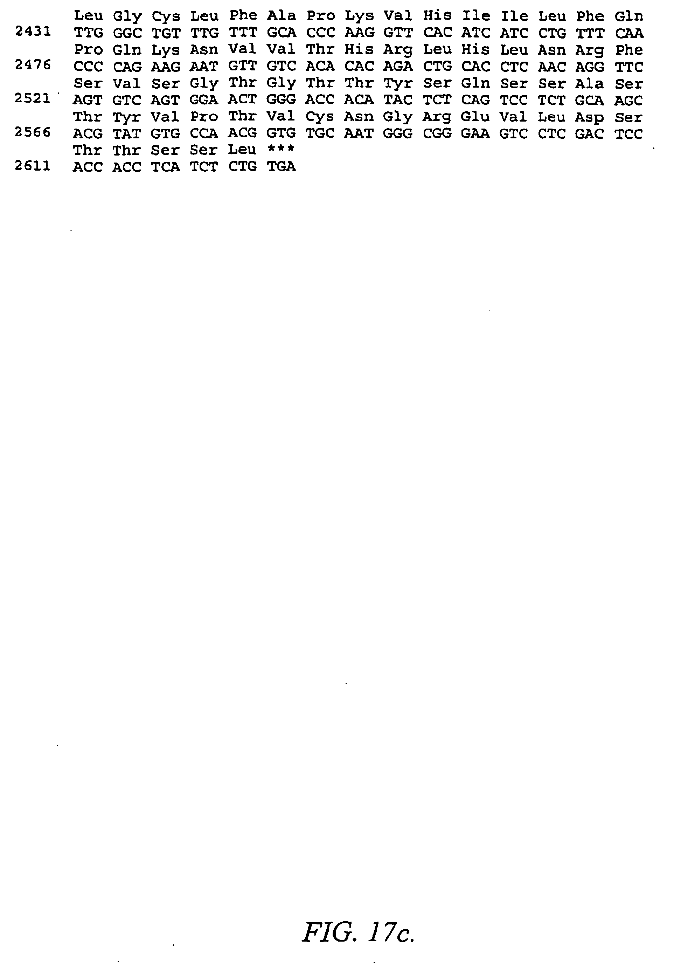

Figures 17a-17c illustrate the nucleic acid sequence (SEQ ID NO. 58) and amino acid sequence (SEQ ID NO. 59) of ph2SPmGluR3 chimeric construct.

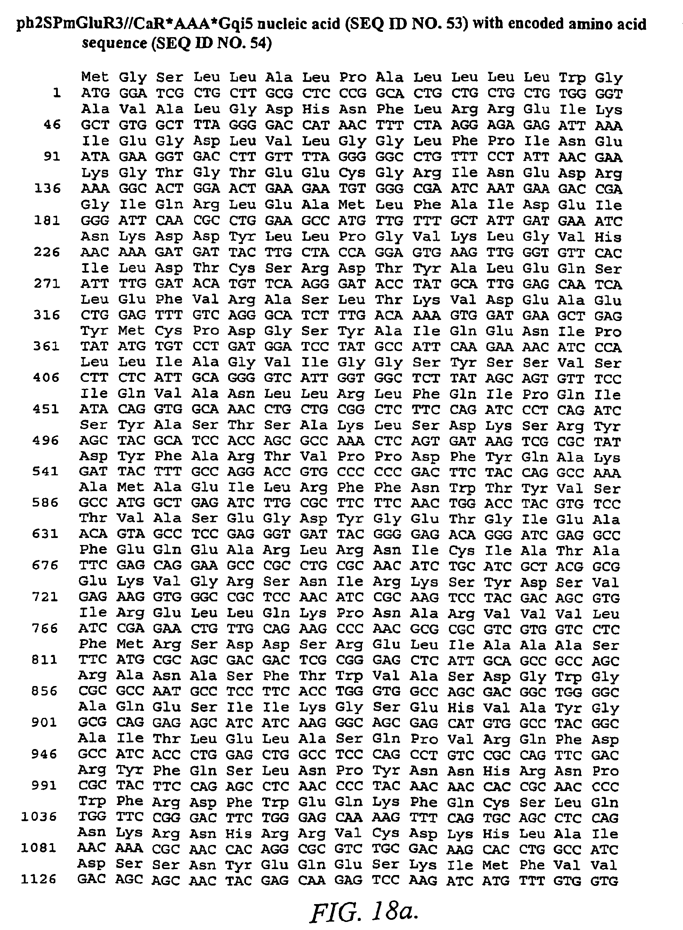

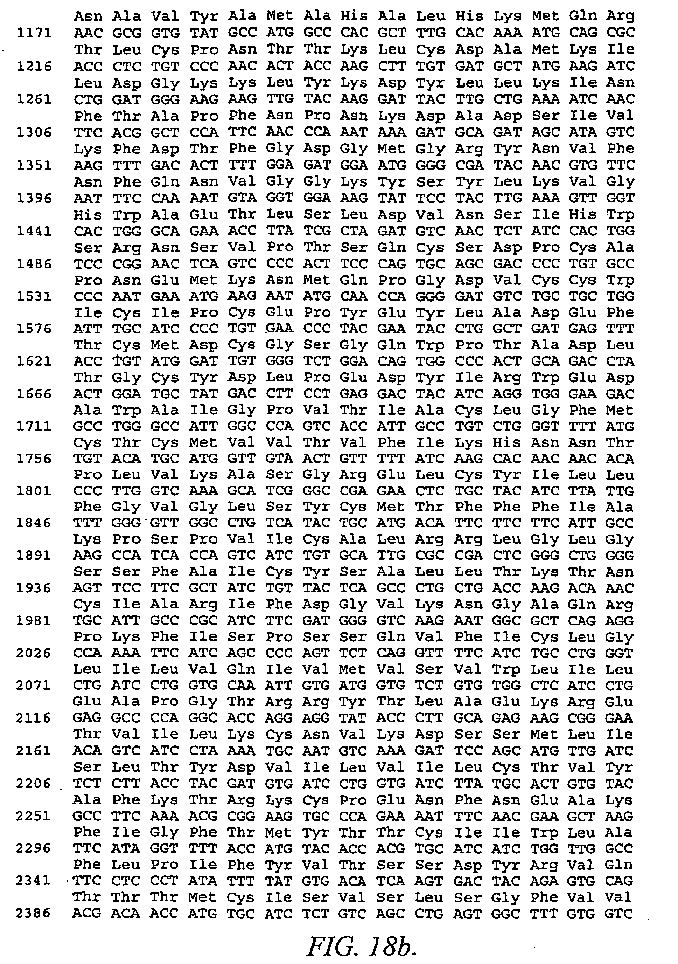

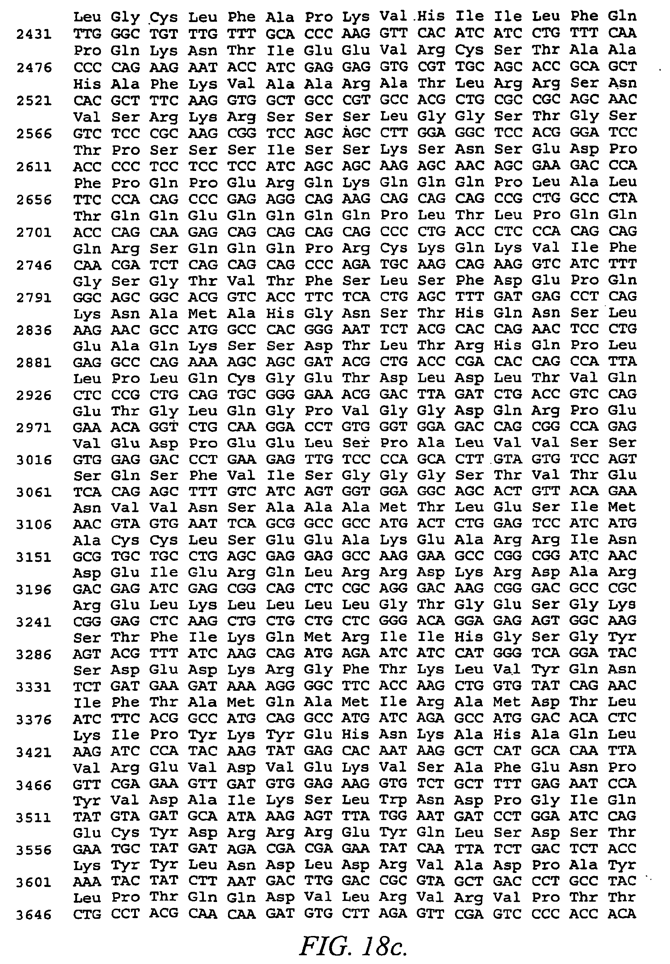

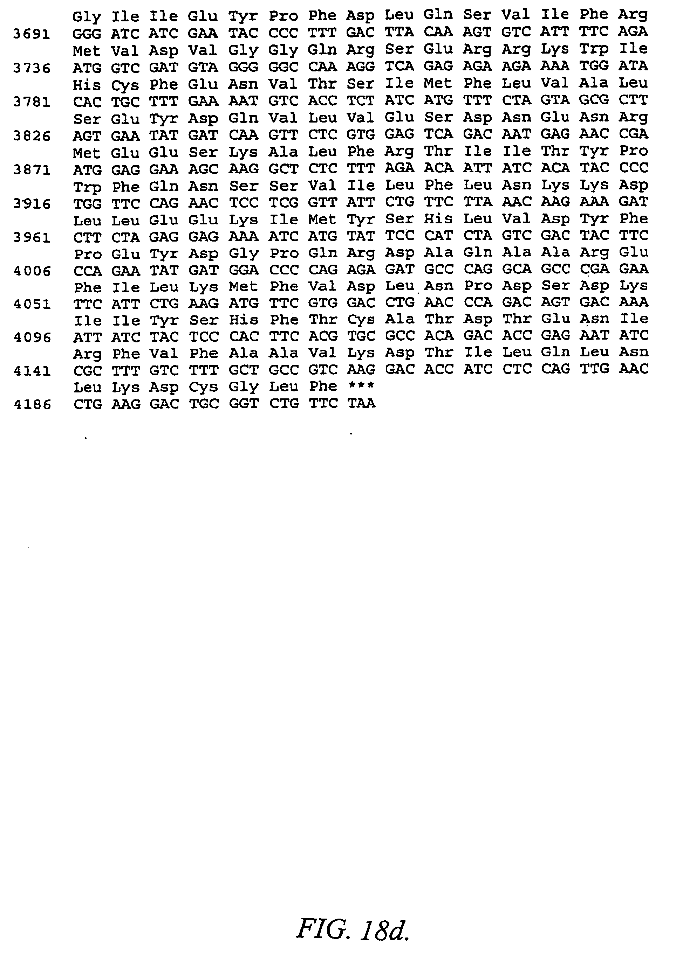

Figures 18a-18d illustrate the nucleic acid sequence (SEQ ID NO. 60) and amino acid sequence (SEQ. ID NO. 61) of ph2SPmGluR3//CaR*AAA*Gqi5 fusion construct.

### DETAILED DESCRIPTION OF THE INVENTION

The CaR, mGluR, and the GABA_{B}R are structurally similar in that they are each a single subunit membrane protein possessing an extracellular domain, a transmembrane domain comprising seven putative membrane spanning helices connected by three intracellular and three extracellular loops, and an intracellular carboxy-terminal domain. Signal transduction is activated by the extracellular binding of an agonist. The signal is transduced to the intracellular components of the receptor causing an intracellular effect.

Signal transduction from agonist binding to an extracellular region can be modulated by compounds acting at a downstream transmembrane domain or the intracellular domain. Downstream effects include antagonist actions of compounds and allosteric actions of compounds.

The transmembrane domain provides different types of target sites for compounds modulating receptor activity in different environments. As noted above, the transmembrane domain contains extracellular, transmembrane, and intracellular components.

Compounds modulating GABA_{B}R, CaR, or mGluR activity can be obtained, for example, by screening a group or library of compounds to identify those compounds having the desired activity and then synthesizing such compound.

### Metabotropic Glutamate Receptors (mGluRs)

mGluRs are G protein-coupled receptors capable of activating a variety of intracellular secondary messenger systems following the binding of glutamate (Schoepp et al., Trends Pharmacol. Sci. 11:508, 1990; Schoepp and Conn, Trends Pharmacol. Sci. 14:13, 1993, hereby incorporated by reference herein).

Activation of different mGluR subtypes *in situ* elicits one or more of the following responses: activation of phospholipase C, increases in phosphoinositide (PI) hydrolysis, intracellular calcium release, activation of phospholipase D, activation or inhibition of adenylyl cyclase, increases and decreases in the formation of cyclic adenosine monophosphate (cAMP), activation of guanylyl cyclase, increases in the formation of cyclic guanosine monophosphate (cGMP), activation of phospholipase A₂, increases in arachidonic acid release, and increases or decreases in the activity of voltage- and ligand-gated ion channels (Schoepp and Conn, Trends Pharmacol. Sci. 14:13, 1993; Schoepp, Neurochem. Int. 24:439, 1994; Pin and Duvoisin, Neuropharmacology 34:1,1995, hereby incorporated by reference herein).

Eight distinct mGluR subtypes have been isolated. (Nakanishi, Neuron 13:1031,1994; Pin and Duvoisin, Neuropharmacology 34:1, 1995; Knopfel et al., J. Med. Chem. 38:1417; Eur. J. Neuroscience 7:622-629, 1995, each of these references is hereby incorporated by reference herein.) The different mGluRs possess a large amino-terminal extracellular domain (BCD) followed by a seven putative transmembrane domain (7TMD) comprising seven putative membrane spanning helices connected by three intracellular and three extracellular loops, and an intracellular carboxy-terminal domain of variable length (cytoplasmic tail).

Human mGluR8 is described by Stormann *et al.,* U.S. Patent Nos. 6,051,688, 6,077,675, and 6,084,084, and mouse mGluR8 is described by Duvoisin et al., J. Neurosci. 15:3075-3083,1995, (both of these references are hereby incorporated by reference herein). mGluR8 couples to Gᵢ. Agonists of mGluR8 include L-glutamate and L-2-ammo-4-phosphonobutyrate.

mGluR8 activity can be measured using standard techniques. For example, Gᵢ negatively couples to adenylate cyclase to inhibit intracellular cAMP accumulation in a pertussis toxin-sensitive fashion. Thus, mGluR8 activity can be measured, for example, by measuring inhibition of forskolin-stimulated cAMP production as described by Duvoisin et al., J. Neurosci. 15:3075-3083, 1995.

mGluRs have been implicated in a variety of neurological pathologies. Examples of such pathologies include stroke, head trauma, spinal cord injury, epilepsy, ischemia, hypoglycemia, anoxia, and neurodegenerative diseases such as Alzheimer's disease (Schoepp and Conn, Trends Pharmacol. Sci. 14:13, 1993; Cunningham et al., Life Sci. 54: 135, 1994; Pin et al., Neuropharmacology 34:1, 1995; Knopfel et al., J. Med. Chem. 38:1417, 1995, each of which is hereby incorporated by reference herein).

### Calcium Receptor

The CaR responds to changes of extracellular calcium concentration and also responds to other divalent and trivalent cations. The CaR is a G-protein coupled receptor containing an extracellular Ca²⁺ binding domain. Activation of the CaR, descriptions of CaRs isolated from different sources, and examples of CaR active compound are provided in Nemeth NIPS 10:1-5,1995, Brown et al. U.S, Patent No. 5,688,938, Van Wagenen et al, International Application Number PCT/US97/05558 International Publication Number WO 97/37967, Brown B.M. et al., Nature 366:575,1993, Riccardi D., et al., Proc. Nat'l. Acad Sci. USA 92:131-135,1995, and Garrett J.B., et al., J. Biol. Chem. 31:12919-12925,1995. (Each of these references are hereby incorporated by reference herein.) Brown et al. U.S. Patent No. 5,688,938 and Van Wagenen et al., International Application Number PCT/US97/05558 International Publication Number WO 97/37967, describe different types of compounds active at the CaR including compounds which appear to be allosteric modulators and CaR antagonists.

The CaR can be targeted to achieve therapeutic effects. Examples of target diseases are provided in Brown et al. U.S. Patent No. 5,688,938, and Van Wagenen et al., International Application Number PCT/US97/05558 International Publication Number WO 97/37967, and include hyperparathyroidism and osteoporosis.

### γ-Aminobutyric acid Receptors (GABA_{B}Rs)

GABA_{B}Rs are G-protein coupled metabotropic receptors. GABA_{B}Rs modulate synaptic transmission by inhibiting presynaptic transmitter release and by increasing K⁺ conductance responsible for long-lasting inhibitory postsynaptic potentials. (*See,* Kaupmann et al, Nature 386:239-246,1997, hereby incorporated by reference herein.) GABA_{B}Rs are found in the mammalian brain, in locations outside of the brain, and in lower species. Outside of the brain, GABA_{B}Rs have been identified on axon terminals and ganglion cell bodies of the autonomic nervous system, on fallopian tube and uterine intestinal smooth muscle cells, in the kidney cortex, urinary bladder muscle and on testicular interstitial cells. (*See*, Bowery, Annu Rev. Pharmacol Toxicol. 33:109-147,1993, hereby incorporated by reference herein,)

Different GABA_{B}Rs subtypes exist Kaupmann et al., Nature 386:239-246,1997, indicate that they cloned GABA_{B}Rs. Nucleic acid encoding two GABA_{B}R proteins were indicated to be cloned from rat brain: GABAaRIa and GABA_{B}R1b. GABA_{B}R1 a differs from GABA_{B}R1b in that the N-terminal 147 residues are replaced by 18 amino acids. GABA_{B}R1a and GABA_{B}R1b appear to be splice variants. The cloned GAHA_{B}Rs were indicated to negatively couple adenylyl cyclases and show sequence similarity to the metabotropic receptors for L-glutamate (mGluR). Northern blot analysis indicated that GABA_{B}R1a and GABA_{B}R1b is present in brain and testis, but not in kidney, skeletal muscle, liver, lung, spleen, or heart.

Kaupmann et al., International Application Number PCT/EP97/01370, International Publication Number WO 97/46675, indicate that they have obtained rat GABA_{B}R clones, GABA_{B}R1a and GABA_{B}R1b; and humans GABA_{B}R clones, GABA_{B}R1a/b (representing a partial receptor clone) and GABA_{B}R1b (representing a full-length receptor clone). Amino acid sequence information, and encoding cDNA sequence information, is provided for the different GABA_{B}R clones.

Another GABA_{B}R subtype is GABA_{B}R2. Northern blot analysis reveals than an approximately 6.3 Kb human GABA_{B}R2 transcript is abundantly expressed in the human brain. Expression is not detected in the heart, placenta, lung, liver, skeletal muscle, kidney and pancreas under conditions where GABA_{B}R2 transcript was identified in the human brain. Within the human brain GABA_{B}R2 is broadly expressed at variable levels.

GABA_{B}R functions as a heterodimer of the subunits GARA_{B}R1 or GABA_{B}R2. (Jones et al. Nature 396:674-679,1998, hereby incorporated by reference herein.)

GABA_{B}Rs have been targeted to achieve therapeutic effects. Kerr and Ong, DDT 1:371-380, 1996, describe different compounds indicated to be GABA_{B}R agonists and GABA_{B}R antagonists. Kerr and Ong also review therapeutic implications of affecting GABA_{B}R activity including, spasticity and motor control, analgesia, epilepsy, cognitive effects, psychiatric disorders, alcohol dependence and withdrawal, feeding behavior, cardiovascular and respiratory functions, and peripheral functions.

Bittiger et al., Tips 4:391-394,1993. review therapeutic applications of GARA_{B}R antagonists. Potential therapeutic applications noted by Bittiger *et al.* include cognitive processes, epilepsy, and depression.

### G-Protein Fusion Receptors

Examples of some different types of G-protein fusion receptors, and advantages of some receptors, are provided below. Using the present application as guide additional G-protein receptors fusion can be constructed.

G-protein fusion receptors contain an intracellular domain of a receptor fused to a G-protein α subunit (G_{α}). G_{α} fusions to adrenoreceptors have been reported by Bertin et al., Receptors and Channels 5:41-51,1997; Wise and Milligan, Journal of Biological Chemistry 39:24673-24678,1997; and Bertin et al., Proc, Natl. Acad. Sci. USA 91:8827-8831,1994 (each of which are hereby incorporated by reference herein), These studies were indicated to produce a functional chimeric by fusing the α_{2A}-adrenoreceptor to the G_{i1α}, or the β₂-adrenoreceptor to the G_{α},

The G-protem fusion receptors described by the present invention include a G-protein fused to an intracellular domain, where the intracellular domain when present in a wild type receptor does not interact with that type of G-protein. Thus, the present invention also describes swapping of signals by fusing an intracellular domain to a G_{α} normally not coupled to that intracellular domain. The use of such fusion proteins, while applicable to chimeric GABA_{B}Rs, is not limited to chimeric GABA_{B}Rs. Indeed, such technology can be applied to receptors containing an extracellular domain, transmembrane domain and intracellular domain of a wild type receptor.

The G-proteins fusion receptors contain an intracellular domain fused to a promiscuous G_{α} that couples to phospholipase C resulting in the mobilization of intracellular calcium. Increases in intracellular calcium can be conveniently measured through the use of dyes. Such techniques are well known in the art and are described, for example by Brown et al. U.S. Patent No. 5,688,938.

G-proteins fusions can also be used to decrease receptor desensitization.

Examples of promiscuous G_{α}'s coupling to phospholipase C include naturally occurring G-protsms such as G_{α15} and G_{α16}, and chimeric G-protein such as Gqo5 and Gqi5. Gqo5 and Cqi5 are made of a Gq portion where the five amino acids at the C-terminal are from either Gₒ or Gᵢ, respectively (Conklin et al. Nature 363;274-277,1993, hereby incorporated by reference herein). The Gq portion of such chimeric receptors provides for phospholipase C coupling while the terminal Gₒ or Gᵢ portion allows the chimeric G-protein to couple to different receptor proteins that are normally involved in inhibitor effects on adenylate cyclase.

The receptor is fused indirectly to a G-protein consisting of the amino acid sequence of Gqi5.

The intracellular domain is linked to a G-protein indirectly linked through a linker. The linker is 3 amino acids in lenght and is made up of alanine.

### Chimeric Receptors

Examples of some different types of chimeric receptors, and advantages of some receptors, are provided below. Using the present application as guide additional chimeric receptors can be constructed.

An intracellular CaR domain can be used to couple with G-proteins which activate phospholipase C and mobilize intracellular calcium. Mobilization of intracellular calcium is readily detected, for example, by fluorescent indicators of intracellular Ca²⁺.

An additional advantage of using the intracellular CaR domain is that CaR G-protein activation is not rapidly desensitized. Thus, the intracellular CaR domain can be used to produce a stronger intracellular signal than a signal produced from a receptor which is desensitized rapidity.

### Receptor Domains

Domains of a G-protein fusion receptor, a chimeric receptor, and G_{α}, substantially similar to a particular sequence can be readily produced using the disclosure provided herein in conjunction with information well known in the art. Substantially similar sequences can be obtained taking into account sequence information for a particular type of receptor obtained from different sources, different types of amino acids which are to some extent interchangeable, and the ease of experimentation with which functional receptor activity can be assayed.

Substantially similar sequences includes amino acid alterations such as deletions, substitutions, additions, and amino acid modifications. A "deletion" refers to the absence of one or more amino acid residue(s) in the related polypeptide. An "addition" refers to the presence of one or more amino acid residue(s) in the related polypeptide. Additions and deletions to a polypeptide may be at the amino terminus, the carboxy terminus, and/or internal. Amino acid "modification" refers to the alteration of a naturally occurring amino acid to produce anon-naturally occurring amino acid A "substitution" refers to the replacement of one or more ammo acid reside(s) by another amino acid reside(s) in the polypeptide. Derivatives can contain different combinations of alterations including more than one alteration and different types of alterations.

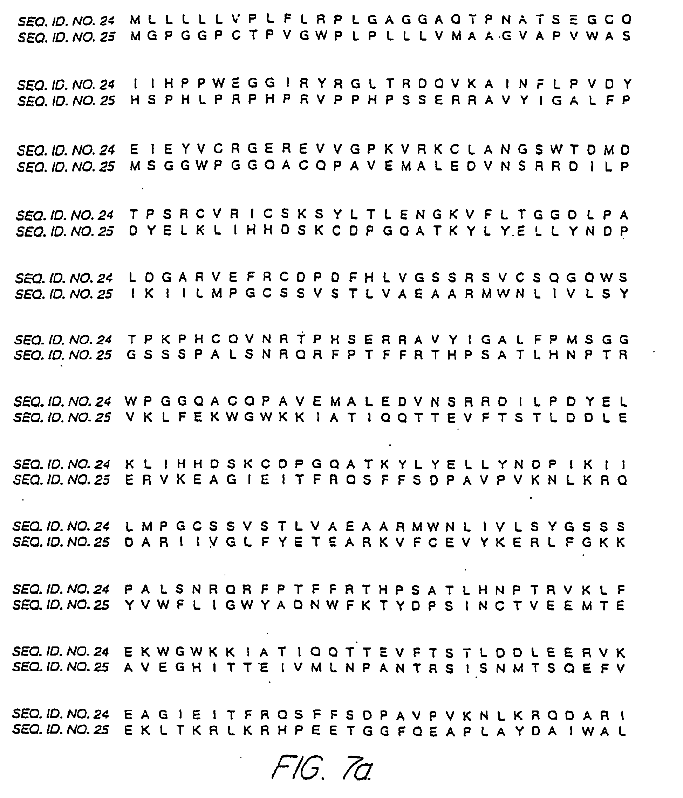

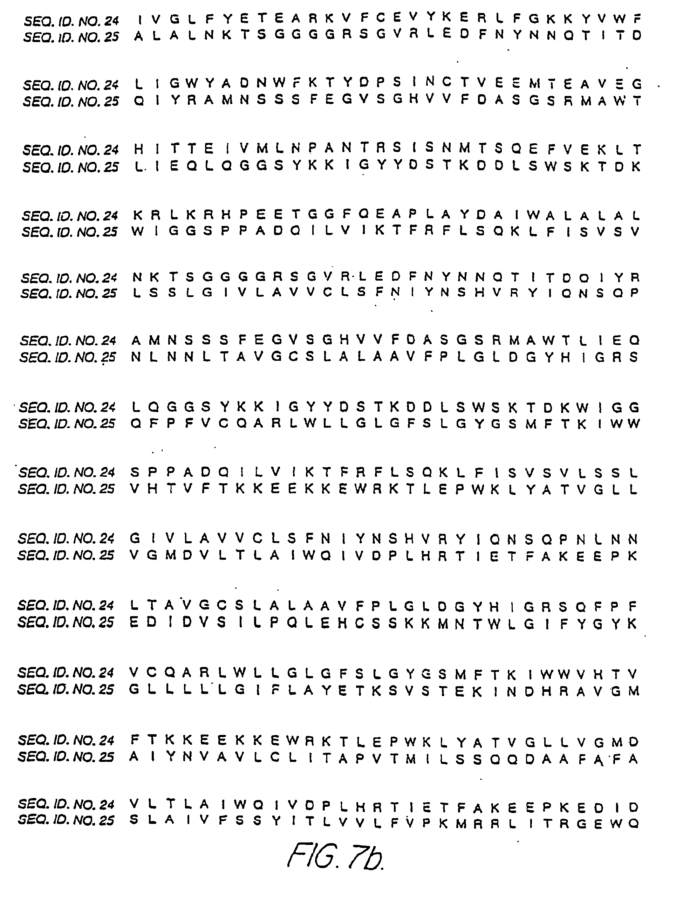

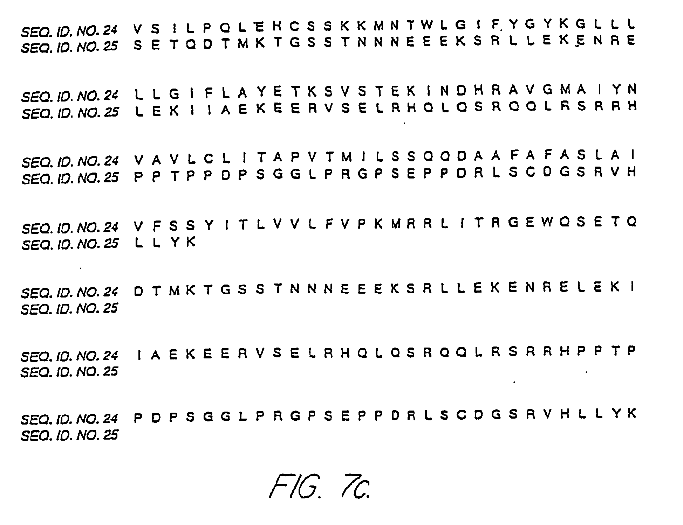

The sequences of polypeptides can be compared from different sources to help identify variable amino acids not essential for receptor activity For example, Figure 7 provides the rat GABA_{B}R1a and GABA_{B}R1b amino acid sequences. The rat GABA_{B}R1a and GABA_{B}R1b amino acid sequences can be compared with the human GABA_{B}R1a and GABA_{B}R1b sequences to identify conserved and variable amino acids. Derivatives can then be produced where a variable amino acid is changed, and receptor activity can be readily tested.

Similarly, the amino acid sequences for CaR, mGluR8, and G-proteins from different sources are either known in the art or can readily be obtained. Examples of such references are provided above.

While the effect of an amino acid change varies depending upon factors such as phosphorylation, glycosylation, intra-chain linkages, tertiary structure, and the role of the amino acid in the active site or a possible allosteric site, it is generally preferred that a substituted amino acid is from the same group as the amino acid being replaced. To some extent the following groups contain amino acids which are interchangeable: the basic amino acids lysine, arginine, and histidine; the acidic amino acids aspartic and glutamic acids; the neutral polar amino acids serine, threonine, cysteine, glutamine, asparagine and, to a lesser extent, methionine; the nonpolar aliphatic amino acids glycine, alanine, valine, isoleucine, and leucine (however, because of size, glycine and alanine are more closely related and valine, isoleucine and leucine are more closely related); and the aromatic amino acids phenylalanine, tryptophan, and tyrosine. In addition, although classified in different categories, alanine, glycine, and serine seem to be interchangeable to some extent, and cysteine additionally fits into this group, or maybe classified with the polar neutral amino acids.

While proline is a nonpolar neutral amino acid, its replacement represents difficulties because of its effects on conformation. Thus, substitutions by or for proline are not preferred, except when the same or similar conformational results can be obtained. The conformation conferring properties of proline residues may be obtained if one or more of these is substituted by hydroxyproline (Hyp).

Examples of modified amino acids include the following altered neutral nonpolar amino acids such as ω-amino acids of the formula H₂N(CH₂)ₙCOOH where n is 2-6, sarcosine (Sar), t-butylalanine (t-BuAla), t-butylglycine (t-BuGly), N-methyl isoleucine (N-MeIle), and noriencine (Nleu); altered neutral aromatic ammo acids soch as phenylglycine; altered polar, but neutral amino acids such as citrulline (Cit) and methionine sulfoxide (MSO); altered neutral and nonpolar amino acids such as cyclohexyl alanine (Cha); altered acidic amino acids such as cysteic acid (Cya); and altered basic ammo acids such as ornithine (Om).

Preferred derivatives have one or more amino acid alteration(s) which do not significantly affect the receptor activity of the related receptor protein. In regions of receptor domains not necessary for receptor activity, amino acids may be deleted, added or substituted with less risk of affecting activity, In regions required for receptor activity, amino acid alterations are less preferred as there is a greater risk of affecting receptor activity.

Derivatives can be produced using standard chemical techniques and recombinant nucleic acid techniques. Modifications to a specific polypeptide may be deliberate, as through site-directed mutagenesis and amino acid substitution during solid-phase synthesis, or maybe accidental such as through mutations in hosts which produce the polypeptide. Polypeptides including derivatives can be obtained using standard techniques such as those described by Sambrook *et al., Molecular Cloning,* Cold Spring Harbor Laboratory Press (1989). For example, Chapter 15 of Sambrook describes procedures for site-directed mutagenesis of cloned DNA.

### Receptor Nucleic Acid

G-protein fusion and chimeric receptor nucleic acid can be produced based on the information provided herein along with standard recombinant nucleic acid techniques. Examples of references describing recombinant nucleic acid techniques include *Molecular Cloning.* Sambrook *et al.,* Cold Spring Harbor Laboratory Press (1989); and *Current Protocols in Molecular Biology,* Frederick *et al.,* John Wiley & Sons, Inc. (1995).

Due to the degeneracy of the genetic code different nucleic acid sequences can encode for a particular polypeptide. Thus, a large number of nucleic acids encoding for a receptor having the same amino acid sequence can be produced.

### Recombinant Cells

Nucleic acid expressing a functional G-Protein fusion or a chimeric receptor can be used to create transfected cells lines expressing such receptors. Such cell lines have a variety of uses such as being used for high-throughput screening for compounds modulating receptor activity; being used to assay binding to the receptor; and as factories to produce large amounts of a receptor.

A variety of cell lines can couple exogenously expressed receptors to endogenous functional responses. Cell lines such as NIH-3T3, HeLa, NG115, CHO, HEK 293 and COS7 which are expected to lack CaR, mGluR8, and GABA_{B}R can be tested to confirm that they lack these receptors.

Production of stable transfectants can be accomplished by transfection of an appropriate cell line with, for example, an expression vector such as pMSG vector, in which the coding sequence for the G-protein fusion cDNA has been cloned. Expression vectors containing a promoter region, such as the mouse mammary tumor virus promoter (MMTV), drive high-level transcription of cDNAs in a variety of mammalian cells. In addition, these vectors contain genes for selecting cells stably expressing cDNA of interest. The selectable marker in the pMSG vectors encode an enzyme, xanthine-guanine phosphoribosyl transferase (XGPRT), conferring resistance to a metabolic inhibitor that is added to the culture to kill nontransfected cells.

The most effective method for transfection of eukaryotic cell lines with plasmid DNA varies with the given cell type. The expression construct will be introduced into cultured cells by the appropriate technique, such as Ca²⁺ phosphate precipitation, DEAE-dextran transfection, lipofection or electroporation. Expression of the receptor cDNA in cell lines can be assessed by solution hybridization and Northern blot analysis.

### Binding Assays

The present invention also includes using G-protein fusion receptors in a binding assay. G-protein fusion receptors can be used, for example to facilitate obtaining compounds able to bind to that particular receptor domain; and to determine whether a compound which binds to a particular domain.

Binding assays can be carried out using techniques well known in the art. Binding assays preferably employ radiolabeled binding agents.

### Therapeutic Modulation

As pointed out above, different types of diseases and disorders can be treated using compounds modulating CaR or mGluR activity. Additionally, such compounds can be used prophylactically. Patients are mammals, preferably humans.

Modulators of CaR or mGluR activity can be administered to a patient using standard techniques. Techniques and formulations generally may be found in Remington's Pharmaceutical Sciences,18th ed., Mack Publishing Co., Easton, PA, 1990 (hereby incorporated by reference herein).
Suitable dosage forms, in part, depend upon the use or the route of entry, for example, oral transdermal, transmucosal, or by injection (patenteral). Such dosage forms should allow the therapeutic agent to reach a target cell whether the target cell is present in a multicellular host or in culture. For example, pharmacological compounds or compositions injected into the blood stream should be soluble. Other factors are well known in the art, and include considerations such as toxicity and dosage forms which retard the therapeutic agent from exerting its effect.

Therapeutic compounds can be formulated as pharmaceutically acceptable salts and complexes thereof. Pharmaceutically acceptable salts are non-toxic salts in the amounts and concentrations at which they are administered. The preparation of such salts can facilitate the pharmacological use by altering the physical characteristics of the compound without preventing it from exerting its physiological effect. Useful alterations in physical properties include lowering the melting point to facilitate transmucosal administration and increasing the solubility to facilitate administering higher concentrations of the drug.

The pharmaceutically acceptable salt of a compound may be present as a complex. Examples of complexes include an 8-chlorotheophylline complex (analogous to, *e.g.,* dimenhydrinate:diphenhydramine 8-chlorotheophylline (1:1) complex; Dramamine) and various cyclodextrin inclusion complexes.

Pharmaceutically acceptable salts include acid addition salts such as those containing sulfate, hydrochloride, fumarate, maleate, phosphate, sulfamate, acetate, citrate, lactate, tartrate, methanesulfonate, ethanesulfonate, benzenesulfonate, *p-*toluene-sulfonate, cyclohexylsulfamate and quinate.

Pharmaceutically acceptable salts can be obtained from acids such as hydro-chloric acid, maleic acid, sulfuric acid, phosphoric acid, sulfamic acid, acetic acid, citric acid, lactic acid, tartaric acid, malonic acid, methanesulfonic acid, ethanesulfonic acid, benzenesulfonic acid, *p*-toluenesulfonic acid, cyclohexyls-ulfamic acid, fumaric acid, and quinic acid.

Pharmaceutically acceptable salts also include basic addition salts such as those containing benzathine, chloroprocaine, choline, diethanolamine, ethylenediamine, meglumine, procaine, aluminum, calcium, lithium, magnesium, potassium, sodium, ammonium, alkylamine, and zinc, when acidic functional groups, such as carboxylic acid or phenol are present. For example, see Remington's Pharmaceutical Sciences, 18th ed., Mack Publishing Co., Easton, PA, p.1445,1990. Such salts can be prepared using the appropriate corresponding bases.

Camera or excipients can also be used to facilitate administration of therapeutic agents. Examples of carriers include calcium carbonate, calcium phosphate, various sugars such as lactose, glucose; or sucrose, or types of starch, cellulose derivatives, gelatin, vegetable oils, polyethylene glycols and physiologically compatible solvents. Examples of physiologically compatible solvents include sterile solutions of water for injection (WFI), saline solution and dextrose.

Compounds can be administered by different routes including intravenous, intraperitoneal, subcutaneous, intramuscular, oral, topical (transdermal), or transmucosal administration. For systemic administration, oral administration is preferred. For oral administration, for example, the compounds can be formulated into conventional oral dosage forms soch as capsules, tablets, and liquid preparations such as syrups, elixirs, and concentrated drops.

Alternatively, injection (parenteral administration) may be used, *e.g.,* intramuscular, intravenous, intraperitoneal, and subcutaneous. For injection, compounds are formulated in liquid solutions, preferably, in physiologically compatible buffers or solutions, such as saline solution, Hank's solution, or Ringer's solution. In addition, the compounds may be formulated in solid form and redissolved or suspended immediately prior to use. Lyophilized forms can also be produced.

Systemic administration can be by transmucosal or transdermal means. For transmucosal or transdermal administration, penetrants appropriate to the battier to be permeated are used in the formulation. Such penetrants are well known in the art, and include, for example, for transmucosal administration, bile salts and fusidic acid derivatives. In addition, detergents may be used to facilitate permeation. Transmucosal administration, for example, may be through nasal sprays, rectal suppositories, or vaginal suppositories.

For topical administration, compounds can be formulated into ointments, salves, gels, or creams, as is well known in the art.

The amounts of various compounds to be administered can be determined by standard procedures taking into account factors such as the compound IC₅₀, EC₅₀, the biological half-life of the compound, the age, size and weight of the patient, and the disease or disorder associated with the patient. The importance of these and other factors to be considered are well known to those of ordinary skill in the art. Generally, the amount is expected to preferably be between about 0.01 and 50 mg/kg of the animal to be treated.

### EXAMPLES

Examples are provided below illustrating different aspects and embodiments of the present invention. The examples include techniques that can be used to produce and use G-protein fusion receptors and chimeric receptors. These examples are not intended to limit the claimed invention.

### Example 1: Construction of G-Protein Fusions

This example illustrates different G-protein fusion receptor constructs and techniques used to produce different G-protein fusion receptor constructs. Numbering of nucleotide position for all the following constructs is such that nucleotide number 1 corresponds to the A of the ATG start codon of the nucleotide sequence encoding the designated protein. Constructs ph8SPmGluR4, ph2SPmGluR3, ph8SPmGluR4//CaR*AAA*Gα_{q}i5 and ph2SPmGluR3//CaR*AAA*Gα_{q}i5 are examples of the present invention, the remaining constructs are comparative examples.

### I.FULL-LENGTH CONSTRUCTS

### A. phCaR

The cDNA encoding the human CaR (Garrett et al., (1995) J. Biol. Chem.270:12919) is harbored in the Bluescript SK(-) plasmid (Stratagene). This construct is referred to as phCaR.

### B. phmGluR2

A full length human mGluR2 cDNA was amplified from human cerebellum MarathonReady cDNA (Clontech) using PCR primers based on the human mGluR2 cDNA sequence (Genbank Accession # 4504136). The obtained PCR fragment was subcloned into the pT7Blue TA vector (Novagen). A Hind III-Not I fragment containing the human mGluR2 cDNA. was then subcloned into the Bluescript SKII(-) plasmid (Stratagene). This construct is referred to as phmGluR2.

### C. phGα_{q}

A full length human Gα_{q} cDNA was amplified from human cerebral cortex Quick-Clone cDNA (Clontech) using PCR primers based on the human Gα_{q} cDNA sequence (Genbank Accession # 4504044). The obtained PCR fragment was subcloned into the Bluescript SKII(-) plasmid (Stratagene). This construct is referred to as phGα_{q}.

### D. phmGluR8

The cDNA encoding the full length human mGluR8 cDNA (Stormann et al., U.S. Patent Nos. 6,051,688,6,077,675, and 6,084,084) is harbored in the Bluescript SKII(-) plasmid (Stratagene). This construct is referred to as phmGluR8.

### E. ph8SPmGluR4

A full length human mGIuR4 cDNA was amplified from human cerebellum MarathonReady cDNA (Clontech) using PCR primers based on the human mGluR4 cDNA sequence (Genbank Accession #X80818). The obtained PCR fragment was cloned into the pT7Blue TA vector (Novagen). A 2977 bp BamHI fragment containing the human mGluR4 cDNA was then subcloned into the vector pcDNA3.1/Hygro+ (Invitrogen). This construct is referred to as phmGluR4.

Next, the predicted signal peptide of mGluR4 was replaced with the predicted signal peptide and 87 bp of 5' UTR from phmGluR8 using a recombinant PCR strategy similar to those described above. The first reaction used a phmGluR8 construct with two primers, 3.1-535F (sense 21-mer, complementary to vector sequence upstream of the hmGluR8 insert; sequence 5'-ggcattatgcccagtacatga-3') (SEQ. ID NO. 51), and the hybrid primer 8/4RP (antisense 42-mer, containing 21 nucleotides complementary to human mGluR8 and 21 nucleotides complementary human mGluR4; sequence 5'-caagcctctcttcccaggcattttctccacaggtggtattgc-3') (SEQ. ID NO. 52). These primers were used to amplify a 469 bp PCR fragment of human mGluR8.

In a separate PCR reaction using phmGluR4 as template, a 472 bp fragment of human mGluR4 was amplified using a hybrid primer 4/8RP (sense 42-mer, exactly complementary to primer 8/4RP) and oligo mG4-472R, (antisense 18-mer, complementary to the human mGluR4 cDNA; sequence 5'-ctgaagcaccgatgacac-3') (SEQ. ID NO. 53). The two PCR products generated from the above two reactions were annealed together in equimolar ratios in the presence of the external primers mG4-472R and 3.1-535F, and Turbo Pfu DNA polymerase (Stratagene).

The resulting chimeric PCR product was digested with Narl and NheI (New England Biolabs) and subcloned into phmGluR4 digested with the same two restriction enzymes. The sequence of the resultant chimeric construct, ph8SPmGluR4, was verified by ABI automated DNA sequence analysis.

The replacement of the predicted signal peptide of mGluR4 with that of mGluR8 greatly increased the activity of this receptor in *in vitro* assays

### F. ph2SPmGluR3

A full length human mGluR3 cDNA was amplified from human hippocampus MarathonReady cDNA (Clontech) using PCR primers based on the human mGluR3 cDNA sequence (Genbank Accession #NM 000840) and cloned into the pBluescript SKII(-) plasmid (Sbratagene). This construct is referred to as phmGluR3/pSK.

The predicted signal peptide of mGluR was replaced with the predicted signal peptide and 40 bp of 5' UTR from phmGluR2 using a recombinant PCR strategy exactly analogous to that described above. The first reaction used a phmGluR2 construct with two primers, 3.1-535F (sense 21-mer, complementary to vector sequence upstream of the hmGluR2 insert; sequence 5'-ggcattatgcccagtacatga-3').(SEQ. ID NO. 51), and the hybrid primer SP2/3RP (antisense 42-mer, containing 21 nucleotides complementary to human mGluR2 and 21 nucleotides complementary human mGluR3; sequence 5'-tagaaagttatggtcccctaaagccacagcaccccacagcag-3') (SEQ. ID NO. 62). These primers were used to amplify an approximately 500 bp PCR fragment of human mGluR2.

In a separate PCR reaction using phmGluR3//CaR in pcDNA3.1/Hygro(+) (See construction of this chimeric construct phmGluR3//CaR, below) as template, an approximately 1000 bp fragment of human mGluR3 was amplified using a hybrid primer 3/SP2RP (sense 42-mer, exactly complementary to primer SP2/3RP) and oligo 3-1086R, (antisense 19-mer, complementary to the human mGluR3 cDNA; sequence 5'-ctggaggctgcactgaaac-3')(SEQ. ID NO. 63). The two PCR products generated from the above two reactions were annealed together in equimolar ratios in the presence of the external primers 3-1086R and 3.1-535F, and Turbo Pfu DNA polymerase (Stratagene).

The resulting chimeric PCR product was digested with NarI and NheI (New England Biolabs) and subcloned into phmGluR3//CaR digested with the same two restriction enzymes. The sequence of the resultant chimeric construct, ph2SPmGluR3//CaR, was verified by ABI automated DNA sequence analysis.

The replacement of the predicted signal peptide of mGluR3 with that of mGluR2 greatly increased the activity of the chimeric receptor in *in vitro* assays.

The full-length mGluR3 receptor construct with the signal peptide and 5'UTR of mGluR2 was then constructed by replacing the 3' half of ph2SPmGluR3//CaR with that from phmGluR3/pSK Specifically, a 1002 nt fragment digested with Bsu36I (at position 1691 in mGluR3 coding DNA sequence (CDS)) and NotI(in vector) was subcloned in place of a 1484 nt fragment dropped from the ph2SPmGluR3//CaR plasmid digested with the same restriction enzymes. The sequence of the resultant construct, ph2SPmGluR3 in pcDNA3.1/Hygro(+), was verified by ABI automated DNA sequence analysis.

### II. Gα_{q}i5

The cDNA encoding the human Gα_{q}i5 cDNA (Conklin et al (1993) Nature 363:274-77) is harbored in the Bluescript SKH(-) plasmid (Stratagene). This construct is referred to as Gα_{q}i5. The nucleic acid and amino acid sequences for Gα_{q}i5 are provided by SEQ. ID. NOs. 28 and 29 respectively.

### III. phCaR/hmGluR2

This chimera contains the extracellular domain of the human CaR and transmembrane domain and intracellular cytoplasmic tail of human mGluR2. The chimeric junction between the CaR and hmGlnR2 was created using a recombinant PCR strategy similar to those described above.

The first reaction used two primers, CA1156 (sense 19-mer, corresponding to nucleotides 1156-1174 of human CaR), and the hybrid primer CA/2 (antisense 42-mer, containing 21 nucleotides complementary to nucleotides 1774-1794 of human CaR and 21 nucleotides complementary to nucleotides 1660-1680 of the human mGluR2). These primers were used to amplify a 659 bp PCR fragment of human CaR,

In a separate PCR reaction using phmGluR2 as template, a 692 bp fragment of the human mGluR2 was amplified using a hybrid primer 2/CA (sense 42-mer, exactly complementary to primer CA/2) and oligo 2-2330m, (antisense 23-mer, complementary to nucleotides 2309-2331 of the human mGluR2 eDNA). The two PCR products generated from the above two reactions were annealed together in equimolar ratios in the presence of the external primers CA1156 and 2-2330m, and the Pfu DNA polymerase (Stsatagene).

The resulting chimeric PCR product was digested with SexA1 (Boehringer Mannheim) and BamHI (New England Biolabs) and subcloned into phCaR digested with the same two restriction enzymes. In the final cloning step, the 3' end of human mGluR2 was subcloned into this construct using the restriction enzymes BsrGI and BamHI (both New England Biolabs). The sequence of the resultant chimeric construct, phCaR/hmGluR2, was verified by ABI automated DNA sequence analysis.

### IV. phCaR/hmGluR2*Gqi5

This construct contains the phCaR/hmGluR2 chimeric receptor fused to human Gα_{q}i5. A HindIII-BamHI fragment containing the phCaR/hmGluR2 construct was subcloned into pcDNA3.1/Hygro(+) (Invitrogen) to aid in constructing this fusion protein. The chimeric junction between the C-terminns of phCaR/hmGluR2 and the N-terminus of Gα_{q}i5 was created using a recombinant PCR strategy similar to those described above.

The first reaction, used two primers, 2-1713 (sense 21-mar, corresponding to nucleotides 1710-1730 of human mGluR2) and the hybrid primer 2/Q (antisense 42-mar, containing 21 nucleotides complementary to nucleotides 2596-2616 of human mGluR2, and 21 nucleotides complementary to nucleotides 1-21 of pGα_{q}i5). These primers were used to amplify a 927 bp PCR fragment of phCaR/hmGluR2. In a separate PCR reaction all of Gα_{q}i5 was amplified using a hybrid primer Q/2 (sense 42-mer, exactly complementary to primer 2/Q) and the and the T3 primer commercially available from Stratagene.

These two PCR products generated from the above two reactions were annealed together in equimolar ratios in the presence of the external primers 2-1713 and T3, and the Pfu DNA polymerase (Stiaiagene). The resulting chimeric PCR product was digested with Bsu361 and BamHI (New England Biolabs) and subcloned into phCaR/hmGluR2 digested with the same two restriction enzymes. The sequence of the resultant chimeric fusion construct, phCaR/hmGluR2*Gα_{q}i5, was verified by DNA sequence analysis.

### V. phmGluR2//CaR Construct

This chimera contains the extracellular and transmembrane domains of human mGluR2 linked to the intracellular cytoplasmic tail domain of the human CaR. The chimeric junction was created using three separate PCR reactions.

The first reaction used two primers, 2-1713 (sense 21-mer, corresponding to nucleotides 1710-1730 of human mGluR2, Genbank Accession # 4504136) and the hybrid primer 2/CT (antisense 42-mer, containing 21 nucleotides complementary to nucleotides 2452 - 2472 of human mGluR2 and 21 nucleotides complementary to nucleotides 2602-2622 of the human CaR). These primers were used to amplify a 783 bp PCR fragment of human mGluR2. In a separate PCR reaction using phCaR in the BlueScript SK⁻ plasmid as template, a 750 bp fragment of the human CaR was amplified using a hybrid primer CT/2 (sense 42-mer, exactly complementary to primer 2/CT) and the T3 primer commercially available from Stratagene.

The two PCR products generated from the above two reactions were annealed together in equimolar ratios in the presence of the external primers 2-1713 and T3, and the Pfu DNA polymerase (Stratagene). The resulting chimeric PCR product was digested with BsrG I and Not I (New England Biolabs) and subcloned into pmGluR2 digested with the same two restriction enzymes. The sequence of the resultant dimeric construct, pmGluR2//CaR, was verified by ABI automated DNA sequence analysis.

### VL pmGluR2//CaR*Gα_{q}i5 Construct

This construct contains the hmG1uR2//CaR chimeric receptor fused to human Gα_{q}i5. The chimeric junction between the C-terminus of hmGluR2//CaR and the N-terminns of Gα_{q}i5 was created using a recombinant PCR strategy similar to that described above for the construction of phmGluR2//CaR.

The first reaction used two primers, CRP10A (sense 18-mer, corresponding to nucleotides 2812-2829 of phCaR) and the hybrid primer CaRQ (antisense 42-mer, containing 21 nucleotides complementary to nucleotides 3214- 3234 phCaR, and 21 nucleotides complementary to nucleotides 1-21 of pGα_{q}i5). These primers were used to amplify a 443 bp PCR fragment of hmGluR2//CaR. In a separate PCR reaction, all of Gα_{q}i5 was amplified using a hybrid primer QCaR (sense 42-mer, exactly complementary to primer CaRQ) and the T3 primer commercially available from Stratagene.

The two PCR products generated from the above two reactions were annealed together in equimolar ratios in the presence of the external primers CRP10A and T3, and the Pfu DNA polymerase (Stratagene). The resulting chimeric PCR product was digested with BstE II and Not I (New England Biolabs) and subcloned into pmGluR2//CaR digested with the same two restriction enzymes. The sequence of the resultant chimeric fusion construct, pmGluR2//CaR*Gα_{q}i5, was verified by ABI automated DNA sequence analysis.

### VII. Fusion Receptor Protein Linker Addition Constructs

### A, phmGluR2//CaR*AAA*Gα_{q}i5

A linker encoding three alanine residues was incorporated into the phmGluR2//CaR*Gα_{q}i5 construct by mutagenesis (Stratagene QuickChange Mutagenesis Kit). A sense 40-mer, 2CQ+LP, contained 16 nucleotides corresponding to 3219-3234 of human CaR, followed by the 9 nucleotide sequence (GCGGCCGCC) encoding three alanine residues and a NotI restriction enzyme site, and then 15 nucleotides corresponding to nucleotides 1-15 of Gα_{q}i5. 2CQ+LP was annealed to an antisense 40-mer, 2CQ+LM, the exact complement of 2CQ+LP. These oligos were used in the mutagenesis reaction according to the manufacture's protocol. Restriction enzyme analysis and DNA sequence analysis confirmed the insertion of the 9 nucleotide linker (GCGGCCGCC) between the C-terminus of phmGuR2//CaR and the N-terminus of Gα_{q}i5. This construct was designated phmGluR2//CaR*AAA*Gα_{q}i5.

### B. Human GABA_{B}R2*AAA*Gα_{q}o5 and human GABA_{B}R1a*AAA*Gα_{q}o5

These constructs contain the human GABA_{B}R2 (hGAHA_{B}R2: Genbank Accession # AJ 012188) and human GABA_{B}R1a (hGABA_{B}R1a: Genbank Accession # AJ 012185) fused at their C-terminus to the N-terminus of human Gα_{q}o5 (hGα_{q}o5: Nature 363:274-276, 1993). Human GABA_{B}R2, hGABA_{B}R1a, and hGα_{q}o5 were cloned into the plasmid pcDNA3.1/Hygro+ (Invitrogen) and are designated phGABA_{B}R2, pbGABA_{B}RIa, and phGα_{q}o5. The first reaction used two primers, XemI-R2 (sense 20-mer, corresponding to nucleotides 2650-2669 of phGABA_{B}R2) and the hybrid primer R2/Go5(-) (antisense 45-mer, containing 18 nucleotides complementary to nucleotides 2806-2823 of phGABA_{B}R2 and 18 nucleotides complementary to nucleotides 1-18 of hGα_{q}o5). These two complementary areas flank a 9 nucleotide sequence coding for 3 alanine sequences with a unique NotI restriction site. These primers were used to amplify a 200 base-pair PCR fragment.

In a separate PCR reaction, part of hGα_{q}o5 was amplified using a hybrid primer R2/Gα_{q}o5(+) (sense 45-mer), exactly complementary to R2/Go5(-) and XbaI-Go5 primer (22-mer containing 22 nucleotides complementary to nucleotides 873-895 of hGα_{q}o5) These primers were used to amplify a 914 base-pair PCR product. The two PCR products generated from the above two reactions were annealed together in equimolar ratios in the presence of the external primers; XcmI-R2 and XbaI-Go5, and Pfu polymerase (Stratagene).

The resulting chimeric PCR product was digested with the restriction endonucleases XcmI and XbaI (New England Biolabs) and subcloned into phGABA_{B}R2 digested with the same two restriction enzymes. The resulting clone was then digested with HindIII and XbaI and subcloned into phGα_{q}o5 cut with HindIII and XbaI resulting in the chimeric hGABA_{B}R*AAA*Gα_{q}o5. The chimeric junction between the C-terminus hGABA_{B}R1a, the Ala linker, and the N-terminus of hGα_{q}o5 was created using a recombinant PCR strategy similar to those described above.

To construct hGABA_{B}R1a*AAA*Gqo5, the first reaction used a commercially available T7 primer (Novagen) and the NtI hGBR1 primer (CAGAGTCATGGCGGCCGCCTTATAAAGCAAATGCACTCG) (SEQ. ID NO. 54) corresponding to nucleotide numbers 1-9 of hGα_{q}o5 and nucleotide numbers 2863-2883 of hGABA_{B}R1a.

### C. phmGluR8//CaR*AAA*Gα_{q}i5

A linker encoding three alanine residues was incorporated into the phmGluR8//CaR*Gα_{q}i5 construct by mutagenesis (Stratagene QuickChange Mutagenesis Kit), exactly as described in Section A, above to create phmGluR2//CaR*AAA*Gα_{q}i5. The same primers, 2CQ+LP and 2CQ+LM, were used for this mutagenesis. Restriction enzyme analysis and DNA sequence analysis confirmed the insertion of the 9-nucleotide linker (GCGGCCGCC) between the C-terminus ofphmGluR8//CaR and the N-terminus of Gα_{q}i5. This construct was designated phmGluR8//CaR*AAA*Gα_{q}i5.

### D. ph8SPmGluR4//CaR*AAA*Gα_{q}i5

This chimera contains the extracellular and transmembrane domains of the human 8SPmGluR4 construct and intracellular cytoplasmic tail of human CaR fused to Gα_{q}i5 through the three alanine residue linker.

The chimeric junction between the human 8SPmGluR4 and hCaR was created using a recombinant PCR strategy similar to those previously described. The first reaction used two primers, mG4-2028R (sense 19-mer, corresponding to nucleotides of human 8SPmGluR4; sequence5'-catctaccgcatcttcgag-3') (SEQ. ID NO. 55), and the hybrid primer 4CT (antisense 42-mer, containing 21 nucleotides complementary to human 8SPmGluR4 and 21 nucleotides complementary human CaR; sequence 5'-acgcacetcctcgatggtgttctgetccgggtggaagaggat -3') (SEQ. ID NO. 56). These primers were used to amplify a 549 bp PCR fragment from human 8SPmGluR4.

In a separate PCR reaction, using phmGluR2//CaR*AAA*Gα_{q}i5 as a template, a 743 bp fragment of the human CaR*AAA*Gα_{q}i5 was amplified using the hybrid primer CT4 (sense 42-mer, exactly complementary to primer 4CT) and oligo Gaqi58R, (antisense 21-mer, complementary to Gα_{q}i5 cDNA; sequence 5'- ctcgatctcgtegtteatccg -3') (SEQ. ID NO. 57). The two PCR products generated from the above two reactions were annealed together in equimolar ratios in the presence of the external primers mG4-2028R and Gaqi58R, and Pfu DNA polymerase (Stratagene).

The resulting chimeric PCR product was digested sequentially with KpnI and NotI (New England Biolabs) and subcloned into ph8SPmGluR4 prepared with the same two restriction enzymes. This intermediate construct was known as ph8SPmGluR4//CaR(no stop). In the final cloning step, a fragment containing the Gα_{q}i5 cDNA was released from phmGluR8//CaR*AAA*Gα_{q}i5 using the restriction enzymes ApaI and NotI (both New England Biolabs) and subcloned into the ph8SPmGluR4//CaR(no stop) construct, which was prepared with the same restriction enzymes. The sequence of the resultant chimeric construct, ph8SPmGluR4//CaR*AAA*Gα_{q}i5, was verified by ABI automated DNA sequence analysis.

### E. ph2SPmGluR3//CaR*AAA*Gα_{q}i5

This chimera contains the extracellular and transmembrane domains of the human 2SPmGluR3 construct and intracellular cytoplasmic tail of human CaR fused to Gα_{q}i5 through the three alanine residue linker.

The chimeric junction between the human mGluR3 and hCaR was created using a recombinant PCR strategy similar to those previously described. The first reaction used two primers, 3-2032 (sense 18-mer, corresponding to nucleotides of human mGluR3; sequence 5'-ccaaaattcatcagcccc-3') (SEQ. ID NO. 64), and the hybrid primer 3/CT (antisense 42-mer, containing 21 nucleotides complementary to human mGluR3 and 21 nucleotides complementary human CaR; sequence 5'-gcaacgcacctcctcgatggtattcttctggggttgaaacag-3') (SEQ. ID NO. 65). These primers were used to amplify a 479 bp PCR fragment from human mGluR3.

In a separate PCR reaction, using human CaR in pBluescript SK(-) as a template, a 756 bp fragment of the human CaR was amplified using the hybrid primer CT/3 (sense 42-mer, exactly complementary to primer 3/CT) and oligo T3, (antisense 20-mer, complementary to vector sequence; sequence 5'-attaaccctcactaaaggga-3') (SEQ. ID NO. 66). The two PCR products generated from the above two reactions were annealed together in equimolar ratios in the presence of the external primers 3-2032 and T3, and Pfu DNA polymerase (Stratagene).

The resulting chimeric PCR product was digested with AccI and NotI (New England Biolabs) and subcloned into hmGluR3 prepared with the same two restriction enzymes. This construct was known as phmGluR3//CaR.

As detailed under the Full-length Constructs Examples, the 5' UTR and signal peptide of hmGluR2 was then added to this chimeric construct to produce ph2SPmGluR3//CaR.

Next, mutagenesis was performed (Stratagene QuickChange Mutagenesis Kit) to replace the stop codon of ph2SPmGluR3//CaR with a three alanine linker (also creating a Not I site) to facilitate addition of the Gα_{q}i5 coding sequence. The two mutagenic primers used were CNOTP (sense 42mer, sequence 5'-gaaaacgtagtga.attcagcggccgcaatggaaggagaagac-3') (SEQ. ID NO. 67) and CNOTM (antisense 42mer, complementary to CNOTP). This intermediate construct was known as ph2SPmGluR3//CaR(no stop).

In the final cloning step, a fragment containing the Gα_{q}i5 cDNA was released from phmGluR8//CaR*AAA*Gα_{q}i5 using the restriction enzymes ApaI and NotI (both New England Biolabs) and subcloned into the ph2SPmGluR3//CaR(no stop) construct, which was prepared with the same restriction enzymes. The sequence of the resultant chimeric construct, ph2SPmGluR3//CaR*AAA*Gα_{q}i5, was verified by ABI automated DNA sequence analysis.

### VIII. phmGluR8//CaR Construct

This chimera contains the extracellular and transmembrane domains of human mGluR8 linked to the intracellular cytoplasmic tail domain of the human CaR. The chimeric junction between hmGluR8 and the CaR was created using a recombinant PCR strategy similar to those described above.

The first reaction used two primers, CH5A (sense 19-mer, corresponding to nucleotides 2187-2245 of human mGluR8, Stormann et al., U.S. Patent Nos. 6,051,688, 6,077,675, and 6,084,084) and the hybrid primer CH5B (antisense 40-mer, containing 22 nucleotides complementary to nucleotides 2523-2544 of human mGluR8, and 18 nucleotides complementary to nucleotides 2602-2619 of the human CaR). These primers were used to amplify a 375 bp PCR fragment of human mGluR8. In a separate PCR reaction using phCaR in the BlueScript SK(-) plasmid as template, a 750 bp fragment of the human CaR was amplified using a hybrid primer CH5C (sense 40-mar, exactly complementary to primer CH5B) and the T3 primer commercially available from Stratagene.

The two PCR products generated from the above two reactions were annealed together in equimolar ratios in the presence of the external primers CH5A and T3, and the Pfu DNA polymerase (Stratagene). The resulting chimeric PCR product was digested with BsrG I and Xba I (New England Biolabs) and subcloned into pmGluR8 digested with the same two restriction enzymes. The sequence of the resultant chimeric construct, pmGluR8//CaR, was verified by DNA sequence analysis.

### IX. mGluR8//CaR*Gα_{q}i5 Construct

This construct contains the hmGluR8//CaR chimeric receptor fused to human Gα_{q}i5. The chimeric junction between the C-tenmnus of lumGlnR8//CaR and the N-terminns of Gα_{q}i5 was created using a recombinant PCR strategy similar to that described above for the construction of phmGluR2//CaR*Gα_{q}i5.

The first reaction used two primers, CRP10A (sense 18-mer, corresponding to nucleotides 2812-2829 of phCaR) and the hybrid primer Gqi5/CaR. (antisense 40-mer, containing 21 nucleotides complementary to nucleotides 3214-3234 phCaR, and 19 nucleotides complementary to nucleotides 1-19 of pGα_{q}i5). These primers were used to amplify a 441 bp PCR fragment of hmGluR8//CaR.

In a separate PCR reaction all of Gα_{q}i5 was amplified using a hybrid primer CaR/Gqi5 (sense 40-mer, exactly complementary to primer Gqi5/CaR) and the Apa I-mut primer (20-mer). The two PCR products generated from the above two reactions were annealed together in equimolar ratios in the presence of the external primers CRP10A and Apa I-mut, and the Pfu DNA polymerase (Stratagene).

The resulting chimeric PCR product was digested with BstE II and Apa I (New England Biolabs) and subcloned into pmGluR8//CaR digested with the same two restriction enzymes. The sequence of the resultant chimeric fusion construct, pmGluR8//CaR*Gα_{q}i5, was verified by DNA sequence analysis.

### Example 2: Functional Expression of CaR/GAB_{B}R2

In vitro transcribed RNA (7 ng) encoding a chimeric CaR/GABA_{B}R2 (CaR extracellular and transmembrane domains, and intracellular GABA_{B}R2 domain) was coinjected with in vitro transcribed RNA (2 ng) encoding G_{α}15 into *Xenopus* oocytes. Following a 72-hour incubation, the oocytes were voltage-clamped using standard electrophysiological techniques (Hille, B., Ionic Channels of Excitable Membranes, pp.30-33, Sinauer Associates, Inc., Sunderland, Ma., 1992). Activation of the chimeric receptor was detected by increases in the calcium activated chloride current.

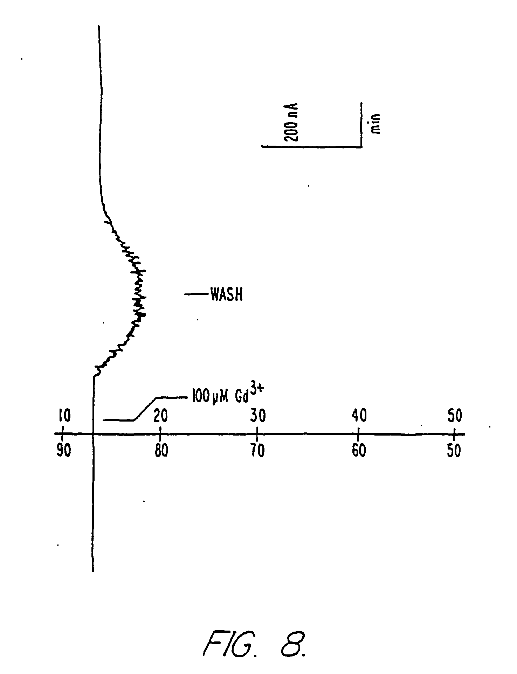

Application of the CaR activator 100 µM Gd³⁺, resulted in reversible, oscillatory increases in the caleium-activated chloride current as shown in Figure 8. These data demonstrate the functional response of the chimeric CaR/GABA_{B}R2 receptor upon activation via a site within the CaR extracellular domain. In this assay, the G_{α}15 subunit acts to promote signal transduction through intracellular pathways that mobilize intracellular Ca⁺⁺.

### Example 3: Expression of Different G-Protein Fusion Receptors

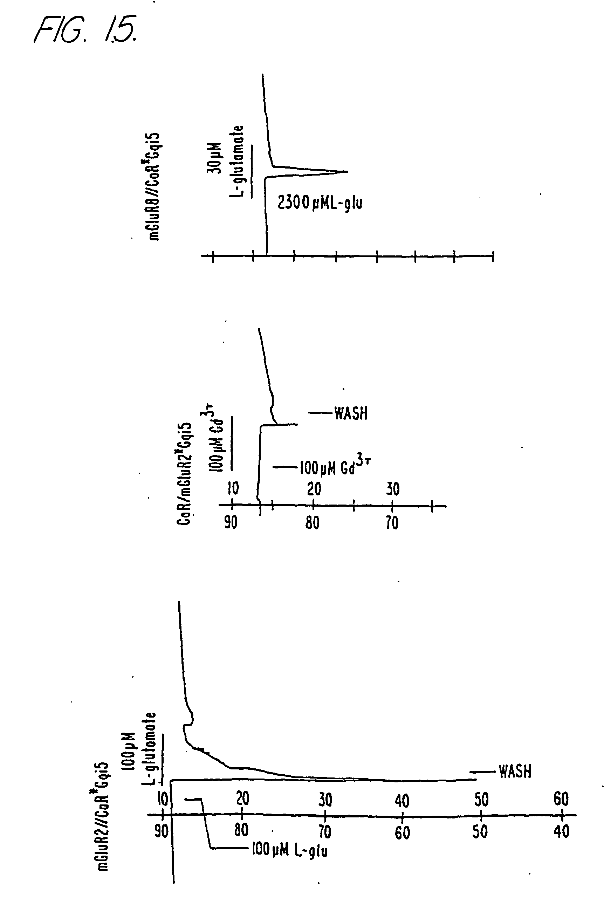

The ability of different G-protein fusions to transduce signal resulting from ligand binding is shown in Figure 15. The different G-protein fusion receptors used in this example were as follows: mGluR2//CaR Gqi5 (SEQ. ID. NO. 37), CaR/mGluR2*Gqi5 (SEQ. ID. NO. 33), andmGluR8//CaR*Gqi5 SEQ. ID. NO. 41.

Oocytes suitable for injection were obtained from adult female Xenopus laevis toads using procedures described in C.J, Marcus-Sekura and M. J. M. Hitchcock, Methods in Enzymology, Vol. 152 (1987).

Receptor fusion cRNAs were dissolved in water and 50 nl (12.5 ng/oocyte) were injected into individual oocytes. Following injection, oocytes were incubated at 16°C in MBS containing 1 mM CaCl₂ for 2 to 7 days prior to electrophysiological recording.

Test substances were applied by superfusion at a flow rate of about 5 ml/min. Receptor fusion activation was determined by measuring the increase in calcium-activated chloride current (I_{C1}). Increases in I_{C1} were quantified by measuring the peak inward current stimulated by activating agent, relative to the holding current at -60 mV. Application of 100 µM L-glutamate elicited a response from the mGluR2//CaR*Gαqi5 and mGluR8//CaR*Gαqi5. Application of 100 µM Gd³⁺ activated the CaR/mGluR2*Gqi5.

### Example 4: Expression of Different G-Protein Fusion Receptors in Mammalian Cells

HBK293 cells were transiently transfected with the p8SPhmGluR4//CaR*AAA*Gαqi5 or phmGluR8//CaR*Gαqi5 plasmid DNAs using the following protocol. Initially, 150 cm² tissue culture flasks containing BEK293 cells at 75% confluence were transfected with 24 ug of plasmid DNA using Gibco BRL Life Technologies' Lipofectamine reagent. Following liposomal gene delivery the cells were allowed to recover for 24 hours. They were then plated overnight at 100,000 cells per well in blank, clear bottom, Collagen I coated 96 well plates (Becton Diekenson, Biocoat) using DMEM supplemented with 10% fetal bovine serum (Hyclone Laboratories). The cells were assayed for function 48 hours after transient tranfection.

On the day of the assay, tissue culture medium was aspirated from the wells of a 96-well plate and 80 µL of Assay Buffer (Assay Buffer is: 20 mM HEPBS, 146 mM NaCl, 5 mM KCl, 1 mM MgCl₂, 1 mM CaCl₂, 1 mg/ml BSA, 1 mg/ml glucose, pH 7.4) supplemented with 6 µM of the Ca²⁺-sensitive dye, Fluo-3 AM (Molecular Probes) and 0.025% Pluronic (Molecular Probes) was added to each well.

The plate was then incubated in the dark for 1 hour at room temperature to efficiently load the cells with Fluo-3. At the end of the incubation, extracellular Fluo-3 was removed by washing the plate with Assay Buffer. Assay Buffer was added back to each well (final volume = 160 µL) prior to beginning the assay. The plate was loaded into a fluorescence imaging plate reader (FLIPR) robotic device (Molecular Devices) with the laser setting at 0.8 Watts. At a time of 15 seconds after initiation of the assay, 40 µL of Assay Buffer containing 150 µM L-AP4 was added to the 160 µL of Assay Buffer in each well of the plate to yield a final concentration of 30 µM L-AP4.

Relative fluorescence intensity (excitation λ = 488 nm / emission λ = 510 nm) was monitored at relevant time intervals throughout the assay period to measure L-AP4-indnced receptor activation.

## Claims

1. A G-protein fusion receptor consisting of the amino acid sequence of SEQ ID NO:49 or SEQ ID NO:61.

2. A nucleic acid comprising a nucleotide sequence encoding for the G-protein fusion receptor of claim 1.

3. An expression vector comprising a nucleotide sequence encoding for the G-protein fusion receptor of claim 1 transcriptionally coupled to a promoter.

4. A recombinant cell comprising the expression vector of claim 3 and a cell wherein the G-protein fusion receptor is expressed and is functional.

5. A recombinant cell produced by combining a vector comprising the nucleic acid of claim 2 and elements for introducing heterologous nucleic acid into a cell wherein the G-protein fusion receptor is expressed, and said cell.

6. A process for the production of a G-protein fusion receptor comprising: growing procaryotic or eukaryotic host cells comprising a nucleic acid sequence expressing the G-protein fusion receptor of claim 1 under suitable nutrient conditions allowing for cell growth.

7. A method of measuring the ability of a compound to effect G-protein fusion receptor activity comprising the steps of:
a) providing said compound to a cell expressing the G-protein fusion receptor of claim 1;
b) measuring the ability of said compound to affect the activity of said receptor as an indication of the ability of said compound to effect G-protein fusion receptor activity.

8. A chimeric receptor consisting of the amino acid sequence of SEQ ID NO:48 or SEQ ID NO:59.

## Patentansprüche

1. G-Protein-Fusionsrezeptor, bestehend aus der Aminosäuresequenz der SEQ ID NO: 49 oder SEQ ID NO: 61.

2. Nukleinsäure, umfassend eine für den G-Protein-Fusionsrezeptor nach Anspruch 1 codierende Nukleotidsequenz.

3. Expressionsvektor, umfassend eine für den G-Protein-Fusionsrezeptor nach Anspruch 1 codierende Nukleotidsequenz in transkriptionaler Kopplung an einen Promotor.

4. Rekombinante Zelle, umfassend den Expressionsvektor nach Anspruch 3 und eine Zelle, wobei der G-Protein-Fusionsrezeptor exprimiert wird und funktionsfähig ist.

5. Rekombinante Zelle, hergestellt durch das Zusammengeben eines die Nukleinsäure nach Anspruch 2 umfassenden Vektors und von Elementen zur Einführung heterologer Nukleinsäure in eine Zelle, wobei der G-Protein-Fusionsrezeptor exprimiert wird, sowie der Zelle.

6. Verfahren zur Herstellung eines G-Protein-Fusionsrezeptors, bei dem man eine Nukleinsäuresequenz, durch die der G-Protein-Fusionsrezeptor nach Anspruch 1 exprimiert wird, umfassende prokaryontische oder eukaryontische Wirtszellen unter geeigneten, das Zellwachstum berücksichtigenden Nährstoffbedingungen kultiviert.

7. Verfahren zur Messung der Fähigkeit einer Verbindung, G-Protein-Fusionsrezeptor-Aktivität zu bewirken, bei dem man in den folgenden Schritten:
a) die Verbindung einer den G-Protein-Fusionsrezeptor nach Anspruch 1 exprimierenden Zelle zuführt;
b) die Fähigkeit der Verbindung zur Beeinflussung der Aktivität des Rezeptors als Zeichen für die Fähigkeit der Verbindung, G-Protein-Fusionsrezeptor-Aktivität zu bewirken, misst.

8. Chimärer Rezeptor, bestehend aus der Aminosäuresequenz der SEQ ID NO: 48 oder SEQ ID NO: 59.

## Revendications

1. Récepteur de fusion à une protéine G constitué de la séquence d'acides aminés de SEQ ID NO : 49 ou de SEQ ID NO : 61.

2. Acide nucléique comprenant une séquence nucléotidique codant pour le récepteur de fusion à une protéine G selon la revendication 1.

3. Vecteur d'expression comprenant une séquence nucléotidique codant pour le récepteur de fusion à une protéine G selon la revendication 1 couplée transcriptionnellement à un promoteur.

4. Cellule recombinante comprenant le vecteur d'expression selon la revendication 3 et une cellule dans laquelle le récepteur de fusion à une protéine G est exprimé et fonctionnel.

5. Cellule recombinante produite en combinant un vecteur comprenant l'acide nucléique selon la revendication 2 et des éléments permettant l'introduction d'un acide nucléique hétérologue dans une cellule dans laquelle le récepteur de fusion à une protéine G est exprimé, et ladite cellule.

6. Procédé de production d'un récepteur de fusion à une protéine G comprenant l'étape consistant à cultiver des cellules hôtes procaryotes ou eucaryotes comprenant une séquence d'acide nucléique exprimant le récepteur de fusion à une protéine G selon la revendication 1 dans des conditions nutritives appropriées propices à la croissance cellulaire.

7. Méthode de mesure de l'aptitude d'un composé à entraîner l'activité d'un récepteur de fusion à une protéine G, comprenant les étapes consistant à :
a) fournir ledit composé à une cellule exprimant le récepteur de fusion à une protéine G selon la revendication 1 ;
b) mesurer l'aptitude dudit composé à affecter l'activité dudit récepteur comme une indication de l'aptitude dudit composé à entraîner l'activité du récepteur de fusion à une protéine G.

8. Récepteur chimère constitué de la séquence d'acides aminés de SEQ ID NO : 48 ou SEQ ID NO : 59.
